# EUROPEAN PATENT APPLICATION

(11) **EP 3 061 748 A1**
(43) Date of publication of application: **31.08.2016**
(21) Application number: 14855618.6
(22) Date of filing: 22.10.2014
(51) Int. Cl.: C07D 215/20, A61K 31/47, A61K 47/10, A61K 47/34, A61K 51/00, A61P 25/28, A61P 43/00

(54) **TAU IMAGING PROBE**

(30) Priority: 22.10.2013 JP 2013233470
(71) Applicant: CLINO Ltd., Sendai-shi, Miyagi 981-0942 (JP)
(72) Inventor: KUDO, Yukitsuka, Sendai-shi Miyagi 980-8577 (JP); OKAMURA, Nobuyuki, Sendai-shi Miyagi 980-8577 (JP); FURUMOTO, Shozo, Sendai-shi Miyagi 980-8577 (JP)
(74) Representative: Vossius & Partner Patentanwälte Rechtsanwälte mbB
(86) International application number: PCT/JP2014/078146
(87) International publication number: WO 2015/060365

(57) **Abstract**

An object of the present invention is to provide a compound represented by formula (I) which is highly specific to tau and can image tau with satisfactory sensitivity, and also has high brain transition, low or non-recognized bone-seeking properties and low or undetected toxicity.

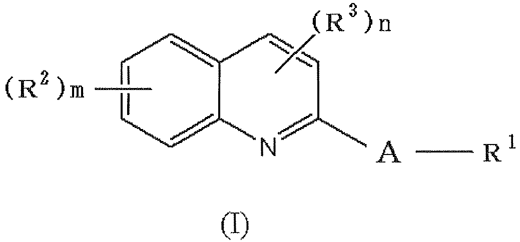

## Description

### Technical Field

The present invention relates to a probe for imaging a β-sheet structure protein which can be used for the diagnosis of conformational diseases, particularly disease (tauopathy) having a cardinal symptom such as intracerebral accumulation of tau protein, for example, Alzheimer's disease.

### Background Art

In Alzheimer's disease, it is known that the accumulation of senile plaque containing amyloid beta-protein (hereinafter referred collectively to as Aβ) as a main component and of neurofibrillary tangles containing hyperphosphorylated tau protein (hereinafter referred collectively to as tau) as a main component proceeds to the degree that it cannot be treated when the specific clinical symptoms of the disease become apparent. In other words, if the current diagnosis of Alzheimer's disease is compared to that of cancer, it is detected only when it has reached the end stage.

Recently, it has been revealed that even in the case of the extremely early stage of very mild Alzheimer's disease that corresponds to mild cognitive impairment (MCI), which is considered as partly precursor state of Alzheimer's disease, autopsy samples show the accumulation of many Aβ and tau, and the state is pathologically almost Alzheimer's disease. Therefore, in Alzheimer's disease, the histopathology is manifested far before the symptom of memory loss appears. In other words, there is a quite large difference between the histopathology and clinical picture of Alzheimer's disease (so-called difference between pathological Alzheimer's disease and clinical Alzheimer's disease).

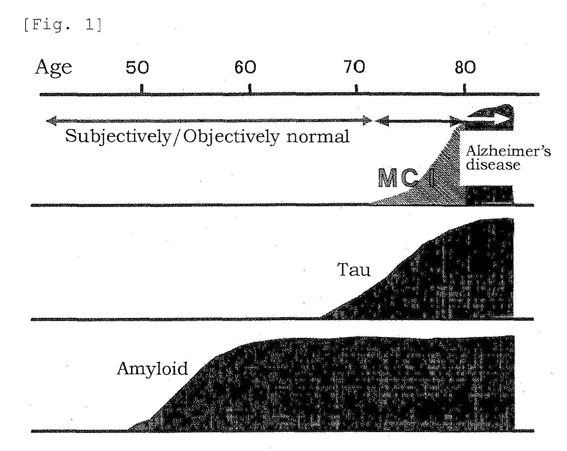

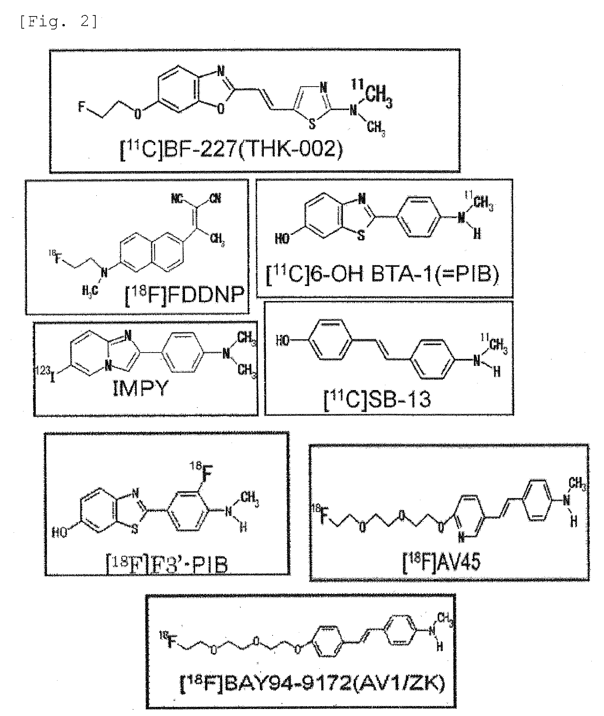

As illustrated in Fig. 1, the accumulation of Aβ is considered to start 10 or more years earlier than that of tau in the brain of Alzheimer's disease. As is apparent from Fig. 1, since the tracing of Aβ was considered most appropriate in order to diagnose Alzheimer's disease in the extremely early stage or before its development, almost all PET probes for the diagnosis of Alzheimer's disease were so-called probes for amyloid imaging to trace Aβ. At first, [¹¹C] labeled probes were mainly used, but afterwards, the development of [¹⁸F] labeled probes that have a long half-life and are easily used in the clinical setting has been attempted. Fig. 2 illustrates the examples of probes for amyloid imaging that has been developed until now.

In the beginning of 2002, images showing administration of PET probes for amyloid imaging of Alzheimer's disease patients were introduced for the first time in the world (refer to Non-Patent Document 1) . The team of Barrio et al., UCLA got the honor of this, and the probes used were [¹⁸F] FDDNP. Afterwards, however, [¹¹C] PIB developed by General Electric, University of Pittsburgh, which has probably now been used for more than 1,000 clinical cases, became the mainstream of probes for amyloid imaging (refer to Non-Patent Document 2).

Many researchers assumed that amyloid imaging in the diagnosis of Alzheimer's disease would be a so-called versatile diagnostic method that enables the diagnosis of the disease with high sensitivity and specificity, as well as early diagnosis, differential diagnosis, diagnosis of severity (or progress), and preclinical diagnosis (so-called detection of presymptomatic high-risk individuals).

However, as clinical research progressed, issues were gradually appeared with amyloid imaging, which had been considered as a versatile diagnostic method. These issues are explained by taking [¹¹C] PIB as an example, as follows:

First, diagnosis of severity (or progress) is impossible. In other words, 2 years after a patient was diagnosed as Alzheimer's disease by [¹¹C] PIB, there was no change in the accumulation of the probe regardless of the progress of the clinical symptoms (refer to Non-Patent Document 3). The reason is considered that the accumulation of Aβ to which [¹¹C] PIB binds reaches a plateau far before MCI is seen prior to development of Alzheimer's disease. Therefore, the severity or progress of Alzheimer's disease cannot be diagnosed with [¹¹C] PIB.

Second, there is a problem that considerable false positives are seen. Surprisingly, the ADNI (Alzheimer's Disease Neuroimaging Initiative) held ahead of the International Conference on Alzheimer's Disease in Chicago in July 2008 reported that 53% of healthy elderly were [¹¹C] PIB positive (refer to Non-Patent Document 4) whereas the incidence rate of Alzheimer's disease is considered to be 4 to 6% of the population of 65 or more years old. Although the present inventors think the figure of 53% is an overestimate, the developers of [¹¹C] PIB themselves recognize the possibility of considerable false positives (refer to Non-Patent Document 5).

The reason for these many false positives is believed to be that there is a considerable dispersion in the accumulation of Aβ in all of normal healthy subjects, MCI, and Alzheimer's disease.

Furthermore, in June to July 2008, it was successively reported that the effects of therapeutic drug (vaccines and secretase inhibitors) groups, which were expected to provide basic remedies based on the Alzheimer's disease/amyloid (or Aβ) hypothesis, were far below expectation. In a report by Holmes et al. in The Lancet it was observed that Aβ vaccines cannot stop the progress of the clinical symptoms at all although Aβ was removed from the brain of Alzheimer's disease patients (refer to Non-Patent Document 6).

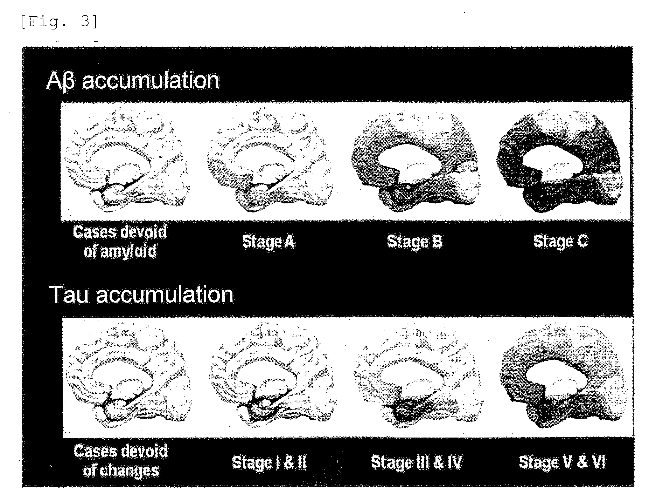

However, another report in The Lancet showed that all accumulation of tau in the patients observed progressed to the final stage. Fig. 3 illustrates the Braak stage of accumulation of Aβ and tau in Alzheimer's disease. For the Braak stage of post-mortem Case 7 and 8, Aβ was considered not to be accumulated (or stage A), while the degree of tau accumulation was stage VI. This implies that in both cases the accumulation of Aβ was mild or less, while the accumulation of tau was the highest level of stage VI.

There were several reports that the histopathology correlating with the clinical symptoms of Alzheimer' s disease was tau rather than Aβ in the early 1990s (Non-Patent Document 7). This was unexpectedly reaffirmed by the report by Holmes et al.

These findings strongly suggest that Aβ binders were less effective as therapeutic drugs after development of Alzheimer's disease, that the degree of Aβ accumulation does not always reflect the severity of Alzheimer's disease, and that it is more reasonable to trace tau rather than Aβ to diagnose the severity of Alzheimer's disease.

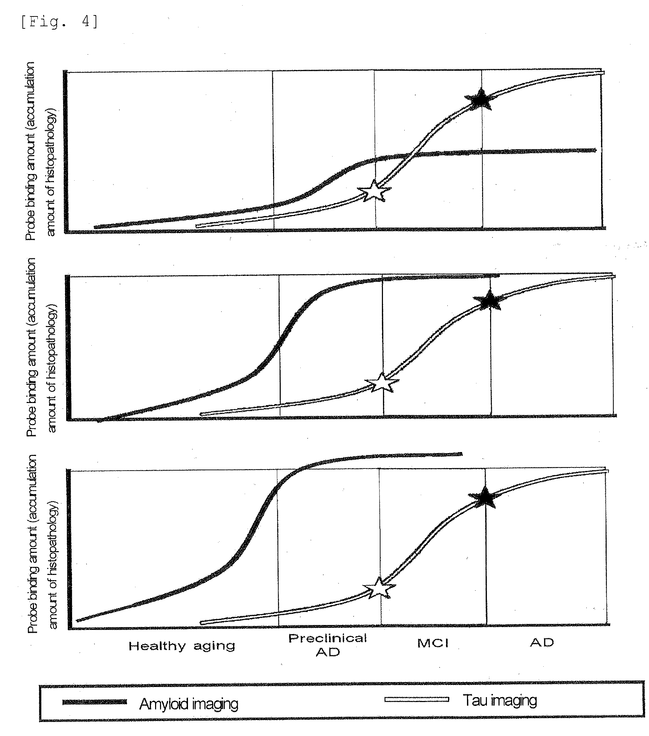

The present inventors think that the relationship between amyloid (or Aβ) and tau in Alzheimer's disease should be revised to Fig. 4. As illustrated in Fig. 4, when there is low accumulation of amyloid, MCI and Alzheimer' s disease develop when the tau accumulation reaches the threshold, and when the accumulation of amyloid is very high, MCI and Alzheimer's disease do not develop when the tau accumulation does not reach the threshold. That is to say, the amount of amyloid accumulation is not related to development of MCI and Alzheimer's disease, while tau accumulation defines this development. It is proposed to say "amyloid (or Aβ) has no threshold, but tau has one".

As described above, tau imaging is probably superior to amyloid imaging, in order to diagnose the severity (or progress) of Alzheimer's disease, or to correctly detect presymptomatic high-risk individuals for Alzheimer's disease.

The present inventors think that it is probable that "tau imaging will play the leading role in diagnosis of Alzheimer's disease, supplemented by amyloid imaging in the future".

The probes for tau imaging are described in, for example, patent literatures 1 to 3, and non-patent literature 8.

The inventors of the present application have successfully provided a compound which is highly specific to tau and can image tau with satisfactory sensitivity, and also has high brain transition, low or non-recognized bone-seeking properties and low or undetected toxicity, and accordingly have filed an international patent application (PCT application) (patent literature 4).

### Prior Art Document

### Patent Document

### (PATENT DOCUMENTS)

[Patent Literature-1]: EP 1574500 A1
[Patent Literature-2]: US 2010/0239496 A1
[Patent Literature-3]: KR 2010-0112423 A
[Patent Literature-4]: WO 2012/057312

### (Non-PATENT DOCUMENTS)

[Non-Patent Literature-1]: Shoghi-Jadid K, Small GW, Agdeppa ED, Kepe V, Ercoli LM, Siddarth P, Read S, Satyamurthy N, Petric A, Huang SC, Barrio JR: Localization of neurofibrillary tangles and beta-amyloid plaques in the brains of living patients with Alzheimer disease. Am. J. Geriatr. Psychiatry 10, 24-35. 2002.
[Non-Patent Literature-2]: Klunk WE, Engler H, Nordberg A, Wang Y, Blomqvist G, Holt DP, Bergstrom M, Savitcheva I, Huang GF, Estrada S, Ausen B, Debnath ML, Barletta J, Price JC, Sandell J, Lopresti BJ, Wall A, Koivisto P, Antoni G, Mathis CA and Langstrom B. : Imaging brain amyloid in Alzheimer's disease with Pittsburgh Compound-B. Ann. Neurol. 55. 306-319 (2004).
[Non-Patent Literature-3]: Engler H, Forsberg A, Almkvist O, Blomquist G, Larsson E, Savitcheva I, Wall A, Ringheim A, Långström B, Nordberg A: Two-year follow-up of amyloid deposition in patients with Alzheimer's disease. Brain. 129. 2856-2866. 2006.
[Non-Patent Literature-4]: Weiner: International Conference on Alzheimer's Disease (ICAD) meeting, Chicago, 2008. Jul 19.
[Non-Patent Literature-5]: Aizenstein HJ, Aizenstein HJ, Nebes RD, Saxton JA, Price JC, Mathis CA, Tsopelas ND, Ziolko SK, James JA, Snitz BE, Houck PR, Bi W, Cohen AD, Lopresti BJ, DeKosky ST, Halligan EM, Klunk WE.: Frequent amyloid deposition without significant cognitive impairment among the elderly. Arch Neurol. 65. 1509-1517. 2008.
[Non-Patent Literature-6]: Holmes C, Boche D, Wilkinson D, Yadegarfar G, Hopkins V, Bayer A, Jones RW, Bullock R, Love S, Neal JW, Zotova E, Nicoll JA : Long-term effects of Abeta42 immunisation in Alzheimer's disease: follow-up of a randomised, placebo-controlled phase I trial. Lancet. 372. 2132-2142. 2008.
[Non-Patent Literature-7]: Arriagada PV, Growdon JH, Hedley-Whyte ET, Hyman BT: Neurofibrillary tangles but not senile plaques parallel duration and severity of Alzheimer's disease. Neurology. 42. 631-639. 1992.
[Non-Patent Literature-8]: Okamura N., Suemoto T., Furumoto S., Suzuki M., Shimadzu H., Akatsu H., Yamamoto T., Fujiwara H., Nemoto M., Maruyama M., Arai H., Yanai K., Sawada T., Kudo Y. Quinoline and benzimidazole derivatives: Candidate probes for in vivo imaging of tau pathology in Alzheimer's disease. Journal of Neuroscience. 25. 10857-10862. 2005.

### Summary of Invention

### Problems to be Solved by the Invention

An object of the present invention is to provide a compound which is highly specific to tau and can image tau with satisfactory sensitivity, and also has high brain transition, low or non-recognized bone-seeking properties and low or undetected toxicity.

### Means for Solving the Problems

In light of the above problems, the present inventors have intensively studied and found a process for preparing a desirable optical isomer with extensively high effectiveness. Also, the present inventors have found that the optical isomer of the compound of a formula (I), a salt thereof or a solvate thereof is a compound which is highly specific to tau and can image tau with satisfactory sensitivity, and also has high brain transition, low or non-recognized bone-seeking properties and low or non-recognized toxicity. They have also found that the compound of a formula (I') can be used as a precursor of the compound of a formula (I), a salt thereof or a solvate thereof.

The present invention provides the following:
[1] A compound represented by the formula (I): 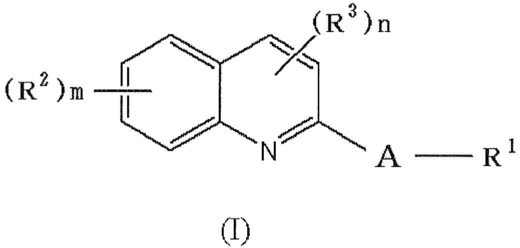 wherein
   A is a cyclic group represented by a formula: 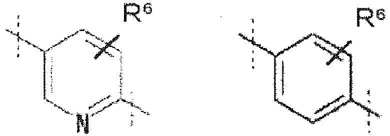 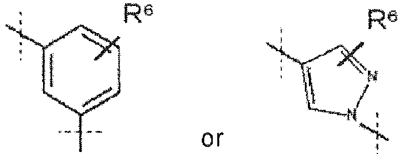
   ring A is unsubstituted, or substituted with R⁶ (the R⁶ is one or more substituents selected independently from halogen, OH, COOH, SO₃H, NO₂, SH, NR^{a}R^{b}, lower alkyl (the alkyl group each independently may be optionally substituted with one or more substituents selected from halogen and hydroxy) and -O-lower alkyl (the alkyl group each independently may be optionally substituted with one or more substituents selected from halogen and hydroxy)),
   R¹ is a halogen atom, a -C (=O)-lower alkyl group (the alkyl group each independently may be optionally substituted with one or more substituents selected from NR^{a}R^{b}, halogen and hydroxy), a lower alkyl group (the alkyl group each independently may be optionally substituted with one or more substituents selected from halogen atom and hydroxy), a -O-lower alkyl group (the alkyl group each independently may be optionally substituted with one or more substituents selected from halogen and hydroxy), or a group represented by a formula: 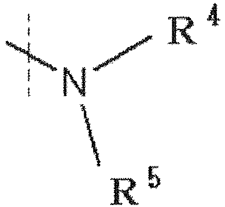 wherein
      R⁴ and R⁵ each independently represents a hydrogen atom, a lower alkyl group (the alkyl group each independently may be optionally substituted with one or more substituents selected from halogen and hydroxy) or a cycloalkyl group, or R⁴, R⁵ and the nitrogen atom to which they are attached are taken together to form a 3- to 8-membered nitrogen-containing aliphatic ring (one or more carbon atoms constituting the nitrogen-containing aliphatic ring each independently may be optionally replaced by a nitrogen atom, a sulfur atom or an oxygen atom, and when a carbon atom is replaced by a nitrogen atom, the nitrogen atom may be optionally substituted with lower alkyl), or
      R⁴ and the nitrogen atom to which it is attached are taken together with ring A to form a 8- to 16-membered nitrogen-containing fused bicyclic ring (one or more carbon atoms constituting the nitrogen-containing fused bicyclic ring each independently may be optionally replaced by a nitrogen atom, a sulfur atom or an oxygen atom, and when a carbon atom is replaced by a nitrogen atom, the nitrogen atom may be optionally substituted with one or two lower alkyl groups), and R⁵ represents a hydrogen atom, a lower alkyl group (the alkyl group each independently may be optionally substituted with one or more substituents selected from halogen and hydroxy), or a cycloalkyl group,
      R² or R³ each independently represents a halogen atom, OH, COOH, SO₃H, NO₂, SH, NR^{a}R^{b}, a lower alkyl group (the alkyl group each independently may be optionally substituted with one or more substituents selected from halogen and hydroxy) or a -O-lower alkyl group (the alkyl group each independently may be optionally substituted with one or more substituents selected from halogen and hydroxy),
      R^{a} and R^{b} each independently represents a hydrogen atom or a lower alkyl group (the alkyl group each independently may be optionally substituted with one or more substituents selected from halogen and hydroxy),
      m is an integer of 0 to 4, and
      n is an integer of 0 to 2,
      with the proviso that regardless of the above-mentioned definitions of R¹, R², R³, and R⁶, at least one of the R¹, R², R³, and R⁶ represents a group represented by formula: 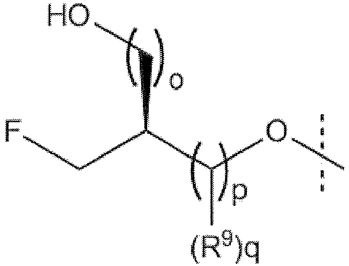 wherein
         R⁹ represents each independently a lower alkyl group (the alkyl group each independently may be optionally substituted with one or more substituents selected from halogen and hydroxy),
         o is an integer of 0 to 1,
         p is an integer of 0 to 1,
         q is an integer of 0 to 2, and
         each line that the above dotted line intersects with means a bond to the other structural moiety of the above general formula (I),
         or a pharmaceutically acceptable salt or solvate thereof.
[2] The compound according to [1] wherein A represents a cyclic group represented by formula: 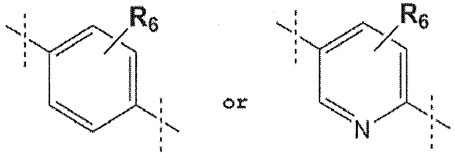
   ring A is unsubstituted, or substituted with R⁶ (the R⁶ is one or more substituents selected independently from halogen and -O-lower alkyl (the alkyl group each independently may be optionally substituted with halogen and hydroxy)),
   R¹ represents a group represented by formula: 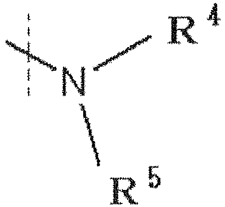 wherein
      R⁴ and R⁵ each independently represent a hydrogen atom, a lower alkyl group (the alkyl group each independently may be optionally substituted with one or more substituents selected from halogen and hydroxy) or a cycloalkyl group,
      R² represents a group represented by formula: 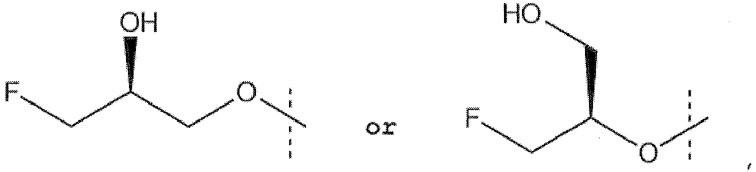
      m is an integer of 1,
      n is an integer of 0, and
      each line that the above dotted line intersects with means a bond to the other structural moiety of the above general formula (I),
   or a pharmaceutically acceptable salt or solvate thereof.
[3] The compound according to [1] or [2] selected from the group consisting of formulae: 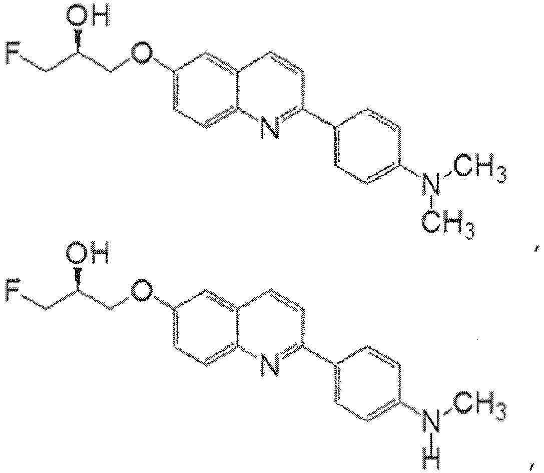 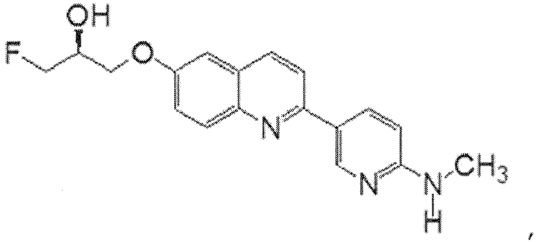 and 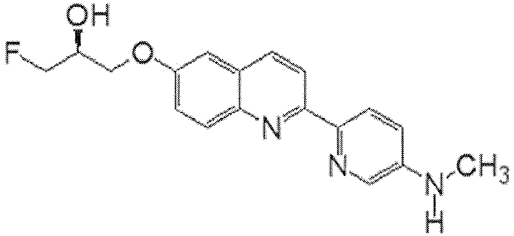 or a pharmaceutically acceptable salt or solvate thereof.
[4] A pharmaceutical composition comprising the compound according to any one of [1] to [3] or a pharmaceutically acceptable salt or solvate thereof, and a pharmaceutically acceptable carrier.
[5] A pharmaceutical composition for the treatment and/or prevention of conformational disease, comprising the compound according to any one of [1] to [3] or a pharmaceutically acceptable salt or solvate thereof, and a pharmaceutically acceptable carrier.
[6] The pharmaceutical composition according to [4] or [5], suitable for injection.
[6a] The pharmaceutical composition according to [4] or [5], wherein the pharmaceutically acceptable carrier is a solubilizing agent.
[6b] The pharmaceutical composition according to [4] or [5], wherein the solubilizing agent is selected from the group consisting of Polysorbate 80, polyethylene glycol, ethanol, propylene glycol and a combination of two or more kinds thereof.
[7] The compound according to any one of [1] to [3], or a pharmaceutically acceptable salt or solvate thereof, wherein the compound is labeled.
[8] The compound according to [7], or a pharmaceutically acceptable salt or solvate thereof, wherein the label is a radioactive nuclide or a positron emitting nuclide.
[9] The compound according to [8], wherein the radioactive nuclide is a γ-ray emitting nuclide.
[10] The compound according to [8], or a pharmaceutically acceptable salt or solvate thereof, wherein the positron emitting nuclide is selected from the group consisting of ¹¹C, ¹³N, ¹⁵O, ¹⁸F, ^{34m}Cl, ⁷⁶Br, ⁴⁵Ti, ⁴⁸V, ⁶⁰Cu, ⁶¹Cu, ⁶²Cu, ⁶⁴Cu, ⁶⁶Ga, ⁸⁹Zr, ^{94m}Tc and ¹²⁴I.
[11] The compound according to [8], or a pharmaceutically acceptable salt or solvate thereof, wherein the positron emitting nuclide is ¹¹C or ¹⁸F.
[12] The compound according to [1], wherein said halogen atom is an F atom and said compound is/are labeled with ¹¹C or ¹⁸F.
[13] The compound according to [2], wherein said halogen atom is an F atom and said compound is/are labeled with ¹⁸F.
[14] The compound according to [12] or [13] selected from the group consisting of the following formulae: 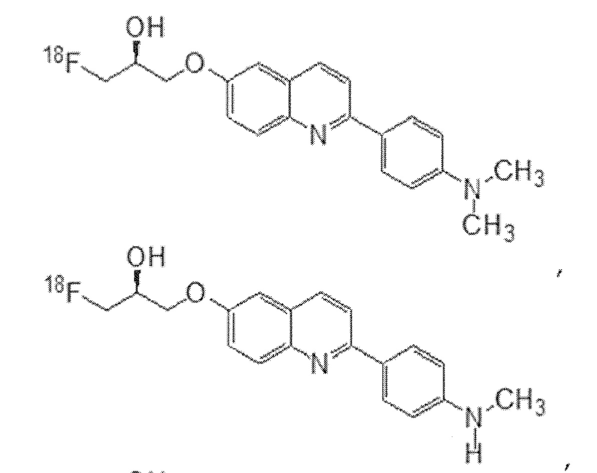 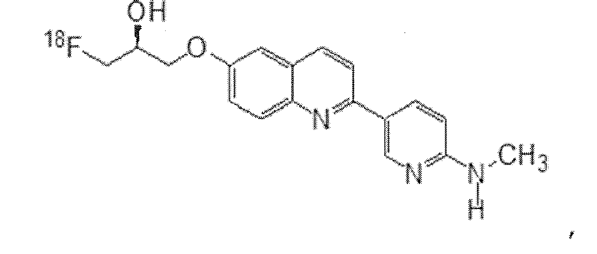 and 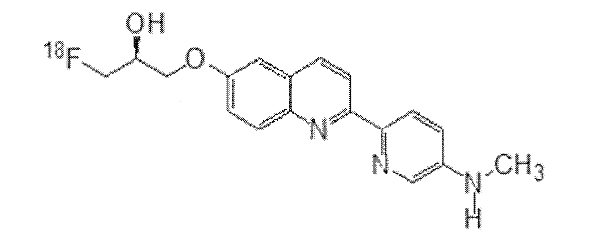 or a pharmaceutically acceptable salt or solvate thereof.
[15] A pharmaceutical composition for diagnostic imaging, comprising any one of the compound according to [7] to [14], or a pharmaceutically acceptable salt or solvate thereof.
[16] The pharmaceutical composition according to [15] for the diagnosis of conformational disease.
[17] The pharmaceutical composition according to [15] for the detection or staining of a β-sheet structure protein.
[18] A kit for diagnostic imaging, comprising the compound according to any one of [7] to [14] or a pharmaceutically acceptable salt or solvate thereof as an essential ingredient.
[19] The kit according to [18] for the diagnosis of conformational disease.
[20] The kit according to [18] for the detection or staining of a β-sheet structure protein.
[21] A method of treating and/or preventing conformational disease in a subject, which comprises administering the compound according to any one of [1] to [3] or a pharmaceutically acceptable salt or solvate thereof to the subject.
[22] A method of diagnostic imaging of conformational disease in a subject, which comprises administering the compound according to any one of [7] to [14] or a pharmaceutically acceptable salt or solvate thereof to the subject.
[23] A method for diagnostic imaging, which comprises detecting or staining a β-sheet structure protein in a sample by staining the sample with the compound according to any one of [7] to [14] or a pharmaceutically acceptable salt or solvate thereof.
[24] Use of the compound according to any one of [1] to [3] or a pharmaceutically acceptable salt or solvate thereof for the production of a pharmaceutical composition for the treatment and/or prevention of conformational disease in a subject.
[25] Use of the compound according to any one of [7] to [14] or a pharmaceutically acceptable salt or solvate thereof for the production of a composition or kit for the diagnostic imaging of conformational disease in a subject.
[26] Use of the compound according to any one of [7] to [14] or a pharmaceutically acceptable salt or solvate thereof for the production of a diagnostic imaging composition or kit for detecting or staining a β-sheet structure protein.
[27] The pharmaceutical composition, kit, method or use according to any one of [15] to [26], wherein the conformational disease is tauopathy including Alzheimer's disease, and the β-sheet structure protein is tau protein.
[28] A method of producing the compound represented by formula (I) according to [1], which comprises the following steps of:
   (i) reacting a compound of a formula (V): 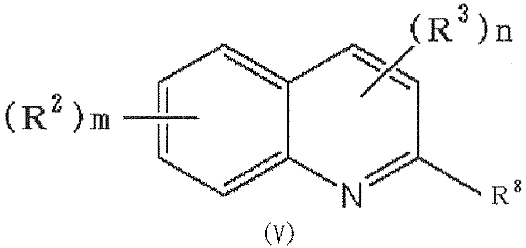 wherein
      R⁸ represents a hydroxy group or a halogen atom. R², R³, m and n are as the same as defined as the above [1], with the proviso that at least one of R² and R³ represents a hydroxy group, with any compound represented by formula: 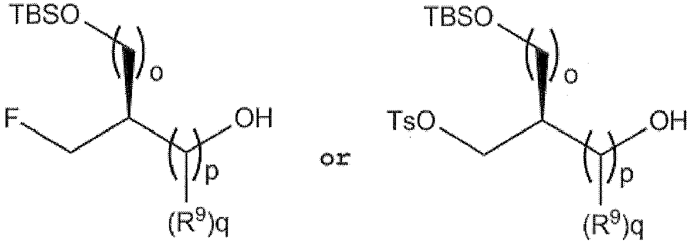 wherein,
         R⁹, o, p and q are as the same as defined as the above [1], to obtain a compound represented by a formula (V'): 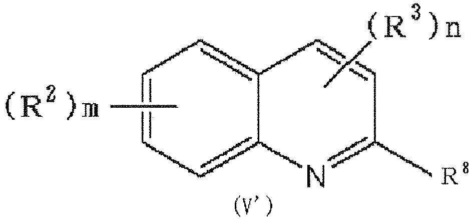 wherein
            R⁸ represents a hydroxy group or a halogen atom. R², R³, m and n are as the same as defined as the above formula (V), with the proviso that regardless of the definition of the formula (I) according to [1], at least one of R² and R³ represents any group represented by formula: 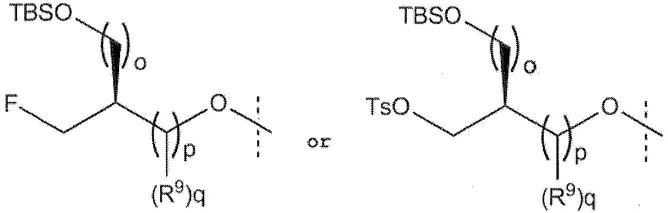 wherein,
               R⁹, o, p and q are as the same as defined above,
   (ii) reacting a compound represented by the above formula (V') with a compound represented by formula (VI) or (VII): 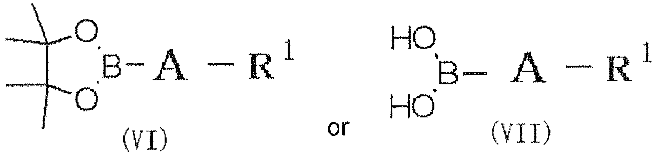 wherein,
      A and R¹ are as the same as defined as the above [1], to obtain the above compound represented by formula (I) wherein at least one of R² and R³ represents a group represented by formula: 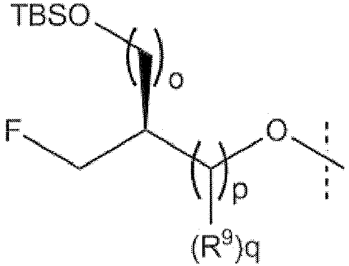 wherein,
      R⁹, o, p and q are as the same as defined above, and then isolating the compound, or
   (iii) optionally, converting the obtained compound represented by formula (I) into another compound represented by formula (I), and then isolating the compound.
[29] A method of producing the compound according to [2], which comprises
   (i) reacting the compound represented by formula (V): 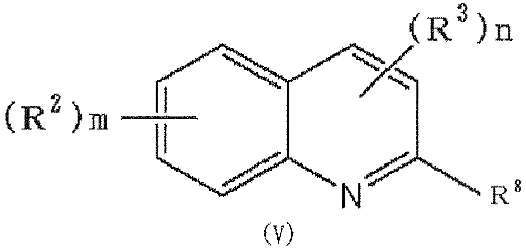 wherein
      R⁸ represents a hydroxy group or a halogen atom. R², R³, m and n are as the same as defined as the above-mentioned [1], with the proviso that at least one of R² and R³ represents a hydroxy group,
      with a compound represented by formula: 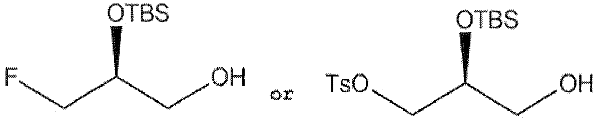 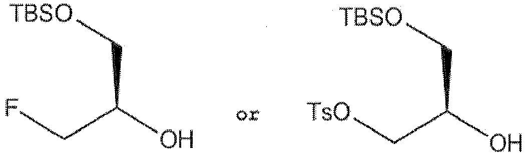 or to obtain a compound represented by formula (V'): 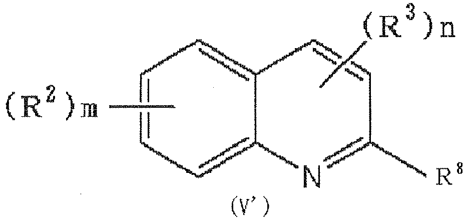 wherein,
      R⁸ represents a hydroxy group or a halogen atom. R², R³, m and n are as the same as defined as the above formula (V), with the proviso that regardless of the definition of the formula (I) according to [1], at least one of R² and R³ represents any group represented by formula: 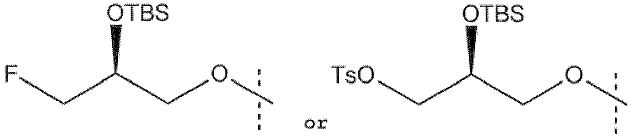 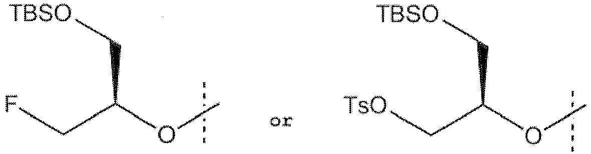 or
   (ii) reacting the above compound represented by formula (V') with a compound represented by formula (VI) or (VII): 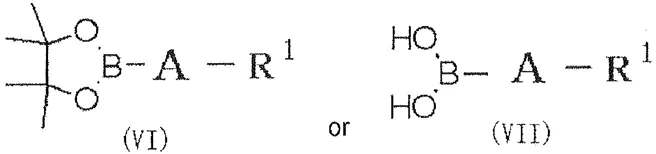 wherein,
      A and R¹ are as the same as defined as the above [1], to obtain the above compound represented by formula (I) wherein at least one of R² and R³ represents a formula: 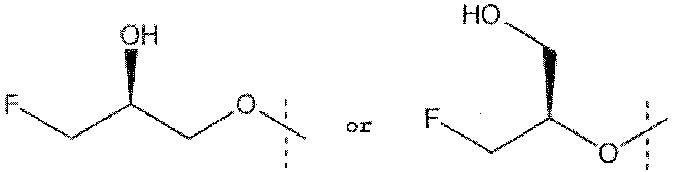 and then isolating the compound, or
   (iii) optionally, converting the obtained compound represented by formula (I) into another compound represented by formula (I), and then isolating the compound.
[30] A method for producing the compound represented by formula (I) according to [1] wherein at least one of R² and R³ represents a group represented by formula: 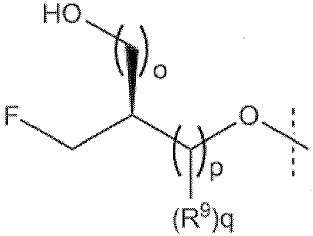 wherein,
   R⁹, o, p and q are as the same as defined as the above [1], which comprises
   (i) reacting a compound represented by formula (V): 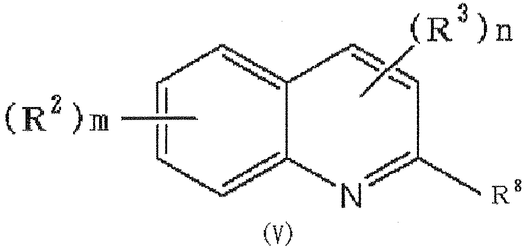 wherein,
      R⁸ represents a hydroxyl group or a halogen atom. R², R³, m and n are as the same as defined as the above [1], with the proviso that at least one of R² and R³ represents a hydroxy group, with a compound represented by formula (VI) or (VII): 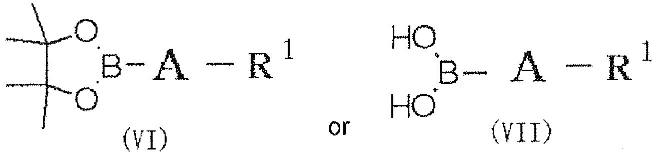 wherein,
      A and R¹ are as the same as defined as the above [1], to obtain a compound represented by formula (V"): 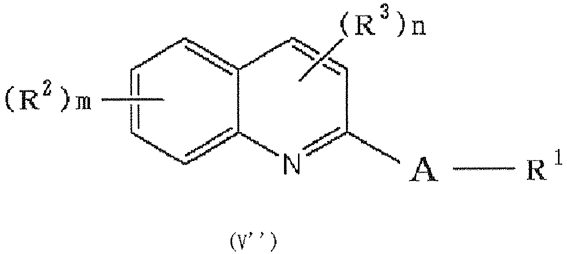 wherein,
      R¹, R², R³, A, m and n are as the same as defined as the above formulae (V), (VI), and (VII), with the proviso that at least one of R² and R³ represents a hydroxy group,
   (ii) reacting the above compound represented by formula (V") with a compound represented by formula: 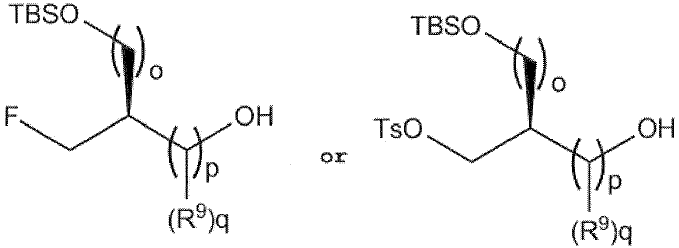 wherein,
      R⁹, o, p and q are as the same as defined as the above [1], to obtain the above-mentioned compound represented by formula (I) wherein at least one of R² and R³ represents a formula: 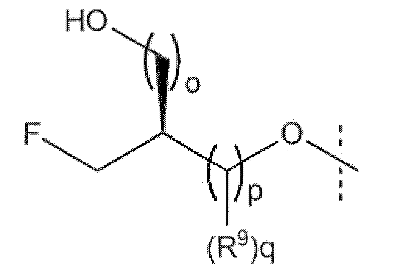 wherein,
      R⁹, o, p and q are as the same as defined as above, and then isolating the compound, or
   (iii) optionally, converting the obtained compound represented by formula (I) into another compound represented by formula (I), and then isolating the compound.
[31] The method for producing the compound according to [2] wherein at least one of R² and R³ represents a formula: 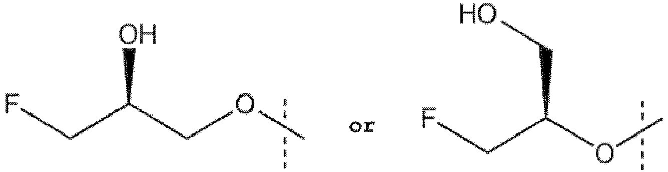 , which comprises
   (i) reacting a compound represented by formula (V): 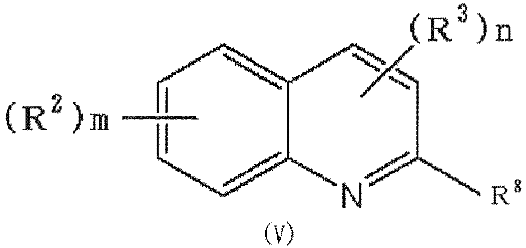 wherein,
      R⁸ represents a hydroxyl group or a halogen atom. R², R³, m and n are as the same as defined as the above [1], with the proviso that at least one of R² and R³ represents a hydroxy group, with a compound represented by formula (VI) or (VII): 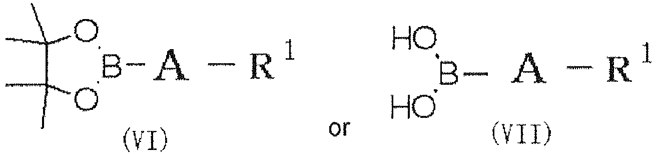 wherein,
      A and R¹ are as the same as defined as the above [1], to obtain a compound represented by formula (V"): 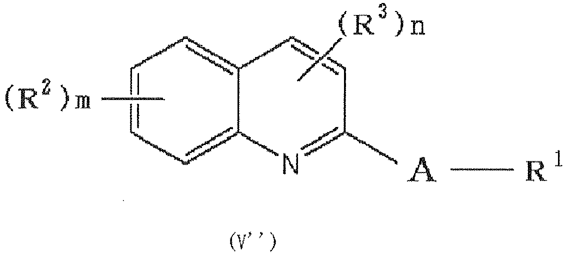 wherein,
      R¹, R², R³, A, m and n are as the same as defined as the formula (V), (VI), and (VII), with proviso that at least of R² and R³ represents a hydroxy group,
   (ii) reacting the above compound represented by formula (V") with any compound represented by formula: 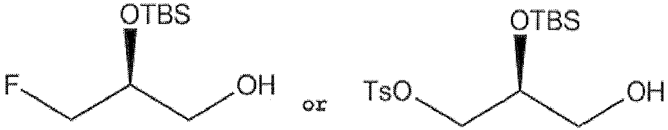 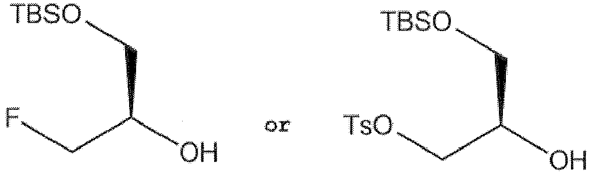 or to obtain the above compound represented by formula wherein at least of R² and R³ represents a formula: 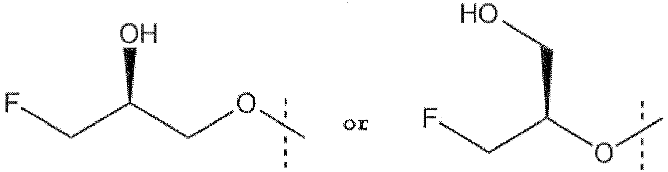 and then isolating the compound, or
   (iii) optionally, converting the obtained compound represented by formula (I) into another compound represented by formula (I), and then isolating the compound.
[32] The method according to any one of [28] to [31], which comprises
   starting with a compound represented by formula (1): 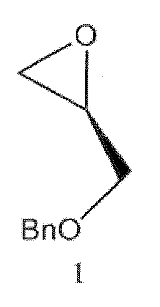 to prepare a compound represented by formula (4): 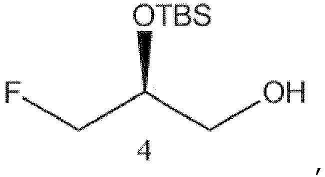 and
   using the compound represented by formula (4) in the reaction in each process according to [28] to [31].
[33] A compound represented by formula (I'): 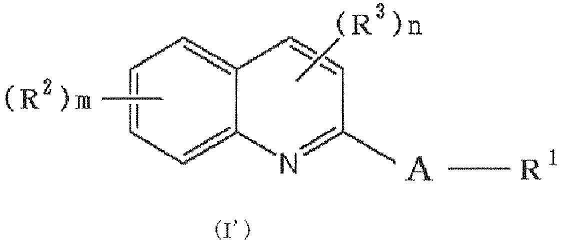 wherein
   A is a cyclic group represented by a formula: 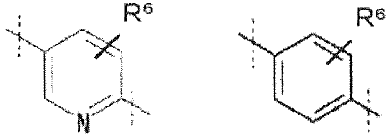 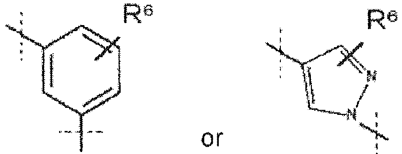
   ring A is unsubstituted, or substituted with R⁶ (the R⁶ is one or more substituents selected independently from halogen, OH, COOH, SO₃H, NO₂, SH, NR^{a}R^{b}, lower alkyl (the alkyl group each independently may be optionally substituted with one or more substituents selected from halogen and hydroxy), and -O-lower alkyl (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group protected with a protecting group for hydroxy)),
   R¹ is a halogen atom, a -C(=O)-lower alkyl group (the alkyl group each independently may be optionally substituted with one or more substituents selected from NR^{a}R^{b}, halogen and hydroxy), a lower alkyl group (the alkyl group each independently may be optionally substituted with one or more substituents selected from halogen atom and hydroxy), a -O-lower alkyl group (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group that is protected with a protecting group for hydroxy), or a group represented by a formula: 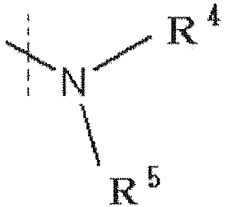 wherein
      R⁴ and R³ each independently represents a hydrogen atom, a lower alkyl group (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group that is protected with a protecting group for hydroxy) or a cycloalkyl group, or R⁴, R⁵ and the nitrogen atom to which they are attached are taken together to form a 3- to 8-membered nitrogen-containing aliphatic ring (one or more carbon atoms constituting the nitrogen-containing aliphatic ring each independently may be optionally replaced by a nitrogen atom, a sulfur atom or an oxygen atom, and when a carbon atom is replaced by a nitrogen atom, the nitrogen atom may be optionally substituted with lower alkyl), or
      R⁴ and the nitrogen atom to which it is attached are taken together with ring A to form a 8- to 16-membered nitrogen-containing fused bicyclic ring (one or more carbon atoms constituting the nitrogen-containing fused bicyclic ring each independently may be optionally replaced by a nitrogen atom, a sulfur atom or an oxygen atom, and when a carbon atom is replaced by a nitrogen atom, the nitrogen atom may be optionally substituted with one or two lower alkyl groups), and R⁵ represents a hydrogen atom, a lower alkyl group, or a cycloalkyl group,
      R² or R³ each independently represents a halogen atom, OH, COOH, SO₃H, NO₂, SH, NR^{a}R^{b}, a -O-lower alkyl group (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group that is protected with a protecting group for hydroxy),
      R^{a} and R^{b} each independently represents a hydrogen atom or a lower alkyl group (the alkyl group each independently may be optionally substituted with one or more substituents selected from halogen and hydroxy),
      m is an integer of 0 to 2, and
      n is an integer of 0 to 2,
      with the proviso that regardless of the above-mentioned definitions of R¹, R², R³, and R⁶, at least one of the R¹, R², R³, and R⁶ represents a group represented by formula: 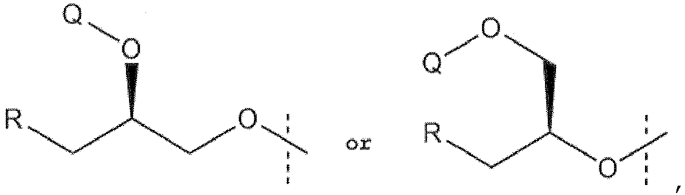
      or the R¹ represents a group represented by formula: 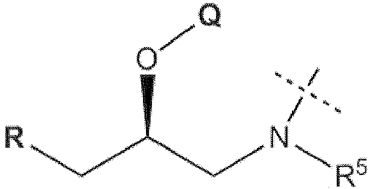 wherein,
         R⁵ is the same as defined above, and
         the Q substituent represents a protecting group for hydroxy group that has a resistance against a nucleophilic substitution by fluorine anion and may be removed under acidic or alkali condition, and the R substituent represents a functional group that works as a leaving group against a nucleophilic substitution by fluorine anion, and
         each line that the above dotted line intersects with means a bond to the other structural moiety of the above general formula I',
         or a pharmaceutically acceptable salt or solvate thereof.
[34] The compound according to [33] or a pharmaceutically acceptable salt or solvate thereof, wherein the protecting group for hydroxy group that has a resistance against a nucleophilic substitution by fluorine anion and may be removed under acidic or alkali condition is selected from a 2-tetrahydropyranyl group, a methoxymethyl group, a 2-methoxyetoxymethyl group, an ethoxyethyl group, an acetyl group, and a pivaloyl group,
   the functional group that works as a leaving group against a nucleophilic substitution by fluorine anion is selected from a p-toluenesulfonyloxy group, a methanesulfonyloxy group, a chloromethanesulfonyloxy group, and a trifluoromethanesulfonyloxy group.
[35] The compound according to [33] wherein
   A represents a cyclic group represented by formula: 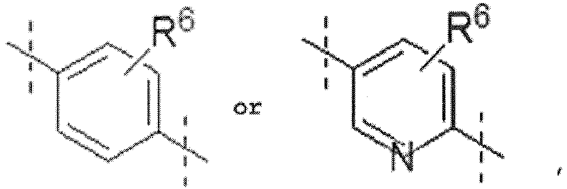
   ring A is unsubstituted, or substituted with R⁶ (the R⁶ is one or more substituents selected independently from halogen and -O-lower alkyl (the alkyl group each independently may be optionally substituted with halogen and hydroxy)),
   R¹ represents a group represented by formula: 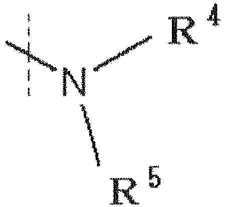 wherein
      R⁹ and R⁵ each independently represent a hydrogen atom, a lower alkyl group (the alkyl group each independently may be optionally substituted with one or more substituents selected from halogen and hydroxy) or a cycloalkyl group,
      R² represents a group represented by formula: 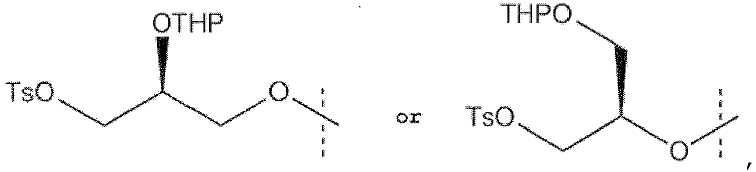
      m is an integer of 1,
      n is an integer of 0, and
      each line that the above dotted line intersects with means a bond to the other structural moiety of the above general formula I',
      or a pharmaceutically acceptable salt or solvate thereof.
[36] The compound according to any one of [33] to [35] selected from the group consisting of formulae: 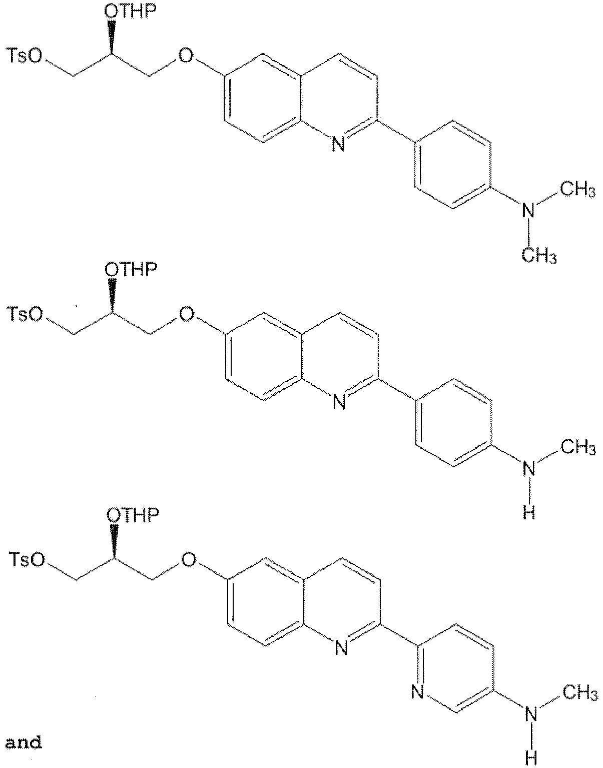 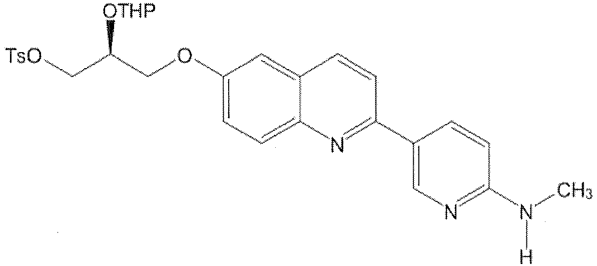 or a pharmaceutically acceptable salt or solvate thereof.
[37] A kit for producing the labeled compound according to any one of [7] to [14], or a pharmaceutically acceptable salt or solvate thereof, comprising:
   the compound according to any one of [33] to [36], or a pharmaceutically acceptable salt or solvate thereof,
   a labeling agent or a reagent for preparing the agent, or a solvent,
   a container or an instrument for use of a labeling synthesis or a formulation such as syringe, three-way stopcock, needle, solid-phase extraction cartridge, and sterilizing filter, and
   optionally, instructions for carrying out labeling.
[38] The kit according to [37], wherein the label is a radioactive nuclide or a positron emitting nuclide, or an agent containing them.
[39] The kit according to [37], wherein the radioactive nuclide is a γ-ray emitting nuclide.
[40] The kit according to [37], wherein the positron emitting nuclide as the labeling agent is selected from the group consisting of ¹¹C, ¹³N, ¹⁵O, ¹⁸F, ^{34m}Cl, ⁷⁶Br, ⁴⁵Ti, ⁴⁸V, ⁶⁰Cu, ⁶¹Cu, ⁶²Cu, ⁶⁴Cu, ⁶⁶Ga, ⁸⁹Zr, ^{94m}Tc and ¹²⁴I.
[41] The kit according to according to [40], wherein the positron emitting nuclide is ¹⁸F.
[42] A method to separate racemic compounds according to claim 1 represented by formula (I): 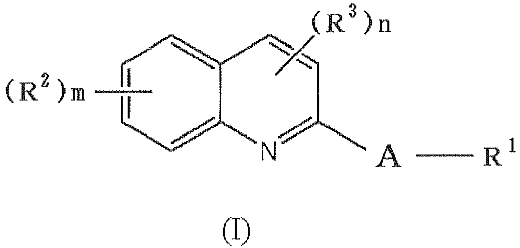 wherein
   A represents a cyclic group represented by formula: 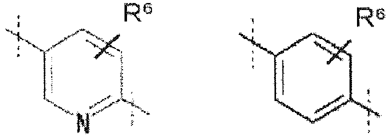 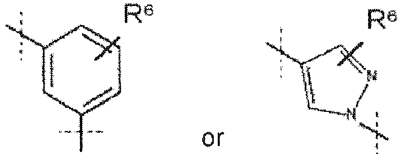
   ring A is unsubstituted, or substituted with R⁶ (the R⁶ is one or more substituents selected independently from halogen, OH, COOH, SO₃H, NO₂, SH, NR^{a}R^{b}, lower alkyl (the alkyl group each independently may be optionally substituted with one or more substituents selected from halogen and hydroxy) and -O-lower alkyl (the alkyl group each independently may be optionally substituted with one or more substituents selected from halogen and hydroxy)),
   R¹ is a halogen atom, a -C (=O)-lower alkyl group (the alkyl group each independently may be optionally substituted with one or more substituents selected from NR^{a}R^{b}, halogen and hydroxy), a lower alkyl group (the alkyl group each independently may be optionally substituted with one or more substituents selected from halogen and hydroxy), a -O-lower alkyl group (the alkyl group each independently may be optionally substituted with one or more substituents selected from halogen and hydroxy), or a group represented by a formula: 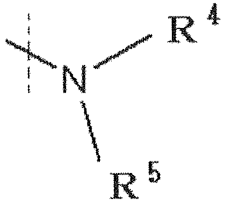 wherein
      R⁴ and R⁵ each independently represent a hydrogen atom, a lower alkyl group (the alkyl group each independently may be optionally substituted with one or more substituents selected from halogen and hydroxy) or a cycloalkyl group, or R⁴, R⁵ and the nitrogen atom to which they are attached are taken together to form a 3- to 8-membered nitrogen-containing aliphatic ring (one or more carbon atoms constituting the nitrogen-containing aliphatic ring each independently may be optionally replaced by a nitrogen atom, a sulfur atom or an oxygen atom, and when a carbon atom is replaced by a nitrogen atom, the nitrogen atom may be optionally substituted with lower alkyl), or
      R⁴ and the nitrogen atom to which it is attached are taken together with ring A to form a 8- to 16-membered nitrogen-containing fused bicyclic ring (one or more carbon atoms constituting the nitrogen-containing fused bicyclic ring each independently may be optionally replaced by a nitrogen atom, a sulfur atom or an oxygen atom, and when a carbon atom is replaced by a nitrogen atom, the nitrogen atom may be optionally substituted with one or two lower alkyl groups), and R⁵ represents a hydrogen atom, a lower alkyl group (the alkyl group each independently may be optionally substituted with one or more substituents selected from halogen and hydroxy), or a cycloalkyl group,
      R² or R³ each independently represents a halogen atom, OH, COOH, SO₃H, NO₂, SH, NR^{a}R^{b}, a lower alkyl group (the alkyl group each independently may be optionally substituted with one or more substituents selected from halogen and hydroxy) or a -O-lower alkyl group (the alkyl group each independently may be optionally substituted with one or more substituents selected from halogen and hydroxy),
      R^{a} and R^{b} each independently represents a hydrogen atom or a lower alkyl group (the alkyl group each independently may be optionally substituted with one or more substituents selected from halogen and hydroxy),
      m is an integer of 0 to 4, and
      n is an integer of 0 to 2,
      with the proviso that regardless of the above-mentioned definitions of R¹, R², R³, and R⁶, at least one of the R¹, R², R³, and R⁶ represents a group represented by formula: 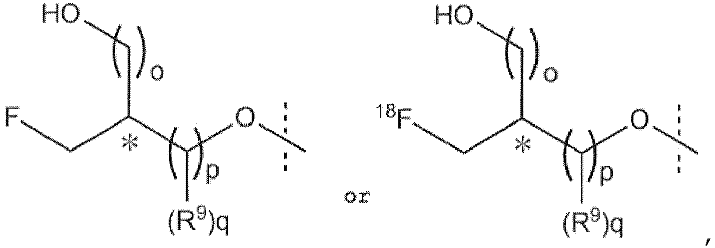 wherein
         R⁹ represents each independently a lower alkyl group (the alkyl group each independently may be optionally substituted with one or more substituents selected from halogen and hydroxy),
         o is an integer of 0 to 1,
         p is an integer of 0 to 1,
         q is an integer of 0 to 2, and
         each line that the above dotted line intersects with means a bond to the other structural moiety of the above general formula (I),
         to the optical active compound according to any one of [1] to [3] as one enantiomer by using an optical active column.
[42a] The separation method according to [42], wherein at least one of the above-mentioned R¹, R², R³ and R⁶ represents a formula: 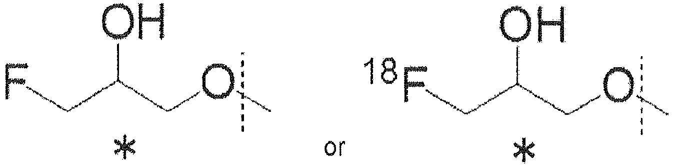 wherein,
   the symbol * represents an asymmetric center.
[43] A method for producing the compound according to any one of [7] to [14] by a labeling synthesis via a chemical synthesis method with a labeling agent or an isotope exchange method.
[44] The method according to [42], which comprises contacting the solution containing the compound according to any one of [33] to [36] with an ion-exchange resin supported by ¹⁸F⁻ to convert to the ¹⁸F⁻ labeled compound according to [7] to [14].
[45] A method for producing the ¹⁸F⁻ labeled compound according to any one of [7] to [14] by using the compound according to any one of [33] to [36] as a label precursor, and ¹⁸F anion.
[46] The method according to [45], which comprises reacting the compound according to [33] to [36] with ¹⁸F anion in absolute highly polar solvent with heating to introduce ¹⁸F into the compound via nucleophilic substitution, followed by removing a protecting group for hydroxy group under acidic or alkali condition.

### Effects of the Invention

According to the present invention, there is provided a compound having very high safety, which is highly specific to tau and can image tau with satisfactory sensitivity, and also has high brain transition, low or undetected bone-seeking properties and low or undetected toxicity, and a precursor thereof. Accordingly, the diagnosis, the treatment and/or prevention of tauopathy can be carried out using the compound of the present invention. Also, the present invention enables diagnostic imaging of tauopathy, particularly diagnostic imaging using positron emission tomography (PET). Accordingly, the present invention facilitates accurate diagnosis, effective treatment and prevention in the early stages of tauopathy, particularly Alzheimer's disease.

### Brief Description of the Drawings

Fig. 1 is a diagram showing deviation between a clinical picture and a histological picture in Alzheimer's disease. Cited from Alzheimer' s Disease (written by Yasuo IHARA, Hiroyuki ARAKI), Asahi Shimbun Company, 2007, Tokyo, partly revised. In the onset of Alzheimer's disease of patients aged 80 years, accumulation of Aβ starts at the age of 50 years and has already reached a plateau at the age of 60 years. On the other hand, tau accumulation proceeds age-dependently at the age of 70 years.
Fig. 2 illustrates PET probes for amyloid imaging developed to date.
Fig. 3 is a diagram showing stages of Aβ accumulation and tau accumulation in Alzheimer's disease. Cited from Braak & Braak: Neurobiol aging. 18. 351-357. 1997, partly revised. Referring to stages of Braak after death of cases 7 and 8 in Non-Patent Document 6, Aβ accumulation was considered as Cases devoid of amyloid (or stage A), while tau accumulation was in stage VI. That is, this means that although Aβ accumulation is mild or globally mild in both cases, tau accumulation was in stage VI in which an accumulation level is the highest.
Fig. 4 is a diagram showing the relationship between amyloid (or Aβ) and tau in Alzheimer's disease (proposed by the present inventors). As shown in upper, middle and lower columns, when amyloid is not much accumulated, MCI and Alzheimer's disease develop when the tau accumulation reaches the threshold, and when amyloid is strongly accumulated, MCI and Alzheimer's disease do not develop when the tau accumulation does not reach the threshold. That is to say, the amount of amyloid accumulation is not related to development of MCI and Alzheimer's disease, while tau accumulation defines this development. In other words, amyloid (or Aβ) has no threshold, but tau has one.

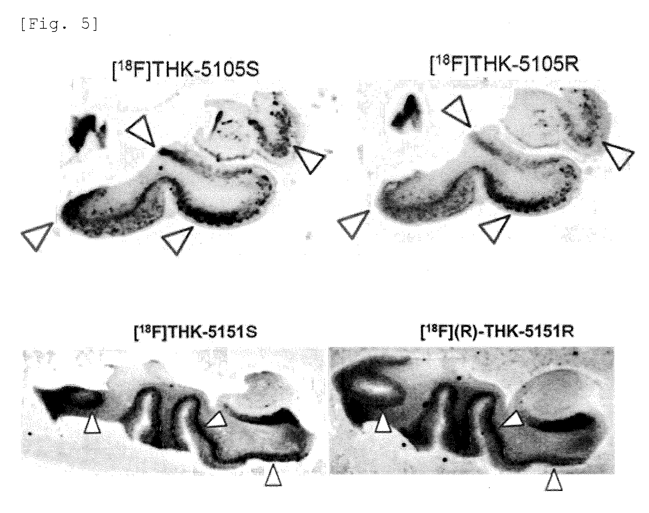

Fig. 5 is an autoradiography image of a hippocampus section of an Alzheimer's disease patient using the compound of the present invention (S- form) and the comparative compound (R-form).

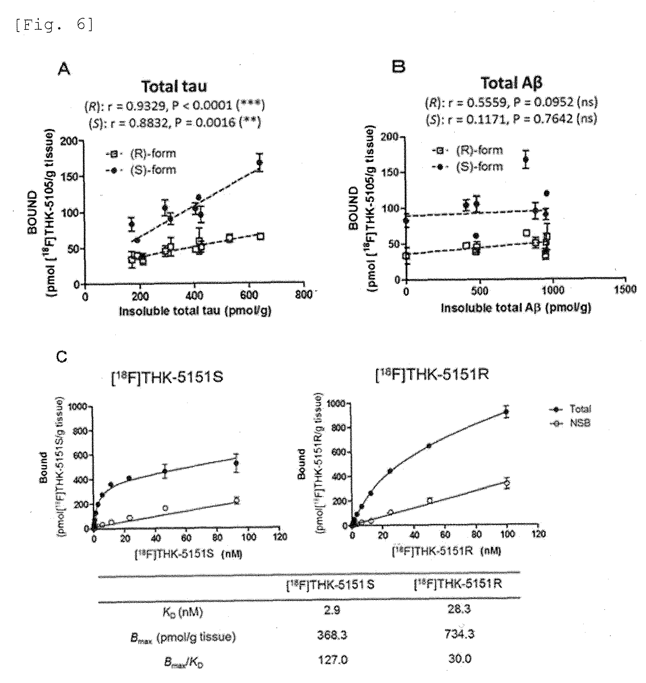

Fig. 6 is a diagram of the binding test results of the compound of the present invention using AD brain tissue homogenate. Figure 6A is a figure indicating the correlation between the tissue binding amounts and the tau concentration. Figure 6B is a figure indicating the correlation between the tissue binding amounts and Aβ concentration. Figure 6C is a saturation binding curve (total binding and non-specific binding), as well as the calculated values from the data, Kd and Bmax.

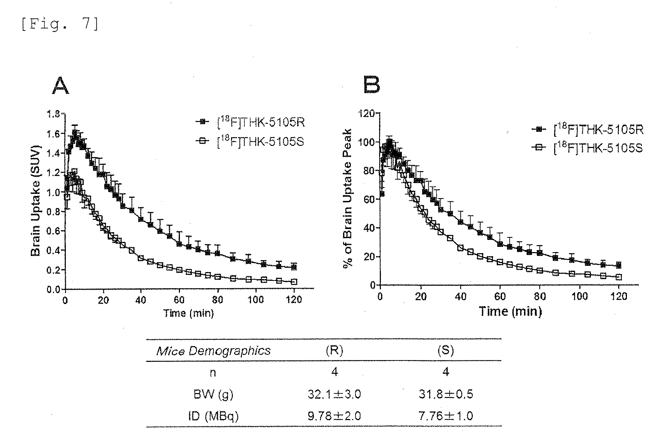

Figure 7 is a graph and a table showing the results of Dynamism evaluation (small animal PET) in normal mouse brain using the compound of the present invention. Each of Figure 7A and Figure 7B is a time-activity curve (TAC) showing retention in brain tissue of a compound of the present invention (S- form) and a comparative compound (R- form).

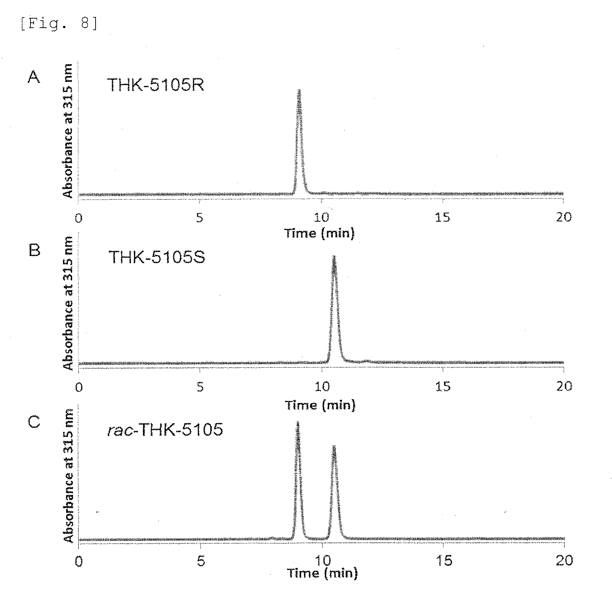

Figure 8 is a figure indicating that a compound of the present invention (S- form) and a comparative compound (R- form) can be separated by chiral column. Figure 8A is a chromatogram of the comparative compound R- form, and Figure 8B is that of the S- form, and Figure 8C is that of racemic form.

### Mode for Carrying Out the Invention

The compounds of the present invention are compounds of formulae (I) and (I') described below, or salts or solvates thereof. As used herein, "compound of the present invention" and "compound according to the present invention" include the compounds of formulae (I) and (I') described below, and salts and solvates thereof, unless otherwise specified.

As used herein, "lower alkyl group" means a linear or branched alkyl group having 1 to 6 carbon atoms, and specific examples thereof include a methyl group, an ethyl group, a propyl group, an isopropyl group, a butyl group, an isobutyl group, a sec-butyl group, a tert-butyl group, a pentyl group, an isopentyl group, a neopentyl group, a 1,1-dimethylpropyl group, a 1-methylbutyl group, a 2-methylbutyl group, a 3-methylbutyl group, a 1,2-dimethylpropyl group, a hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,2,2-trimethylpropyl group, a 1-ethyl-2-methylpropyl group and the like. The term "lower alkoxy" means -O-lower alkyl.

The lower alkyl group each independently may be optionally substituted with one or more (for example, 1 to 3) substituents selected from halogen and hydroxy.

As used herein, "cycloalkyl group" means a cycloalkyl group having 3 to 7 carbon atoms, and specific examples thereof include a cyclopropyl group, a cyclobutyl group, a cyclopentyl group, a cyclohexyl group and a cycloheptyl group.

As used herein, "halogen" means fluorine, chlorine, bromine or iodine.

As used herein, "tau protein" and "tau" have the same meanings. As used herein, "amyloid beta protein", amyloid β protein", "Aβ protein", "amyloid beta", "amyloid β" and "Aβ" have the same meanings.

Non-limiting embodiments of the compound of the formula (I) : 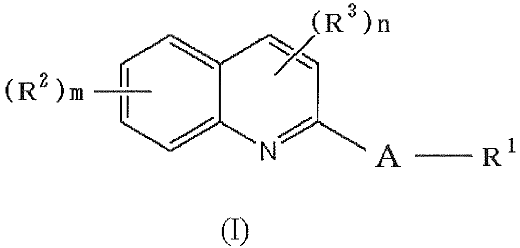 [wherein the respective symbols are as defined above] are described herein by way of specific examples.

Ring A means a formula: 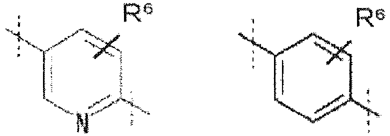 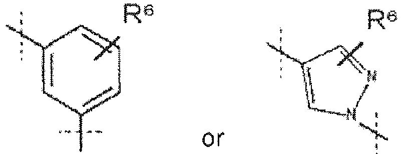 wherein, each line that the dotted line intersects with means a bond to the other structural moiety of the above general formula (I). That is, bonds existing at 2- and 5-positions of pyridine ring are respectively attached to R¹-A- moiety and quinoline ring of the above-mentioned general formula (I). Also, bonds existing at 1- and 4-positions of pyrazole ring are respectively attached to R¹-A- moiety and quinoline ring of the above-mentioned general formula (I).

The ring A is preferably a cyclic group represented by formula:

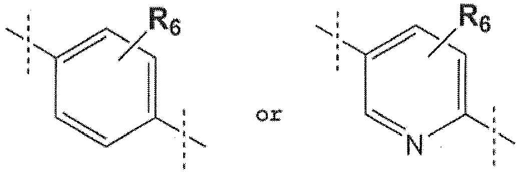

Ring A is unsubstituted, or substituted with one to four (preferably one) R⁶ substituents.

The R⁶ is one or more (preferably one) substituents selected independently from halogen, OH, COOH, SO₃H, NO₂, SH, NR^{a}R^{b}, lower alkyl (the alkyl group each independently may be optionally substituted with one or more (preferably two or more) substituents selected from halogen and hydroxy) and -O-lower alkyl (the alkyl group each independently may be optionally substituted with one or more substituents selected from halogen and hydroxy). Preferably, the R⁶ is one or more (preferably one) substituents selected independently from halogen and -O-lower alkyl (the alkyl group each independently may be optionally substituted with one or more (preferably two or more) substituents selected from halogen and hydroxy). The term "lower alkyl group" represented by the R⁶ means the same groups as those in the lower alkyl group defined above. Among these groups, a linear or branched alkyl group having 1 to 5 carbon atoms, for exmaple, a methyl group, an ethyl group, a propyl group, and a 1,1-dimethylpropyl group are preferable, and the alkyl group each independently may be optionally substituted with substituent selected from one halogen and one hydroxy.

More preferably, the ring A is unsubstituted or substituted with one substituent selected from fluorine, (3-fluoro-2-hydroxy)propoxy, or (3-fluoro-2-hydroxy)-1,2-dimethyl-propoxy.

R¹ is a halogen atom, a -C (=O)-lower alkyl group (the alkyl group each independently may be optionally substituted with one or more substituents selected from NR^{a}R^{b}, halogen and hydroxy), a lower alkyl group (the alkyl group each independently may be optionally substituted with one or more substituents selected from halogen and hydroxy), a -O-lower alkyl group (the alkyl group each independently may be optionally substituted with one or more substituents selected from halogen and hydroxy), or a group represented by a formula: 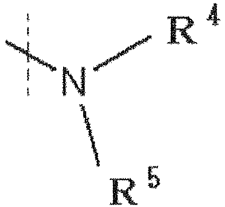 wherein
R⁴ and R⁵ each independently represents a hydrogen atom, a lower alkyl group (the alkyl group each independently may be optionally substituted with one or more substituents selected from halogen and hydroxy) or a cycloalkyl group, or R⁴, R⁵ and the nitrogen atom to which they are attached are taken together to form a 3- to 8-membered nitrogen-containing aliphatic ring (one or more carbon atoms constituting the nitrogen-containing aliphatic ring each independently may be optionally replaced by a nitrogen atom, a sulfur atom or an oxygen atom, and when a carbon atom is replaced by a nitrogen atom, the nitrogen atom may be optionally substituted with lower alkyl), or
R⁴ and the nitrogen atom to which it is attached are taken together with ring A to form a 8- to 16-membered nitrogen-containing fused bicyclic ring (one or more carbon atoms constituting the nitrogen-containing fused bicyclic ring each independently may be optionally replaced by a nitrogen atom, a sulfur atom or an oxygen atom, and when a carbon atom is replaced by a nitrogen atom, the nitrogen atom may be optionally substituted with one or two lower alkyl groups), and R⁵ represents a hydrogen atom, a lower alkyl group (the alkyl group each independently may be optionally substituted with one or more substituents selected from halogen and hydroxy), or a cycloalkyl group.

Preferably, the R¹ is a group represented by formula: 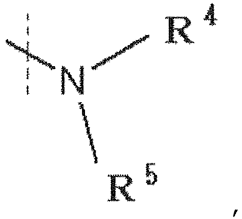 wherein,
R⁴ and R⁵ each independently represents a hydrogen atom, a lower alkyl group (the alkyl group each independently may be optionally substituted with one or more substituents selected from halogen and hydroxy) or a cycloalkyl group,

The "lower alkyl group" represented by R⁴ and R⁵ means the same groups as those in the lower alkyl group defined above. Among these groups, a linear or branched alkyl group having 1 to 3 carbon atoms, that is, a methyl group, an ethyl group and a propyl group are preferable, and the alkyl group each independently may be optionally substituted with one or more substituents selected from halogen and hydroxy.

The "cycloalkyl group" represented by R⁴ and R⁵ means the same groups as those in the cycloalkyl group defined above. Among these groups, a cycloalkyl group having 3 carbon atoms, that is, a cycloalkyl group is preferable.

It is particularly preferable that R⁴ is a hydrogen atom, and R⁵ is a lower alkyl group (for example, a methyl group, an ethyl group, or a propyl group is preferable, and a methyl group is more preferable).

Specific examples of the 3- to 8-membered nitrogen-containing aliphatic ring formed by taking R⁴, R⁵ and the nitrogen atom to which they are attached together (one or more carbon atoms constituting the nitrogen-containing aliphatic ring each independently may be replaced by a nitrogen atom, a sulfur atom or an oxygen atom, and in the case the carbon atom is replaced by a nitrogen atom, the nitrogen atom may be optionally substituted with a lower alkyl group) include, for exmaple, groups of formula: 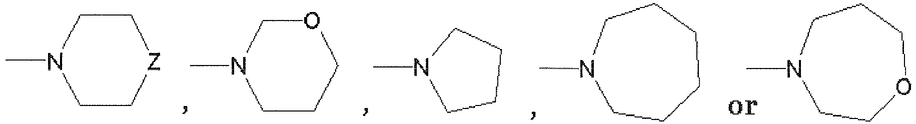 wherein, Z is O, S, CH₂ or NR^{e}, and R^{e} represents a hydrogen atom or a C₁₋₄ alkyl group. Among these groups, a morpholino group, a piperazine group and a 4-methyl-piperazine group are preferable.

Specific examples of the 8- to 16-membered nitrogen-containing fused bicyclic ring formed by taking R⁴ and the nitrogen atom to which it is attached together with ring A (one or more carbon atoms constituting the nitrogen-containing fused bicyclic ring may be replaced by a nitrogen atom, a sulfur atom or an oxygen atom and, in case the carbon atom is replaced by a nitrogen atom, the nitrogen atom may be optionally substituted with one or two lower alkyl groups) include groups of formula: 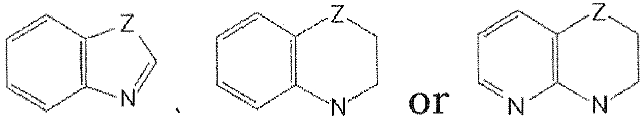 wherein, Z is O, S, CH₂ or NR^{e}, and R^{e} represents a hydrogen atom or a C₁₋₄ alkyl group. Among these groups, a formula: 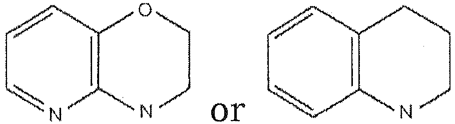 is particularly preferable.

R² and R³ each independently represents a halogen atom, OH, COOH, SO₃H, NO₂, SH, NR^{a}R^{b} or a lower alkyl group (the alkyl group each independently is substituted with one or more substituents selected from halogen and hydroxy), or a -O-lower alkyl group (the alkyl group each independently may be substituted with one or more substituents selected from halogen and hydroxy).

Regardless of the above-mentioned R¹, R², R³ and R⁶, at least one of the R¹, R², R³ and R⁶ represents a group represented by formula: 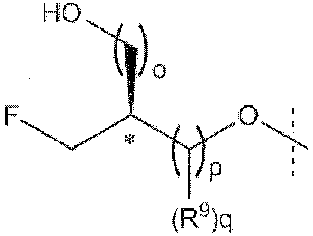 wherein,
the symbol * represents an asymmetric center. Optionally, the other groups are as the same as defined above,
R⁹ each independently represents a lower alkyl group (the alkyl group each independently may be optionally substituted with one or more substituents selected from halogen and hydroxy),
o is an integer of 0 to 1,
p is an integer of 0 to 1,
q is an integer of 0 to 2, and
each line that the above dotted line intersects with means a bond to the other structural moiety of the above general formula (I).

At least one of the R¹, R², R³ and R⁶, for example, the R² represents preferably a group represented by the above-mentioned formula: 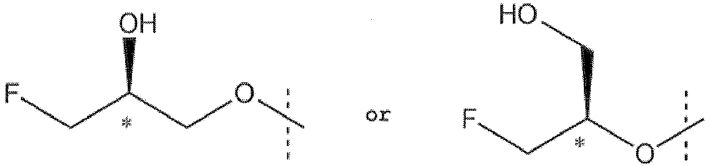 wherein,
o is an integer of 0 to 1,
p is an integer of 0 to 1, and
q is an integer of 0.

The group represented by formula: 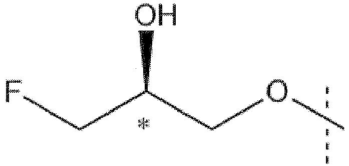 is more preferable.

Here, the compound of the present invention is an S-form having as chiral center an asymmetric carbon in the substituent represented by formula:

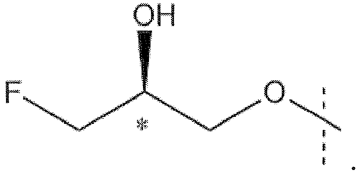

R^{a} and R^{b} each independently represents a hydrogen atom or a lower alkyl group (the alkyl group each independently may be optionally substituted with one or more substituents selected from halogen and hydroxy). Preferred R^{a} and R^{b} is a hydrogen atom.
m is an integer of 0 to 4, and preferably 1.
n is an integer of 0 to 2, and preferably 0.

As shown in Examples, the compound of the formula (I) is highly specific to tau, and also has high brain uptake, and further any compound not bound to tau rapidly clears from the brain. Also, the compound of the formula (I) is a compound having very high safety, which has low or non-recognized bone-seeking properties and low or non-recognized toxicity. Accordingly, the diagnosis of tauopathy can be carried out using the compound of formula (I) as a probe against tau, and also the treatment and/or prevention of tauopathy can be carried out by using the compound of the formula (I). Particularly, the compound of formula (I) is suited for imaging diagnosis of tauopathy, particularly imaging diagnosis using PET. Accordingly, it becomes possible to carry out accurate diagnosis, effective treatment and prevention in the early stages of tauopathy, particularly Alzheimer's disease, using the compound of formula (I).

A conformational disease is a disease in which a protein having a specific β-sheet structure accumulates, and there are various diseases characterized by deposition of an insoluble fibrillar protein to various internal organs and tissues. These diseases include Alzheimer's disease, Pick's disease, progressive supranuclear palsy (PSP), corticobasal degeneration, prion disease, dementia with Lewy bodies, Parkinson's disease, Huntington's disease, spinal and bulbar atrophy, dentate-rubro-pallido-luysian atrophy, Spinocerebellar Degeneration, Machado-Joseph Disease, Amyotrophic Lateral Sclerosis (ALS), Down's syndrome, Pick's disease, FTDP-17 (Frontotemporal Dementia and Parkinsonism linked to Chromosome 17), LNTD (Limbic Neurofibrillary tangles Dementia), Sudanophilic Leukodystrophy, amyloidosis and the like.

In the present invention, the conformational disease preferably means disease (tauopathy) having a cardinal symptom such as intracerebral accumulation of tau protein. Tauopathy includes Alzheimer's disease, Pick's disease, progressive supranuclear palsy (PSP), corticobasal degeneration, and the like.

In order to disclose the compound of a formula (I'): 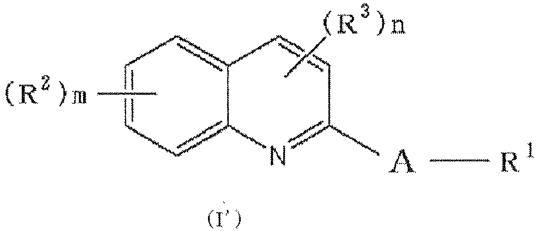 [wherein each symbol is the same as defined above] which is a precursor of the compound of the formula (I) of the present invention in more specifically, various symbols used in the formula (I') is described by way of specific examples. Here, each of the symbol of A, R¹, R², R³, and R⁶ is the same as defined in formula (I), and the specific structural example thereof is indicated above.

In one embodiment, at least one of the R¹, R², R³ and R⁶ represents a NR^{a}R^{b} (the R^{a} and R^{b} are as the same as defined above, and among them, when they represent an lower alkyl group, they each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group protected with a protecting group for hydroxy), a lower alkyl group (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group protected with a protecting group for hydroxy), and -O-lower alkyl (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group protected with a protecting group for hydroxy). Preferably, at least one of the R¹, R², R³ and R⁶ is -O-lower alkyl (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group protected with a protecting group for hydroxy).

In one embodiment, at least one of the R¹, R², R³ and R⁶ represents a group represented by formula: 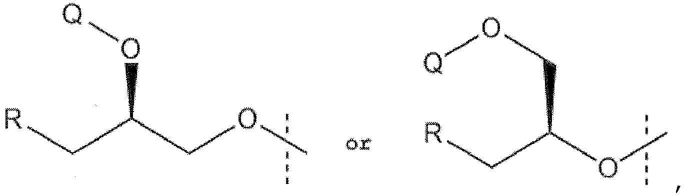 or R¹ represents a group represented by formula: 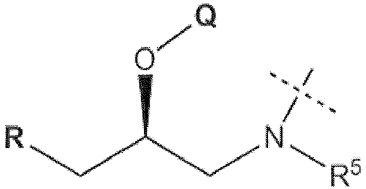 wherein,
R⁵ is the same as defined above.

Further preferably, when A represents a group represented by formula: 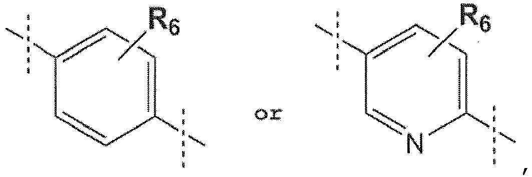 the R² represents a group represented by formula:

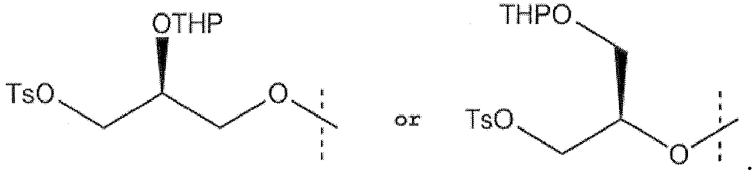

The substituent Q is a protecting group for a hydroxy group that has a resistance against a nucleophilic substitution by fluorine anion and may be removed under acidic or alkali condition, and includes, for example, a 2-tetrahydropyranyl (2-THP) group, a methoxymethyl group, a 2-methoxyetoxymethyl group, an ethoxyethyl group, an acetyl group, and a pivaloyl group. The substituent R is a functional group that works as a leaving group against a nucleophilic substitution by fluorine anion, and includes, for example, a p-toluenesulfonyloxy group, a methanesulfonyloxy group, a chloromethanesulfonyloxy group, and a trifluoromethanesulfonyloxy group.

The compound represented by formula (I') as the precursor of the compound represented by formula (I) of the present invention contains an asymmetric carbon in either a substituent of formula: 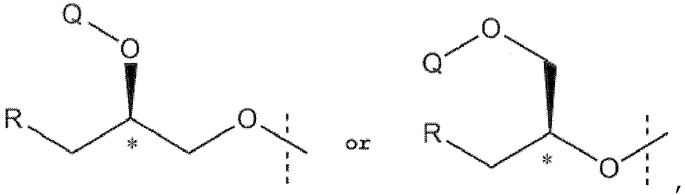 a substituent of formula: 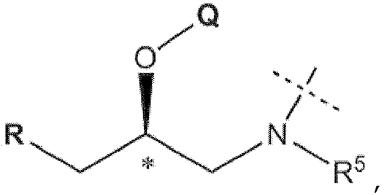 or a substituent of formula: 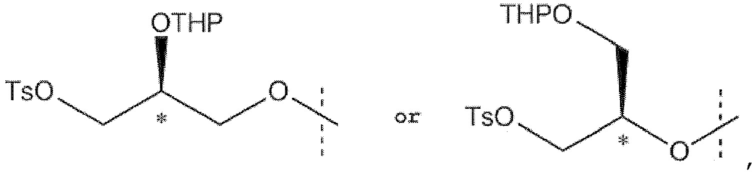 which is thus a S-form compound.

m is an integer of 0 to 4. Preferably, m is 0.
n is an integer of 0 to 2. Preferably, n is 0.

The compound of formula (I') may be used as a precursor of the compound of formula (I). Methods to convert the compound of formula (I') into the compound of formula (I) are well known to persons having ordinary skill in the art, and the compound of formula (I) may be thus easily prepared.

Salts of the compound of the present invention are also included in the present invention. The salt can be produced in accordance with a conventional method using the compound of a formula (I) or (I') provided by the present invention.

Specifically, when the compound of the formula (I) or (I') has, for example, a basic group derived from an amino group, a pyridyl group and the like in the molecule, the compound can be converted into a corresponding salt by treating with an acid.

Examples of the acid addition salt include hydrohalide salts such as hydrochloride, hydrofluoride, hydrobromide and hydroiodide; inorganic acid salts such as nitrate, perchlorate, sulfate, phosphate and carbonate; lower alkyl sulfonic acid salts such as methanesulfonate, trifluoromethanesulfonate and ethanesulfonate; aryl sulfonic acid salts such as benzenesulfonate and p-toluenesulfonate; organic acid salts such as fumarate, succinate, citrate, tartrate, oxalate and maleate; and acid addition salts with amino acids, such as glutamate and aspartate.

Also, when the compound of the present invention has an acidic group such as a carboxyl group in the molecule, the compound can also be converted into a corresponding pharmaceutically acceptable salt by treating with a base. Examples of the base addition salt include alkali metal salts such as sodium and potassium; alkali earth metal salts such as calcium and magnesium; ammonium salts; and base addition salts with organic bases such as guanidine, triethylamine and dicyclohexylamine.

Furthermore, the compound of the present invention may be present as a free compound, or arbitrary hydrate or solvate of a salt thereof.

In starting compounds and precursors which are converted into the compounds of the present invention by the method in the present description, existing functional groups such as amino, thiol, carboxyl and hydroxy groups may be optionally protected with a common conventional protecting group in preparative organic chemistry. The thus protected amino, thiol, carboxyl and hydroxy groups can be converted into free amino, thiol, carboxyl and hydroxy groups under mild conditions without causing breakage of a molecular framework or the other undesirable minor reaction.

The protecting group is inserted so as to protect the functional group from an undesirable reaction with a reaction component under the conditions used to perform a desired chemical conversion. Necessity and selection of the protecting group for a specific reaction are known to those skilled in the art, and depend on properties of the functional group to be protected (hydroxy group, amino group, etc.), structure and stability of the molecule with the substituent constituting a part thereof, and reaction conditions. Examples of the protecting group include OTs, OTHP, methoxymethyl and OAc. The protecting group is preferably a protecting group which is eliminated under acidic conditions.

In the diagnosis of tauopathy, the compound of the present invention can be used as a probe without labeling. For example, the presence or absence of the portion to be stained may be examined by bringing the compound of the present invention into contact with a biopsy tissue sample. However, it is common to use the labeled compound of the present invention as a probe for the diagnosis of tauopathy. Examples of label include a fluorescent substance, an affinity substance, an enzyme substrate, a radioactive nuclide and the like. A probe labeled with a radioactive nuclide is usually used in diagnostic imaging of tauopathy. It is possible to label the compound of the present invention with various radioactive nuclides by the methods which are well known in the art. For example, ³H, ¹⁴C, ³⁵S, ¹³¹I and the like are radioactive nuclides which have been used for a long time, and are often utilized in vivo. General requirements for diagnostic imaging probes and means for their detection are to permit in vivo diagnosis, to cause less harm to patients (particularly, to be non-invasive), to have a high sensitivity of detection, to have an appropriate half-life (to have an appropriate period of time for preparing the labeled probes and for diagnosis) and the like. In certain embodiments, positron emission tomography (PET) or single-photon emission computed tomography (SPECT) are used. Among them, PET, which detects two γ-rays emitting in opposite directions from a positron emitting nuclide by means of simultaneous counting with a pair of detectors, provides information which is excellent in resolution and quantification and thus is preferable. For SPECT, the compound of the present invention can be labeled with a γ-ray emitting nuclide such as ^{99m}Tc, ¹¹¹In, ⁶⁷Ga, ²⁰¹T1, ¹²³I, ¹³³Xe and the like. ^{99m}Tc and ¹²³I are often used for SPECT. For PET, the compound of the present invention can be labeled with a positron emitting nuclide such as ¹¹C, ¹³N, ¹⁵O, ¹⁸F, ^{34m}Cl, ⁴⁵Ti, ⁴⁸V, ⁶⁰Cu, ⁶¹Cu, ⁶²Cu, ⁶⁴Cu, ⁶⁶Ga, ⁷⁶Br, ⁸⁹Zr, ^{94m}Tc, and ¹²⁴I and the like. Among positron emitting nuclides, ¹¹C, ¹³N, ¹⁵O and ¹⁸F are preferable, ¹⁸F and ¹¹C are more preferable, ¹⁸F is particularly preferable, from the viewpoint of having an appropriate half-life, the ease of labeling and the like. Although the position of labeling the compound of the present invention with a radiation emitting nuclide such as a positron emitting nuclides or γ-ray emitting nuclide can be any position, labeling may preferably be carried out at an alkyl group and on phenyl ring in the compound. Such labeled compounds of the present invention are also included in the present invention. For example, when the compound of the present invention is labeled with ¹⁸F, any position of the side chain may be labeled with ¹⁸F, or hydrogen on the ring may be substituted with ¹⁸F. For example, hydrogen contained in any one of alkyl substituents may be substituted with ¹⁸F. Also, when the compound of the present invention is labeled with ¹¹C, carbon contained in any one of alkyl substituents in the side chain may be substituted with ¹¹C. Although it is obvious to a person with an ordinary skill in the art, m of ^{99m}Tc denotes a nuclear isomer in a quasi-stable state.

Radionuclides used in the compounds according to the present invention are generated on an instrument termed cyclotron or generator. A person with an ordinary skill in the art can select methods and instruments for production depending upon nuclides to be produced. Nuclides thus produced can be used to label the compounds of the present invention.

Methods of producing labeled compounds, which have been labeled with these radionuclides, are well known in the art. Typical methods include chemical synthesis, isotope exchange, and biosynthesis processes. Chemical synthesis processes have been traditionally and widely employed, and are essentially the same as usual chemical synthesis processes, except that radioactive starting materials are used. Various nuclides are introduced into compounds by these chemical processes. Isotope exchanging processes are processes by which ³H, ³⁵S, ¹²⁵I or the like contained in a compound of a simple structure is transferred into compound having a complex structure, thereby obtaining a compound having a complex structure that has been labeled with these nuclide. Biosynthesis processes are processes by which a compound labeled with ¹⁴C, ³⁵S or the like is given to cells such as microorganisms to obtain its metabolites having these nuclide introduced therein. In the case of ¹⁸F, a chemical form as fluorine anion, which can be prepared in large amount thereof by cyclotron with high specific radioactivity, is often used in a labeling synthesis, and a salt of ¹⁸F anion with increased nucleophilicity is used as a labeling agent in a nucleophilic substitution with a compound having a leaving group (label precursor) to give the ¹⁸F labeled compound of the present invention. The nucleophilic substitution is preferably carried out in an organic solvent, and is more preferably reacted in anhydrous high polar solvent (such as DMSO, acetonitrile, and DMF). The reaction temperature is not particularly limited, and may either be at room temperature or with heating, for example, a temperature near the boiling point of the used reaction solvent. The reaction period may be within a range of a few minutes to a few days, and the reaction may be achieved, for example, within a range of a few minutes to a few hours. The step comprising introduction of the radionuclide should be as close as possible to the end of the radiosynthesis.

After the nucleophilic substitution, the protecting group for hydroxy group in the obtained product may be removed under acidic or alkali condition to obtain the desired ¹⁸F⁻ labeled compound.

Also, the solution comprising the ¹⁸F⁻ labeled compound of the present invention may be contacted with an ion-exchange resin supported by ¹⁸F⁻ to obtain ¹⁸F⁻ labeled compound of the present invention.

With respect to the labeling position, similarly to a usual synthesis, synthetic schemes can be designed depending upon the purpose, so that a label can be introduced at a desired position. Such design is well known to a person with ordinary skill in the art.

When utilizing positron emitting nuclides such as ¹¹C, ¹³N, ¹⁵O and ¹⁸F, which have relatively short half-lives, for example, it is also possible to generate a desired nuclide from a (highly) small-sized cyclotron placed in a facility such as hospital, which in turn is used to label a desired compound at its desired position by any of the above-described methods, followed by carrying out immediately diagnosis, examination, treatment or the like.

These methods well known to a person with ordinary skill in art enable one to carry out labeling by introducing a desired nuclide into the compound of the present invention at its desired position.

The labeled compound of the present invention may be administered to subjects locally or systemically. Routes for administration include intradermal, intraperitoneal, intravenous, intra-arterial injections or infusions into the spinal fluid and the like, and can be selected depending on factors such as the disease type, nuclide used, compound used, the condition of the subject, the site to be examined. The site to be examined can be investigated with means such as PET, SPECT by administering the probe of the present invention, followed by the elapse of a sufficient time to allow its binding to tau protein and decay. These procedures can be selected as appropriate depending on factors such as the disease type, nuclide used, compound used, the condition of the subject, the site to be examined.

The dose of the compound of the present invention, which has been labeled with a radionuclide, varies depending on the disease type, nuclide used, compound used, the age, physical condition, and gender of the subject, the degree of the disease, the site to be examined and the like. In particular, sufficient care has to be taken in connection with radioactive exposure to the subject. For example, the amount of radioactivity of the compound labeled with a positron emitting nuclide such as ¹¹C, ¹³N, ¹⁵O and ¹⁸F of the present invention, is usually within a range from 3.7 megabecquerels to 3.7 gigabecquerels, and preferably from 18 megabecquerels to 740 megabecquerels.

The compound of the present invention or a salt or solvate thereof is suited for use in a treatment method of tauopathy, a diagnosis method, a composition for treatment, a composition for diagnosis, a kit for diagnosis, use for the production of these compositions and kits, and other uses, which will be described below. The compounds or salts or solvates thereof exemplified in the above description about the compounds of formulae (I) to (VI) are preferable, and those included in the compound of formula (I) or a salt or solvate thereof are particularly preferable. Among the compounds of the present invention, a compound of formula (I) wherein R¹, R², R³, R⁴, R⁵, or R⁶ represents formula: 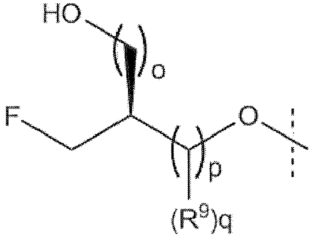 wherein,
each symbol is as the same as defined above, preferably, a compound represented by formula: 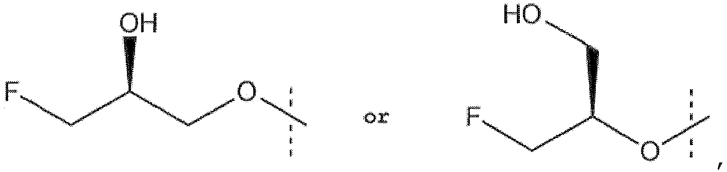 particularly preferably, a compound represented by formula: 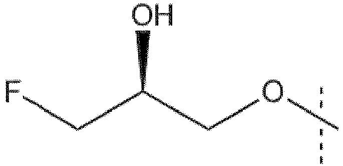 has a property in which a transition into brain is excellent and also non-binding compound disappears rapidly from the brain, and thus the positron nuclide, preferably the ¹⁸F labeled compound is suited as a PET probe for sensitively imaging tau protein accumulated in the brain. These compounds are also suited for administration to the human body because of considerably less or scarce accumulation in bone.

The present invention provides a composition containing the compound of the present invention for diagnostic imaging of tauopathy. The composition of the present invention contains the compound of the present invention and a pharmaceutically acceptable carrier. It is preferred that the compound of the present invention in the composition is labeled. Although various labeling methods are possible as described above, labeling with radionuclides (in particular, positron emitting nuclides such as ¹¹C, ¹³N, ¹⁵O and ¹⁸F for PET) is desirable for in vivo image diagnosis applications. It is preferable from their purposes that the form of the composition of the present invention is one allowing injection or infusion. Accordingly, a pharmaceutically acceptable carrier is preferably liquid and examples thereof include, but are not limited to, aqueous solvents such as potassium phosphate buffer, physiological saline, ringer solution and distilled water; and non-aqueous solvents such as polyethylene glycol, vegetable oil, ethanol, glycerin, dimethyl sulfoxide and propylene glycol. A mixing ratio of the carrier to the compound of the present invention can be appropriately selected depending on the site of application, detection means and the like, and is usually from 100,000 : 1 to 2:1, and preferably from 10,000: 1 to 10: 1. The composition of the present invention may further contain known antimicrobials (for example, antimicrobial drug, etc.), local anesthetics (for example, procaine hydrochloride, etc.), buffers (for example, Tris-hydrochloride buffer, HEPES buffer, etc.), osmolytes (for example, glucose, sorbitol, sodium chloride, etc.) and the like.

Furthermore, the present invention provides a kit for image diagnosis of tauopathy, containing the compound of the present invention as the essential ingredient. Usually, the kit is a package in which each of the components such as the labeled compound of the present invention, or its labeled precursor, a solvent for dissolving the compound, a reagent used for labeling synthesis or a solution of the same, a buffer, an osmoregulatory agent, an antimicrobial, a local anesthetic, a solubilizing agent, a radiolysis- preventing agent are packaged separately into respective containers, or some of the components are packaged together into respective containers. The compound of the present invention may be unlabeled or labeled. When not labeled, a kit may contain a labeled precursor of the present invention, and the labeled compound of the present invention can be prepared using the labeled precursor by a labeling synthesis, prior to use, according to usual methods as described above. In addition, the compound of the present invention may be presented as a solid, such as a lyophilized powder, or in solution in appropriate solvents. Solvents may be similar to carriers used in the above composition of the present invention. Each of the components such as a buffer, an osmoregulatory agent, an antimicrobial, a local anesthetic, also may be similar to those used in the above composition of the present invention. While various containers can be selected as appropriate, they may be of shapes suitable for carrying out the introduction of a label into the compound of the present invention, or of light-shielding materials, depending on the nature of compounds, or take forms such as vials or syringes, so as to be convenient for administration to patients. The kit may also contains, as appropriate, container or instruments for labeling synthesis, such as vials, syringe, three-way stopcock, needle, solid-phase extraction cartridge, sterilizing filter and the others. The kit may further contains, as appropriate, tools necessary for diagnosis, for example, syringes, an infusion set, or device for use in a PET or SPECT apparatus. The kit usually has its instructions attached thereto.

Furthermore, the compounds of the present invention specifically bind to tau protein, and thus can be also used, for example, for detecting and quantifying tau protein with or without labeling by contacting with sample specimens in vitro. For example, the compounds of the present invention can be used for staining tau protein in microscopic specimens, for colorimetric determination of tau protein in samples, or for quantifying tau protein using a scintillation counter. Preparation of a microscope specimen and staining using the compound of the present invention can be carried out by a conventional method known to a person with an ordinary skill in the art.

As described above, the compounds of the present invention are highly specific to tau protein. Therefore, the compounds of the present invention are useful, for example, for studies of disease with tau protein accumulation or in their diagnosis before and after death, and could be useful, for example, as agents for staining neurofibrillary tangles in brain sections of Alzheimer's disease patients. Staining of specimens, for example, brain sections using the compounds of the present invention can be carried out in a conventional method known to a person of ordinary skill in the art.

As described above, among the compounds of the present invention, compounds having, as R¹, R², R³, R⁴, R⁵, or R⁶ in formula (I), a compound represented by formula: 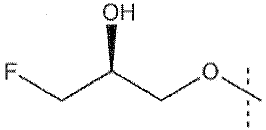 has a property in which a transition into brain is excellent and also non-binding compound may be disappeared rapidly from the brain, and an accumulation into brain is thus low, and also an accumulation into bone is considerably less or scarce. Accordingly, these compounds of the present invention are not only considerably safe probes for the diagnosis of tauopathy, but also exhibit high safety even when used as remedies or preventives described hereinafter.

Accordingly, the present invention is directed to a composition for staining of amyloid β protein, particularly tau in a sample, containing the compound of the present invention or a pharmaceutically acceptable salt or solvate thereof, and a kit for staining of amyloid β protein, particularly tau in a sample, containing the compound of the present invention or a pharmaceutically acceptable salt or solvate thereof as essential ingredients. Furthermore, the present invention is directed to a method of staining amyloid β protein, particularly tau in a sample, the method comprising using the compound of the present invention or a pharmaceutically acceptable salt or solvate thereof. Samples suited for above staining are brain sections.

As described above, it has been found that neurotoxicity is present in amyloid β protein or tau of a β-sheet structure. It is considered that the compound of the present invention is specifically bound to amyloid β protein of a β-sheet structure, particularly tau, and thus neurotoxicity is inhibited. Accordingly, it is considered that the compound of the present invention serves as remedies or preventives for causes of a disease, particularly tauopathy, for example, Alzheimer's disease since protein itself has a β-sheet structure.

Accordingly, the present invention provides:
a method of treating and/or preventing diseases with amyloid β protein accumulation, particularly tauopathy, the method comprising administering a compound of the formula (I) or a salt or solvate thereof;
a method of diagnosing diseases with amyloid β protein accumulation, particularly tauopathy, the method comprising using a compound of the formula (I) or a salt or solvate thereof; and
a use of a compound of the formula (I) or a salt or solvate thereof for the production of a composition or kit for the treatment, prevention or diagnosis of diseases with amyloid β protein accumulation, particularly tauopathy.

Forms of such pharmaceutical compositions are not limited in particular, but liquid formulations, particularly formulations for injection, are preferable. Such formulations for injection can be infused directly into the brain, or alternatively the above pharmaceutical compositions can be formulated for intravenous injection or drip and administered, since the compounds of the present invention have high permeability through the blood-brain barrier, as shown in the Examples. Such liquid formulations can be prepared by methods well known in the art. Solutions can be prepared, for example, by dissolving the compound of the present invention in an appropriate carrier, water for injection, physiological saline, Ringer's solution or the like, sterilizing the solution through a filter or the like, and then filling the sterilized solution into appropriate containers, for example, vials or ampules. Solutions also can be lyophilized and when used, reconstituted with an appropriate carrier. Suspensions can be prepared, for example, by sterilizing the compound of the present invention, for example, by exposure to ethylene oxide, and then suspending it in a sterilized liquid carrier.

When such a pharmaceutical composition is used in a liquid formulation, particularly a formulation for injection, an injection can be prepared by adding a solubilizing agent to a quinoline derivative according to the present invention.

It is possible to use, as the solubilizing agent, nonionic surfactants, cationic surfactants, amphoteric surfactants and the like used in the art. Among these solubilizing agents, Polysorbate 80, polyethylene glycol, ethanol or propylene glycol is preferable, and Polysorbate 80 is more preferable.

The amount of the compounds of the present invention to be administered to a human subject in the above treatment method, prevention method and use varies depending on the condition, gender, age, weight of the patient and the like, and is generally within a range from 0.1 mg to 1 g, preferably from 1 mg to 100 mg, and more preferably from 5 mg to 50 mg, per day for adult humans weighing 70 kg. It is possible to conduct a treatment with such a dose for a specified period of time, followed by increasing or reducing the dose according to the outcome.

Furthermore, the compound of the present invention or a pharmaceutically acceptable salt or solvate thereof can also be used as a probe for the diagnosis of conformational disease, particularly tauopathy, preferably an image diagnosis probe labeled with a radiation nuclide. Furthermore, the compounds of the present invention have the effect for the treatment and/or prevention of conformational disease, particularly tauopathy.

Accordingly, the present invention is also directed to:
a compound of the present invention used as an image diagnosis probe of conformational disease, particularly tauopathy, or a salt or solvate thereof;
a composition or kit for image diagnosis of conformational disease, particularly tauopathy, comprising the compound of the present invention or a salt or solvate thereof;
a pharmaceutical composition for the prevention and/or treatment of conformational disease, particularly tauopathy, comprising a compound of the present invention or a pharmaceutically acceptable salt or solvate thereof, and a pharmaceutically acceptable carrier;
a method of diagnosing conformational disease, particularly tauopathy, the method comprising using a compound of the present invention or a pharmaceutically acceptable salt or solvate thereof;
use of a compound of the present invention or a pharmaceutically acceptable salt or solvate thereof for the diagnosis of conformational disease, particularly tauopathy;
a method of preventing and/or treating conformational disease, particularly tauopathy, the method comprising administering a compound of the present invention or a pharmaceutically acceptable salt or solvate thereof to the subject;
use of a compound of the present invention or a pharmaceutically acceptable salt or solvate thereof for the prevention and/or treatment of conformational disease, particularly tauopathy; and
use of a compound of the present invention in the production of a pharmaceutical composition for the prevention and/or treatment of conformational disease, particularly tauopathy.

The dose of the compounds of the present invention to be administered to a human subject in the above treatment methods and prevention methods is as described above.

Further, the present invention provides a kit for preparing a compound of the present invention or a pharmaceutically acceptable salt or solvate thereof, the kit comprising a compound of the present invention or a pharmaceutically acceptable salt or solvate thereof, a labeling agent, and optionally instructions for labeling the compound. The labeling agent is, for example, a radioactive nuclide or a positron emitting nuclide. The radioactive nuclide is, for example, a γ-ray emitting nuclide. The positron emitting nuclide is selected from, for example, the group consisting of ¹¹C, ¹³N, ¹⁵O, ¹⁸F, ^{35m}Cl, ⁷⁶Br, ⁴⁵Ti, ⁴⁸V, ⁶⁰Cu, ⁶¹Cu, ⁶²Cu, ⁶⁴Cu, ⁶⁶Ga, ⁸⁹Zr, ^{94m}Tc and ¹²⁴I. Preferably, the positron emitting nuclide is ¹¹C or ¹⁸F. The labeling agent is an agent in which the radioactive nuclide has such chemical form as being suitable for labeling the compound, and is known to those skilled in the art.

### EXAMPLES

Hereinafter are provided Examples of non-limiting compounds of the present invention as well as Preparation Examples, Reference Examples and Use Examples of the same, which serve to illustrate embodiments of the present invention.

First, the non-limiting examples of compounds of the present invention are shown below.

**Table 1**

| | |
|---|---|
| THK-5105S | 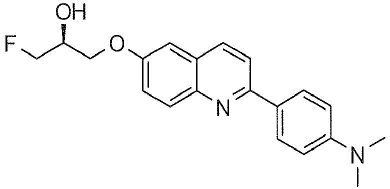 |
| THK-5121S (Intermediate, label precursor) | 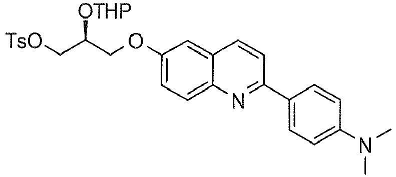 |
| THK-5117S | 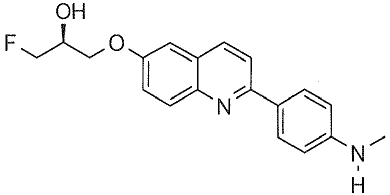 |
| THK-5119S (Intermediate, label precursor) | 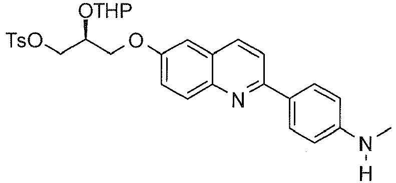 |
| THK-5151S | 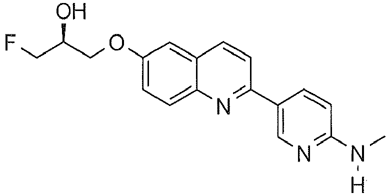 |
| THK-5152S (Intermediate, label precursor) | 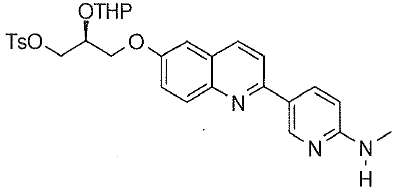 |

Next, a general synthesis method for the compound of the present invention is shown below, but is not limited thereto.

The optically active compound of the present invention can be prepared stereo specifically by using a chiral synthon having an optical activity of the compound of the present invention. Hereinafter, examples of the chiral synthon that can be used in a preparation of the compound of the present invention are shown.

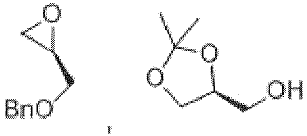

The chiral synthon can be prepared according to a general chemical synthesis method in an organic chemical field, or can be obtained as a commercial reagent.

An example of a general synthesis method of the compound of the present invention is shown below.

First, a chiral synthon is derivatized to a compound of formula (II) that can offer an optical activity of the compound of the present invention.

Next, the compound of formula (II) is reacted with a compound of formula (V) to prepare an intermediate compound of formula (V) (step i)). Here, a step of binding reaction of the chiral synthon to the compound of formula (V) may be carried out to convert the structure of a chiral side chain of the resulting compound into a compound of formula (V').

Further, after preparation of the intermediate compound of formula (V), the resulting compound may be reacted with the boron compound having -A-R¹ group that represented by formula (VI) or formula (VII) to prepare a desired compound of formula (I) or formula (I') (step ii)).

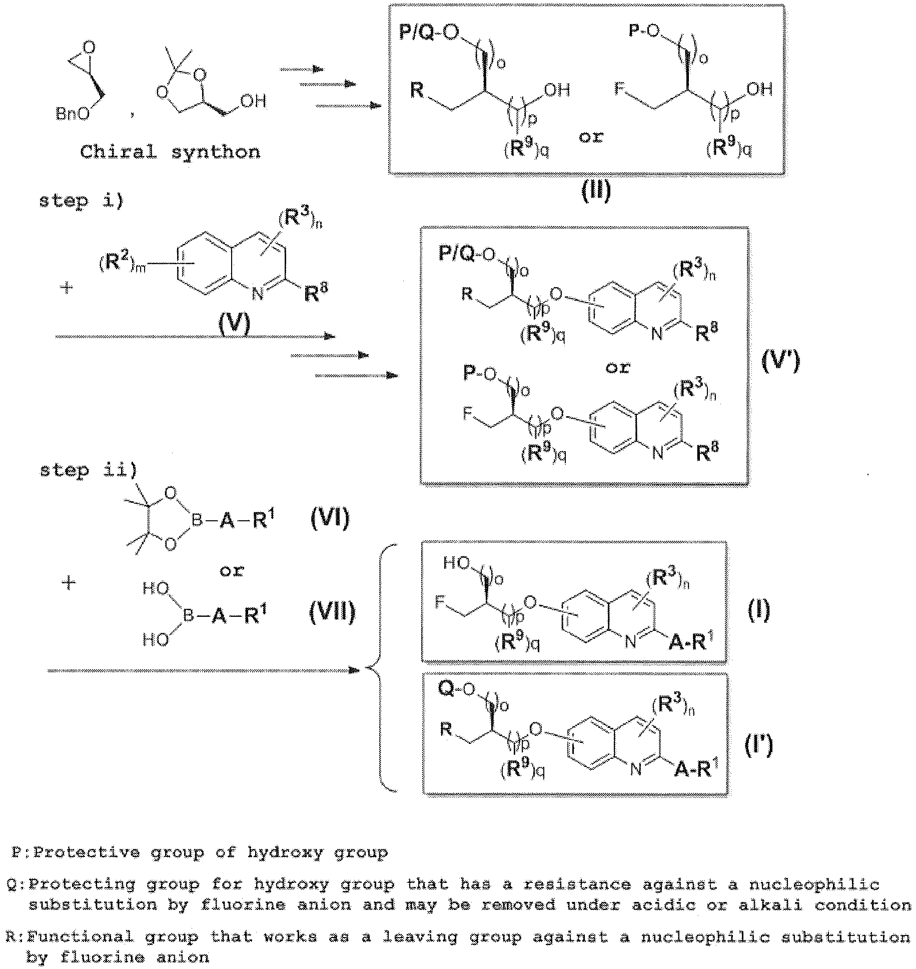

In the above-mentioned process, a procedure in each reaction step can be carried out according to a reaction condition (for example, reagent, reation temperature, reaction period) that is generally known in an organic chemical field.

Separately, the reaction sequences of the step i) and the step ii) in the above-mentioned process may be reversed.

First, a chiral synthon is derivatized to a compound of formula (II) that can offer an optical activity of the compound of the present invention.

Next, the compound of formula (V) is reacted with the boron compound having -A-R¹ group that represented by formula (VI) or formula (VII) to prepare an intermediate compound of formula (V'') (step i)).

Further, the intermediate compound of formula (V'') may be reacted with the compound of formula (II) to prepare the desired compound of formula (I) or formula (I') (step ii)).

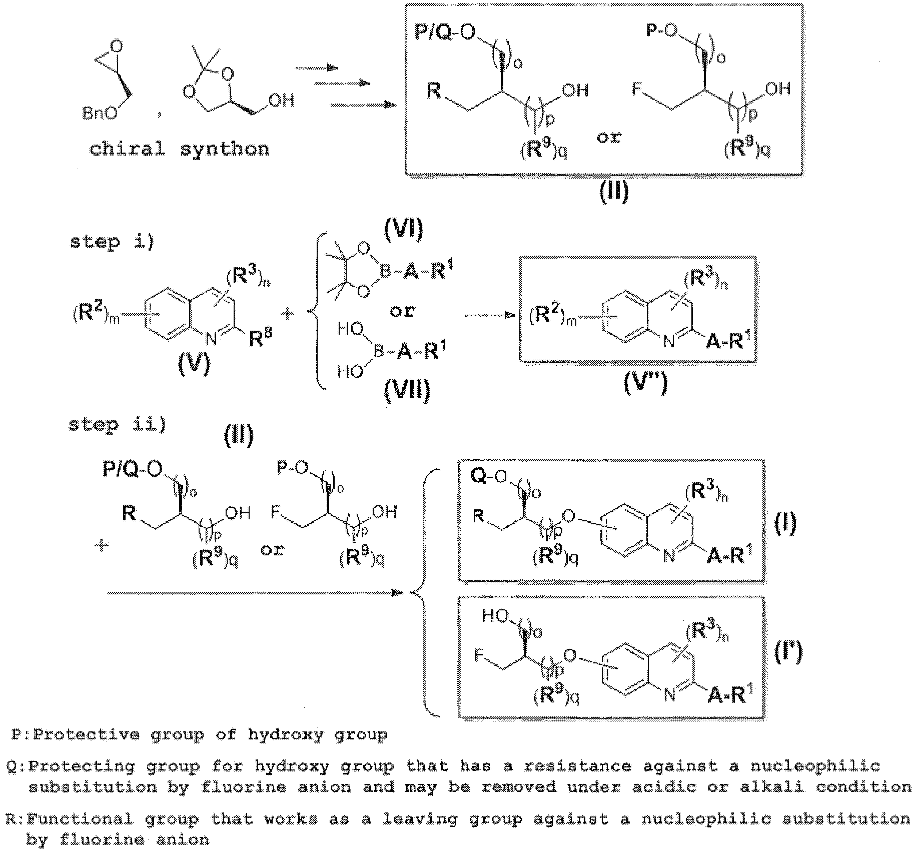

In the above-mentioned process, a procedure in each reaction step can be carried out according to a reaction condition (for example, reagent, reation temperature, reaction period) that is generally known in an organic chemical field. Here, when a compound of formula: 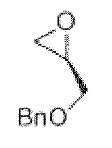 is used as a chiral synthon, effects such as a reduction in the number of reaction steps and an increase in yield through the overall synthesis steps.

Hereinafter, the synthesis method of the above-mentioned representive compounds is shown below, but is not limited thereto.

One example of a synthesis of THK-5105S is shown below. Synthesis of THK-5105S

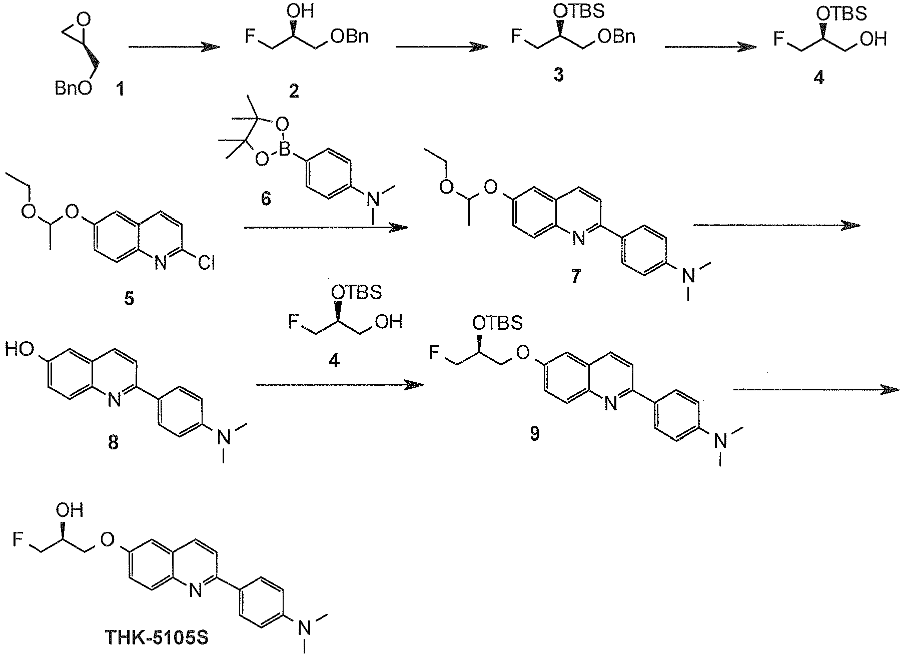

### Synthesis of Compound 2

A suspension of a compound 1 (25.02 g, 152 mmol), KHF₂ (23.8 g, 305 mmol), and Bu₄N⁺H₂F₃⁻ (4.95 g, 15.2 mmol) was stirred at 120°C for 6 hours. To the reaction solution was added water, and the mixture was extracted with ethyl acetate, and the extraction liquid was washed with water, and dried, and then the solvent was distilled off under reduced pressure. The residue was purified by silica gel column chromatography (eluting solvent: ethyl acetate/n-hexane=1/9) to obtain Compound 2 (9.98 g, 35 %) as colorless oil.

### Synthesis of Compound 3

To a solution of Compound 2 (9.97 g, 54.1 mmol) in N,N-dimethylformamide (100 mL) were added imidazole (4.05 g, 60 mmol) and t-butyl dimethyl silyl chloride (8.97g, 60 mmol), and the mixture was stirred at room temperature for 16 hours. To the reaction mixture was solution was added cooled water, and the mixture was extracted with ethyl acetate, and the extraction liquid was washed with water, dried, and the solvent was distilled off under reduced pressure. The residue was purified by silica gel column chromatography (eluting solvent: ethyl acetate/n-hexane=1/50) to obtain Compound 3 (14.77 g, 91 %) as colorless oil.

### Synthesis of Compound 4

To a solution of Compound 3 (14.76 g, 49.5 mmol) in tetrahydrofuran-water (190 mL-10 mL) was added 10% Pd-C (moisture of about 50%; 8.86 g), and the mixture was hydrogenated at room temperature under normal pressure for 4 days. The catalyst was removed by filtration and the solvent was distilled off under reduced pressure, and the residue was purified by silica gel column chromatography (eluting solvent: ethyl acetate/n-hexane=1/20, 1/9) to obtain Compound 4 (6.65 g, 64 %) as colorless oil.
¹H NMR (400MHZ, DMSO-d₆) δ 0.06 (3H, s), 0.06 (3H, s), 0.08 (9H, s), 3.25-3.33 (2H, m), 3.72-3.84 (1H, m), 4.16-4.46 (2H, m), 4.69
(1H, t, J=5.5Hz)

### Synthesis of Compound 7

To a solution of Compound 5 (2.00 g, 7. 95 mmol) and Compound 6 (2.16 g, 8.74 mmol) in 1,2-dimethoxyethane (65 mL) were added potassium carbonate (3.3 g, 23.9 mmol), water (1.38 mL) and tetrakistriphenylphosphine palladium (0.46 g, 0.398 mmol) under an argon atmosphere, and the mixture was stirred at 80°C for 24 hours. The reaction solution was allowed to return to room temperature, and thereto were added ethyl acetate and sodium sulfate, and the mixture was stirred for 5 minutes, and the mixture was filtered through Celite (trade mark) and washed with ethyl acetate. The filtrate liquid and the wash liquid were combined, and the solvent was distilled off, and the residue was purified by silica gel column chromatography (eluting solvent: ethyl acetate/n-hexane=1/9) to obtain Compound 7 (1.83 g, 68 %) as pale yellow solids.
mp 92-93 °C
ESI-MS m/z 337 [M+H]⁺

### Synthesis of Compound 8

To a solution of Compound 7 (1.82 g, 5.41 mmol) in chloroform (20 mL) was added dropwise trifluoroacetic acid (4 mL) under ice cooling, and the mixture was stirred at room temperature for 1 hour. To the reaction solution was added ice water, and the mixture was adjusted with aqueous potassium carbonate solution to pH 9, and the mixture was extracted with tetrahydrofuran. The extraction liquid was washed with water, dried, and the solvent was distilled off, and the residue was purified by silica gel column chromatography (eluting solvent: ethyl acetate/n-hexane = 1/2, ethyl acetate) to obtain Compound 8 (1.14 g, 80 %) as yellow crystals.
mp 273-275 °C
ESI-MS m/z 265 [M+H]⁺

### Synthesis of Compound 9

To a solution of Compound 8 (400 mg, 1.51 mmol), Compound 4 (760 mg, 3.63 mmol), triphenylphosphine (0.95 g, 3.63 mmol) in tetrahydrofuran (30 mL) was added dropwise an solution of diisopropyl azodicarboxylate (0.72 mL, 3.63 mmol) in tetrahydrofuran (10 mL) under ice cooling with stirring over 10 minutes, and the mixture was stirred under ice cooling for 1 hour and at room temperature for 3 days. The solvent of the reaction solution was distilled off under reduced pressure, and the residue was purified by silica gel column chromatography (eluting solvent: ethyl acetate/n-hexane = 1/9, 1/4), followed by washed with ethyl acetate/n-hexane (1/9) to obtain Compound 9 (563 mg, 82 %) as pale yellow solid.
mp 154-155 °C
ESI-MS m/z 455 [M+H]⁺

### Synthesis of THK-5105S

To a solution of Compound 9 (605 mg, 1.33 mmol) in chloroform (18 mL) was added dropwise trifluoroacetic acid (12 mL) under ice cooling, and thereto was added water (3 mL), and the mixture was stirred at room temperature for 3 days. To the reaction solution were added ice water and ethyl acetate, and the mixture was adjusted with aqueous potassium carbonate solution to pH 8, and the mixture was extracted with ethyl acetate. The extraction liquid was washed with water, dried, and the solvent was distilled off under reduced pressure, and the residue was purified by silica gel column chromatography (eluting solvent: ethyl acetate/n-hexane = 1/2, 1/1), and followed by recrystallization from ethyl acetate to obtain THK-5105S (394 mg, 87 %) as pale yellow crystals.
mp 178-179 °C
ESI-MS m/z 455 [M+H]⁺
¹H NMR (400 MHz, DMSO-d₆) δ 2.99 (6H, s), 4.06-4.20 (3H, m), 4.44-4.65 (2H, m), 5.55 (1H, br), 6. 83 (2H, d, J=9.1Hz), 7.35-7.40 (2H, m), 7.89 (1H, d, J=9.1 Hz), 7.98 (2H, d, J=8.8Hz), 8.09 (2H, d, J=8.8Hz), 8.22 (1H, d, J=8.8Hz)
IR (Nujol) 3409, 1616 cm⁻¹
APCI-MS m/z 341[M+H]⁺

Also, one example for another synthesis method of THK 5105S is shown below.

### Synthesis of THK-5105S

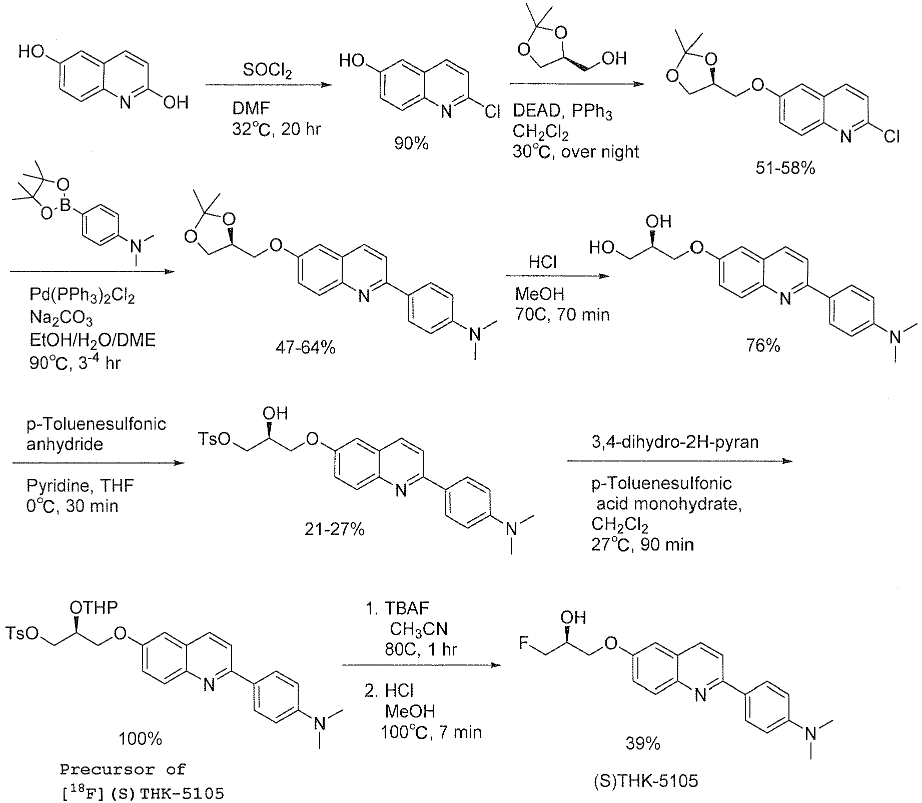

Hereinafter, one example of synthesis method of THK-5121S is shown.

### Synthesis of THK-5151S

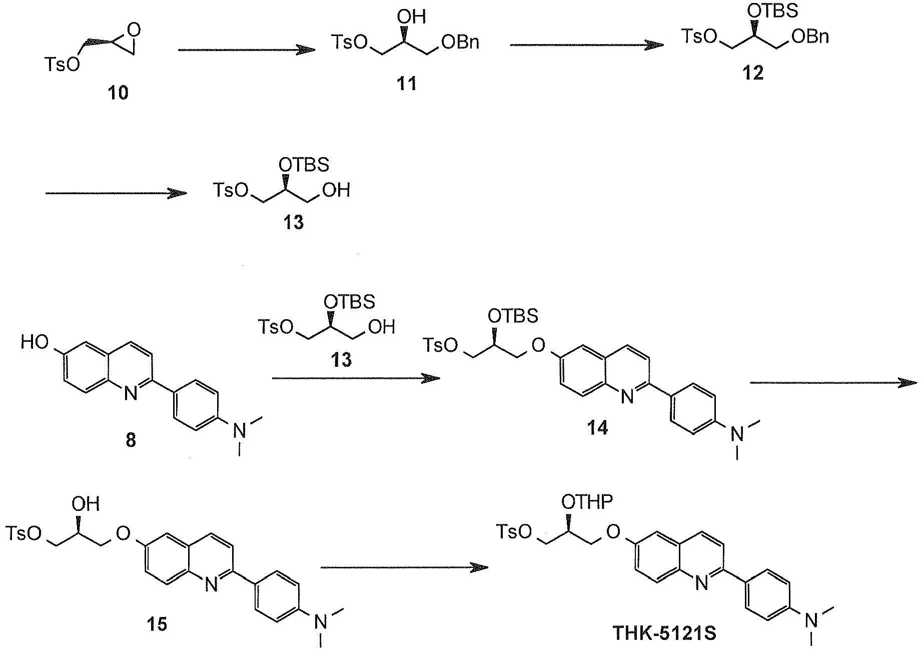

### Synthesis of Compound 11

To a solution of Compound 10 (25.26 g, 111 mmol) in chloroform (250 mL) was added benzyl alcohol (23. 93 g, 221 mmol), and to the mixture was added BF₃ ·Et₂O (25 drops), and the mixture was stirred at the same temperature for 1 day and at room temperature for 1 day. The reaction solution was neutralized with aqueous sodium hydrogen carbonate solution, and the organic layer was fractionated. The organic layer was dried, and the solvent was distilled off under reduced pressure, and the residue was purified by silica gel column chromatography (eluting solvent: ethyl acetate/n-hexane = 1/4) to obtain Compound 11 (35.17 g, 94%) as colorless oil.

### Synthesis of Compound 12

To a solution of Compound 11 (35.16 g, 105 mmol) in N,N-dimethylformamide (200 mL) were added imidazole (7.83 g, 115 mmol) and 50% t-butyl dimethyl silyl chloride/toluene (34.66 g, 115 mmol), and the mixture was stirred at room temperature for 3 days. To the reaction was added cool water, and the mixture was extracted with ethyl acetate, and the extraction liquid was washed with water, dried, and the solvent is distilled off under reduced pressure. The residue was purified by silica gel column chromatography (eluting solvent: ethyl acetate/n-hexane = 1/19, 1/9) to obtain Compound 12 (42.16 g, 89 %) as colorless oil.

### Synthesis of Compound 13

To a solution of Compound 12 (42.15 g, 93.5 mmol) in tetrahydrofuran-water (270 mL-30 mL) was added 10 Pd-C (moisture of about 50%; 16.9 g), and the mixture was hydrogenated at room temperature under normal pressure for 4 days. The catalyst was removed and the solvent was concentrated to about half volumes, washed with saturated saline, dried, and the solvent was distilled off under reduced pressure. The residue was purified by silica gel column chromatography (eluting solvent: ethyl acetate/n-hexane = 1/9, 1/4, 1/2) to obtain Compound 13 (11.12 g, 33 %) as colorless oil.
¹H NMR (400MHz, DMSO-d₆) δ 0.00 (3H, s), 0.02 (3H, s), 0.81 (9H, s), 2.43 (3H, s), 3.23-3.37 (2H, m), 3.78-3.84 (1H, m), 3.87 (1H, J=9.9, 6.6 Hz), 4.06 (1H, dd, J=9.6, 3.0 Hz), 4.78 (1H, t, J=5.5 Hz), 7.49 (2H, d, J=8.0 Hz), 7.77 (2H, d, J=8.5 Hz)

### Synthesis of Compound 14

To a suspension of Compound 8 (900 mg, 3.41 mmol), Compound 13 (2.95 g, 8.17 mmol) and triphenylphosphine (2.14 g, 8.17 mmol) in tetrahydrofuran (40 mL) was added dropwise a solution of diisopropyl azodicarboxylate (1.62 mL, 8.17 mmol) in tetrahydrofuran (10 mL) under ice cooling with stirring over 10 minutes, and the mixture was stirred at the same temperature for 1 hour and at room temperature for 4 days. The solvent of the reaction solution was distilled off under reduced pressure, and the residue was purified by silica gel column chromatography (eluting solvent: ethyl acetate/n-hexane = 1/9, 1/4), followed by recrystallization from ethyl acetate/n-hexane to obtain Compound 14 (1.72 g, 83 %) as yellow crystals.
mp 128-130 °C
ESI-MS m/z 607 [M+H]⁺

### Synthesis of Compound 15

To a solution of Compound 14 (1.715 g, 2.83 mmol) in chloroform (18 mL) was added dropwise trifluoroacetic acid (12 mL) under ice cooling with stirring, and thereto was added water (3 mL), and the mixture was stirred at room temperature for 16 hours. To the reaction solution were added ice water and ethyl acetate, and the mixture was adjusted with aqueous potassium carbonate solution to pH 8, and extracted with ethyl acetate-tetrahydrofuran. The extraction liquid was washed with water, dried, and the solvent was distilled off under reduced pressure, and the residue was purified by silica gel column chromatography (eluting solvent: ethyl acetate/n-hexane = 1/2, ethyl acetate, ethyl acetate/tetrahydrofuran 1/1) to obtain Compound 15 (1.288g, 92 %) as pale yellow solids.
mp 188-189 °C
ESI-MS m/z 493 [M+H]⁺

### Synthesis of THK-5121S

To a mixture of Compound 15 (200 mg, 0.41 mmol), 3.4-dihydro-2H-pyran (0.74 mL, 8.12 mmol) and chloroform (20 mL) was added paratoluenesulfonic acid monohydrate (162 mg, 0.94 mmol), and the mixture was stirred at room temperature for 1 hour. The reaction solution was adjusted with triethylamine to pH 9, and the solvent was distilled off under reduced pressure. The residue was purified by silica gel column chromatography (eluting solvent: ethyl acetate/n-hexane = 1/4, 1/2), followed by recrystallization from ethyl acetate/n-hexane (1/2) to obtain THK-5121S (205 mg, 87 %) as pale yellow crystals.
mp 118-119 °C
¹H NMR (400 MHz, DMSO-d₆) δ 1.33-1.52 (4H, m), 1.52-1. 73 (2H, m), 2.34 (3H, s), 3.00 (6H, s), 3.34-3.48 (1H, m), 3.65-3.73, 3.81-3.89 (H, each m), 4.08-4.37 (5H, m), 4.69-4.72, 4.84-4.88 (1H, each m), 6.84 (2H, d, J=9.1 Hz), 7.23, 7.24 (1H, each dd, J=9.4, 1.5 Hz), 7.28-7.30 (1H, m), 7.39-7.43 (2H, m), 7.77-7.82 (2H, m), 7.87 (1H, d, J=9.1 Hz), 7.99 (1Hm d, J=8.8 Hz), 8.11 (2H, d, J=9.1 Hz), 8.20 (1H, d, J=8.8 Hz)
IR (Nujol) 1600 cm⁻¹
APCI-MS m/z 577 [M+H]⁺

Hereinafter, one example of synthesis of THK-5117S is shown.

### Synthesis of THK-5117S

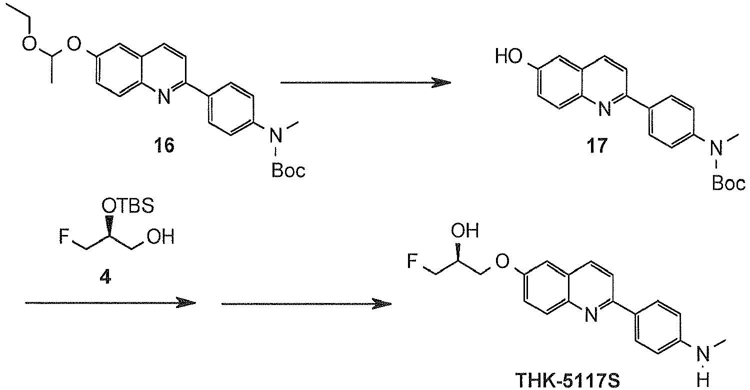

### Synthesis of Compound 17

To a solution of Compound 16 (2.48 g, 5.87 mmol) in chloroform (20 mL) was added dropwise trifluoroacetic acid (4 mL) under ice cooling with stirring, and the mixture was stirred at the same temperature for 1 hour. To the reaction solution were added ice water and ethyl acetate, and the mixture was adjusted with aqueous potassium carbonate solution to pH 8, and the mixture was extracted with ethyl acetate. The extraction liquid was washed with water, and dried, and the solvent was distilled off under reduced pressure, and the residue was purified by silica gel column chromatography (eluting solvent: ethyl acetate/n-hexane=1/1), followed by recrystallization from ethyl acetate/n-hexane to obtain Compound 17 (1.71 g, 83 %) as pale yellow crystals.
mp 198-200 °C
ESI-MS m/z 351 [M+H]⁺

### Synthesis of THK-5117S

To a solution of Compound 17 (600 mg, 1.71 mmol), Compound 4 (860 mg, 4.11 mmol), triphenylphosphine (1.08 g, 4.11 mmol) and triphenylphosphine (1.08 g, 4.11 mmol) in tetrahydrofuran (20 mL) was added dropwise a solution of diisopropyl azodicarboxylate (0.81 mL, 4.11 mmol) in tetrahydrofuran (10 mL) under ice cooling with stirring over 10 minutes, and the mixture was stirred at the same temperature for 1 hour and at room temperature for 3 days. The reaction solution was distilled off under reduced pressure, and the residue was subjected to silica gel column chromatography (eluting solvent: ethyl acetate/hexane = 1/9) to obtain pale yellow oil (1.84 g). To a solution of this product (1.84 g) in chloroform (18 mL) was added dropwise trifluoroacetic acid (12 mL) under ice cooling with stirring, and thereto was added water (3 mL), and the mixture was stirred at room temperature for 16 hours. To the reaction solution were added water and ethyl acetate, and the mixture was adjusted with aqueous potassium carbonate solution to pH 8, and extracted with ethyl acetate. The extraction liquid was washed with water, dried, and the solvent was distilled off under reduced pressure, and the residue was purified by silica gel column chromatography (eluting solvent: ethyl acetate/n-hexane = 1/9, 1/4, 1/2), followed by recrystallization from ethyl acetate to obtain THK-5117S (503 mg, 90 %) as pale yellow crystals.
mp 144-145 °C
¹H NMR (400 MHz, DMSO-d₆) δ 2.75 (3H, d, J-4.8 Hz), 4.00-4.20 (3H, m), 4.44-4.65 (2H, m), 5.55 (1H, d, J=3.3 Hz), 6.08 (1H, m), 6.65 (2H, d, J=8.8 Hz), 7.33-7.39 (2H, m), 7.87 (1H, d, J=8.8 Hz), 7.94 (1H, d, J=8.8 Hz), 8.02 (2H, d, J=9.1 Hz), 8.20 (1H, d, J=8.8 Hz) IR (Nujol) 3429, 3190, 1621, 1599 cm⁻¹
APCI-MS m/z 327 [M+H]⁺

Hereinafter, one example of synthesis of THK-5119S is shown.

### Synthesis of THK-5119S

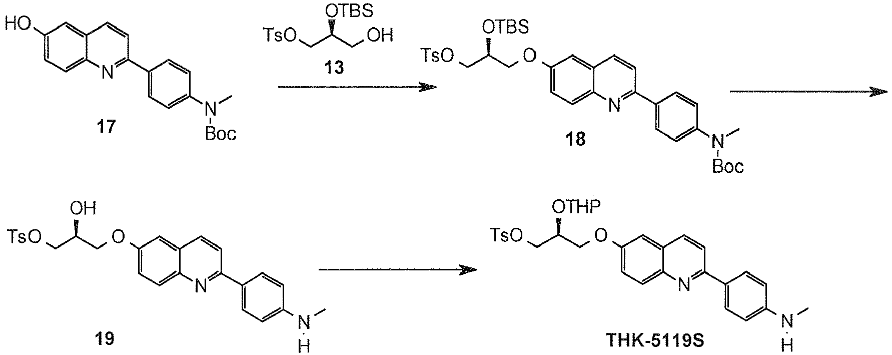

### Synthesis of Compound 18

To a solution of Compound 17 (600 mg, 1. 71 mmol), Compound 13 (1.48 g, 4.11 mmol), triphenylphosphine (1.08 g, 4.11 mmol) in tetrahydrofuran (40 mL) was added dropwise a solution of diisopropyl azodicarboxylate (0.81 mL, 4.11 mmol) in tetrahydrofuran (10 mL) under ice cooling with stirring over 10 minutes, and the mixture was stirred at the same temperature for 1 hour and at room temperature for four days. The solvent of the reaction solution was distilled off under reduced pressure, and the residue was subjected to silica gel column chromatography (eluting solvent: ethyl acetate/n-hexane = 1/9, 1/4) to obtain crude Compound 18 (2.15 g) as pale yellow viscous oil.

### Synthesis of Compound 19

To a solution of the crude Compound 18 (2. 15 g) in chloroform (18 mL) was added dropwise trifluoroacetic acid (12 mL) under ice cooling with stirring, and thereto was added water (3 mL), and the mixture was stirred at room temperature for 16 hours. To the reaction solution were added ice water and ethyl acetate, and the mixture was adjusted with aqueous potassium carbonate solution to pH 8, and extracted with ethyl acetate. The extraction solution was washed with water, dried, and concentrated under reduced pressure to about 50 mL, and purified by silica gel column chromatography (eluting solvent: ethyl acetate/n-hexane = 1/2, 1/1, ethyl acetate), followed by recrystallization from ethyl acetate to Compound 19 (722 mg, 88%/yields over 2 steps from Compound 17) as pale yellow crystals.
mp 163.5-164 °C
ESI-MS m/z 479 [M+H]⁺

### Synthesis of THK-5119S

To a mixture of Compound 19 (511 mg, 1.07 mmol), 3,4-dihydro-2H-pyran (1.94 mL, 21 mmol) and chloroform (50 mL) was added paratoluenesulfonic acid monohydrate (239 mg, 1.39 mmol) at room temperature, and the mixture was stirred at room temperature for 20 minutes. The reaction solution was ice-cooled, and adjusted with triethylamine to pH 8, and the solvent was distilled off under reduced pressure. The residue was purified by silica gel column chromatography (eluting solvent: ethyl acetate/n-hexane = 1/4, 1/2, 1/1), followed by recrystallization from ethyl acetate to obtain THK-5119S (474 mg, 79 %) as pale yellow crystals.
mp 143-144 °C
¹H NMR (400 MHz, DMSO-d₆) δ 1.33-1.51 (4H, m), 1.51-1.71 (2H, m), 2.33, 2.34 83H, each s), 2.75 83H, d, J=4.8 Hz), 3.30-3.47 (1H, m), 3.65-3.73 , 3.81-3.88 (1H, each m), 4.00-4.37 (5H, m), 4.70, 4.86 (1H, each m), 6.08 (1H, q, J=4.8Hz), 6.66 (2H, d, J=8.8Hz), 7.21 -7.25 (1H, m), 7.26-7.29 (1H, m), 7.39-7.43 (2H, m9, 7.77-7.81 (2H, m), 7.85 (1H, d, J=9.4Hz), 7.95 (1H, d, J=9.4Hz), 8.03 (2H, d, J=8.8 Hz), 8.17 (1H, d, J=8.8 Hz)
1R (Nujol) 3378, 1622, 1600 cm⁻¹
APCI-MS m/z 563 [M+H]⁺

Also, one example of another synthesis of HK-5117S is shown below.

### Synthesis of THK-5117S

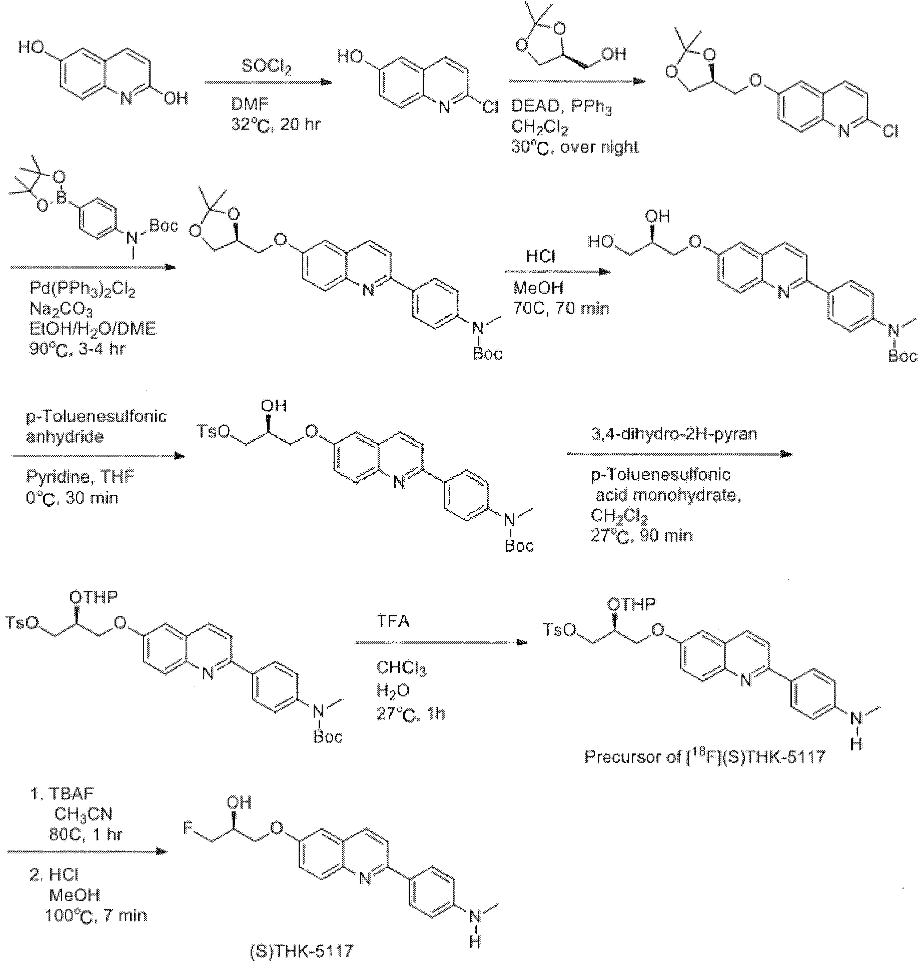

Hereinafter, one example of synthesis of THK-5151S is shown.

### Synthesis of THK-5151S

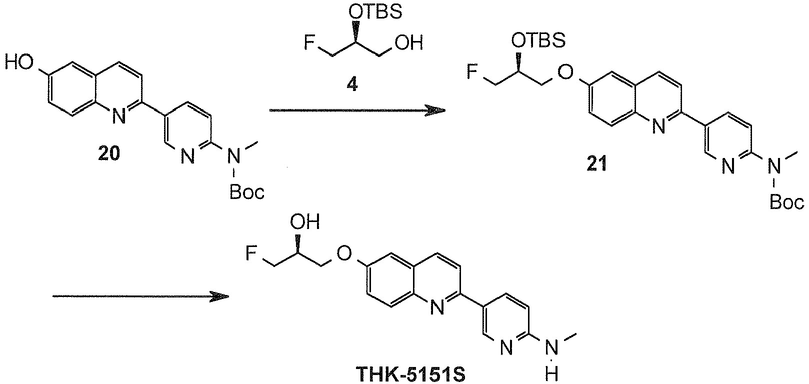

### Synthesis of Compound 21

To a mixture of Compound 20 (1.00 g, 2.86 mmol), Compound 4 (1.43 g, 6.86 mmol), triphenylphosphine (1.80 g, 6.86 mmol), and tetrahydrofuran (40 mL) was added dropwise diisopropyl azodicarboxylate (1.40 g, 6.92 mmol) under ice cooling with stirring over 30 minutes, and the mixture was stirred at the same temperature for 3 hours and at room temperature for 16 hours. The solvent of the reaction solution was distilled off under reduced pressure, and the residue was subjected to silica gel column chromatography (eluting solvent: ethyl acetate/n-hexane = 1/10, then ethyl acetate/chloroform = 1/1, ethyl acetate) to obtain crude compound 21 (2.77 g) as colorless wax.

### Synthesis of THK-5151S

To a solution of crude Compound 21 (2.77 g) in chloroform (20 mL) was added dropwise trifluoroacetic acid (10 mL) under ice cooling with stirring, and the mixture was stirred at room temperature for 16 hours, and thereto was added water (10 mL), and the mixture was stirred at room temperature for another 2 hours. The reaction solution was concentrated under reduced pressure, and thereto was added water (20 mL), and the mixture was basified with potassium carbonate and extracted with ethyl acetate. The extraction liquid was washed with saturated saline, dried, and the solvent was distilled off under reduced pressure. The residue was purified by silica gel column chromatography (eluting solvent: ethylacetate/chloroform = 1/1, ethyl acetate), followed by recrystallization from ethanol to obtain THK-5151S (632 mg, 68 %/yields over 2 steps from Compound 20) as colorless crystals. mP 165-166 °C
¹H NMR (400 MHz, DMSO-d₆) δ 2.85 (3H, d, J=4.8 Hz), 3.99-4.28 (3H, m), 4.41-4.71 (2H, m), 5.55 (1H, s), 6.58 (1H, d, J=8.8 Hz), 6.90 (1H, q, J=4.6 Hz), 7.28-7.51 (2H, m), 7.80-7.94 (1H, m), 7.98 (1H, d, J=8.8 Hz), 8.18-8.36 (2H, m), 8.87 (1H, d, J=2.4 Hz)
IR (Nujol) 1623, 1595 cm⁻¹
APCI-MS m/z 328 [M+H]⁺

Hereinafter, one exmaple of synthesis of THK-5152S is shown. Synthesis of THK-5152S

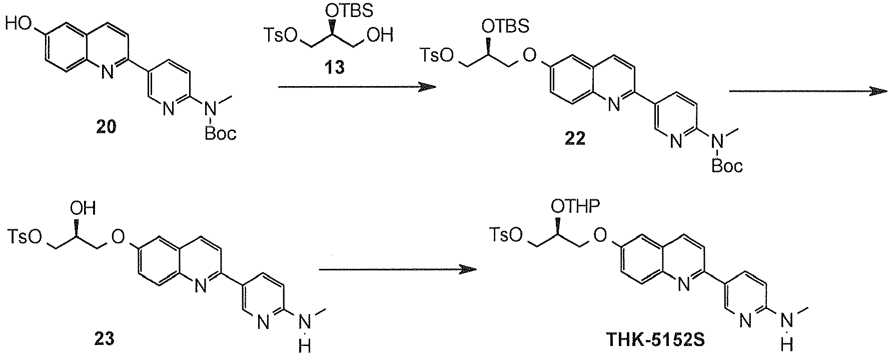

### Synthesis of Compound 22

To a solution of Compound 20 (800 mg, 2.28 mmol), Compound 13 (1.64g, 4.55 mmol) and triphenylphosphine (1.19 g, 4.54 mmol) in tetrahydrofuran (40 mL) was added dropwise diisopropyl azodicarboxylate (0.90 mL, 4.45 mmol) in tetrahydrofuran (10 mL) under ice cooling with stirring over 10 minutes, and the mixture was stirred at the same temperature for 1 hour and at room temperature for 4 days. The solvent of the reaction solution was distilled off under reduced pressure, and the residue was subjected to silica gel column chromatography (eluting solvent: ethyl acetate/n-hexane = 1/9, 1/6, 1/4) to obtain Compound 22 (1.87 g) as colorless solid.

### Synthesis of Compound 23

To a solution of Compound 22 (1.87 g) in chloroform (24 mL) was added dropwise trifluoroacetic acid (16 mL), and thereto was added water (3 mL), and the mixture was stirred at room temperature for 16 hours. To the reaction solution were iced water and ethyl acetate, and the mixture was adjusted with aqueous potassium carbonate solution to pH 8, and extracted with ethyl acetate. The extraction solution was washed with water, dried, and the solvent was distilled off under reduced pressure, and the residue was washed with ethyl acetate/n-hexane (1/1) to obtain Compound 23 (806 mg, 74 %/ yields over 2 steps from Compound 20) as pale yellow crystals.
mp 167-168 °C
ESI-MS m/z 480 [M+H]⁺

### Synthesis of THK-5152S

To a mixture of Compound 23 (895 mg, 1.87 mmol), 3.4-dihydro-2H-pyran (3.38 mL, 37.3 mmol) and chloroform (90 mL) was added paratoluenesulfonic acid monohydrate (707 mg, 4.11 mmol) at room temperature, and the mixture was stirred at room temperature for 20 minutes. The reaction solution was ice-cooled and adjusted with triethylamine to pH 8, and the solvent was distilled off. The residue was purified by silica gel column chromatography (eluting solvent: ethyl acetate/n-hexane = 2/1, ethyl acetate), followed by recrystallization from ethyl acetate/n-hexane (1/2) to obtain THK-5152S (961 mg, 91 %) as colorless crystals.
mp 146-147 °C
¹H NMR (400 MHz, DMSO-d₆) δ 1.33-1.51 (4H, m), 1.51-1.71 (2H, m), 2.34 (3H, s), 2.85 (3H, d, J=4.8Hz), 3.35-3.45 (1H, m), 3.65-3.72, 3.81-3.88 (1H, each m), 4.09-4.39 (5H, m), 4.70, 4.86 (1H, t, J=3.5 Hz, and br), 6.58 (1H, d, J=8.8 Hz), 6.90 (1H, q, J=4.7 Hz), 7.25 (1H, dd, J=9.1, 2.7 Hz), 7.30, 7.31 (1H, each d, J=2.1, 2.7 Hz), 7.39-7.43 (2H, m), 7.79 (1H, d, J=8.5Hz), 7.80 (1H, d, J=8.5Hz), 7.87 (1H, d, J=9.4 Hz), 7.99 (1H, d, J=8.8 Hz), 8.22 (1H, d, J=9.1 Hz), 8.27 (1H, dd, J=8.8, 2.4 Hz), 8.87 (1H, d, J=2.4 Hz)
IR (Nujol) 3356, 1623, 1604 cm⁻¹
APCI-MS m/z 564 [M+H]⁺

Further, the optically active compounds can be also obtained by separating the racemic form using an optical resolution method that is generally known in an organic chemical field. Examples of the optical resolution method include an optical resolution by chromatography with an optically active column, a preferential crystallization, diastereomeric salt formation method, and optical resolution. Examples of the optically active column include commercially available chiral column.

### Reference Examples

The racemic form of the compound of the present invention can be prepared according to WO 2012/057312. The reference examples are shown in Tables 2-1 to 2-17 below.

**Table 2-1**

| | | |
|---|---|---|
| THK-5004 | 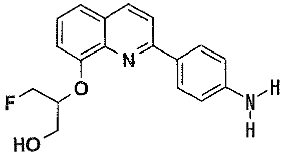 | 2-(4-aminophenyl)-8-( 1-fluoromethyl-2-hydr oxyethoxy)quinoline |
| THK-5035 | 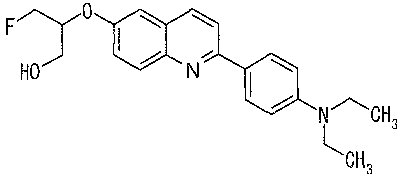 | 2-(4-diethylaminophen yl)-6-(1-fluoromethyl -2-hydroxy)quinoline |
| THK-5038 | 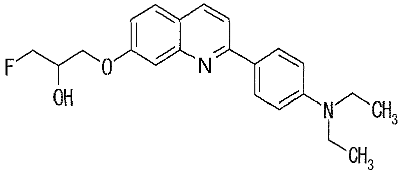 | 2-(4-diethylaminophen yl)-7-(2-fluoromethyl -2-hydroxyethoxy)quin oline |
| THK-5051 | 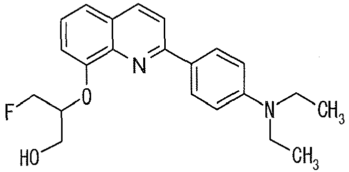 | 2-(4-diethylaminophen yl)-8-(1-fluoromethyl -2-hydroxyethoxy)quin oline |

**Table 2-2**

| | | |
|---|---|---|
| THK-5058 | 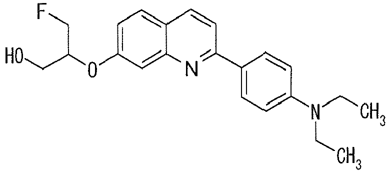 | 2-(4-diethylaminophe nyl)-7-(1-fluorometh yl-2-hydroxyethoxy)q uinoline |
| THK-5059 | 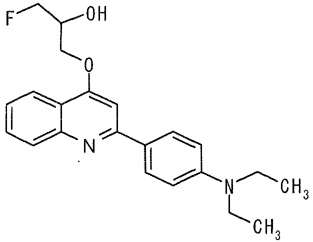 | 2-(4-diethylaminophe nyl)-4-(3-fluoro-2-h ydroxypropoxy)quinol ine |
| THK-5064 | 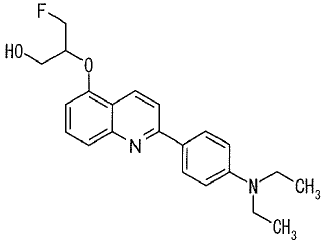 | 2-(4-diethylaminophe nyl)-5-(1-fluorometh yl-2-hydroxyethoxy)q uinoline |
| THK-5065 | 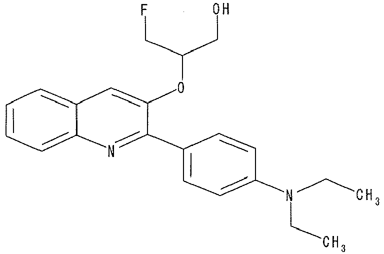 | 2-(4-diethylaminophe nyl)-3-(1-fluorometh yl-2-hydroxyethoxy)q uinoline |

**Table 2-3**

| | | |
|---|---|---|
| THK-5066 | 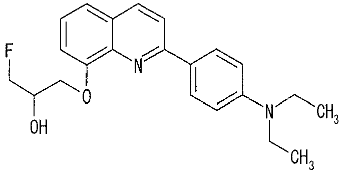 | 2-(4-diethylaminophe nyl)-8-[(3-fluoro-2-hydroxy)propoxy]quin oline |
| THK-5071 | 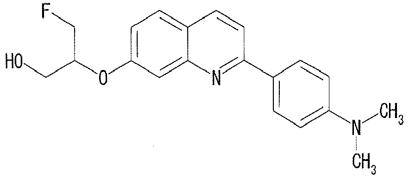 | 2-(4-fluoromethyl-2-hydroxyethoxy)-2-(4-dimethylaminophenyl) quinoline |
| THK-5077 | 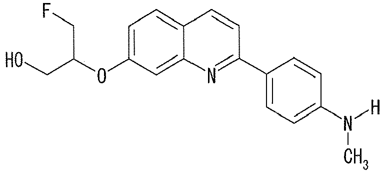 | 7-(1-fluoromethyl-2-hydroxyethoxy)-2-(4-methylaminophenyl)qu inoline |
| THK-5078 | 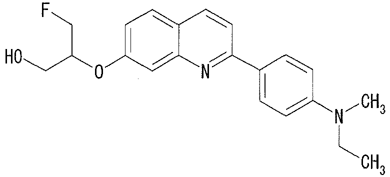 | 2-(4-ethylmethylamin ophenyl)-7-(1-fluoro methyl-2-hydroxyetho xy)-quinoline |

**Table 2-4**

| | | |
|---|---|---|
| THK-5105 | 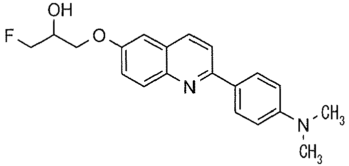 | 6-[(3-fluoro-2-hydro xy)propoxy]-2-(4-dim ethylaminophenyl)qui noline |
| THK-5106 | 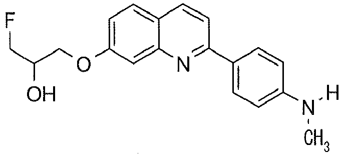 | 7-[(3-fluoro-2-hydro xy)propoxy]-2-(4-met hylaminophenyl)quino line |
| THK5107 | 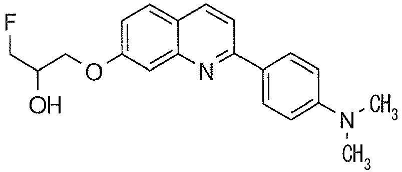 | 7-[(3-fluoro-2-hydro xy)propoxy]-2-(4-dim ethylaminophenyl)qui noline |
| THK-5112 | 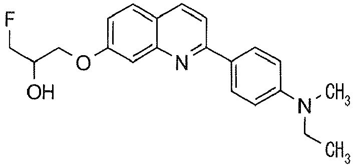 | 2-(4-ethylmethylamin ophenyl)-7-[(3-fluor o-2-hydroxy)propoxy] quinoline |

**Table 2-5**

| | | |
|---|---|---|
| THK-5116 | 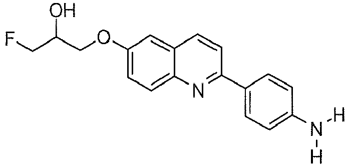 | 2-(4-aminophenyl)-6-[(3-fluoro-2-hydroxy )propoxy]quinoline |
| THK-5117 | 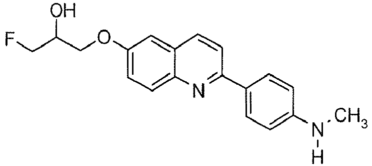 | 6-[(3-fluoro-2-hydro xy)propoxy]-2-(4-met hylaminophenyl)quino line |
| THK-5122 | 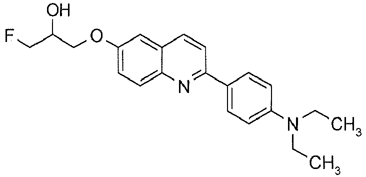 | 6-[(3-fluoro-2-hydro xy)propoxy]-2-(4-die thylaminophenyl)quin oline |
| THK-5075 | 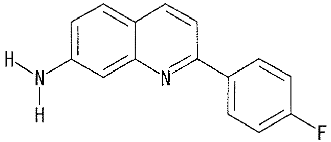 | 7-amino-2-(4-fluorop henyl)quinoline |

**Table 2-6**

| | | |
|---|---|---|
| THK-5076 | 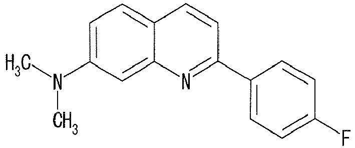 | 2-(4-fluorophenyl)-7 -dimethylaminoquinol ine |
| THK-5079 | 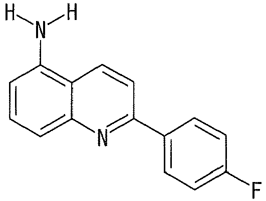 | 5-amino-2-(4-fluorop henyl)quinoline |
| THK-5080 | 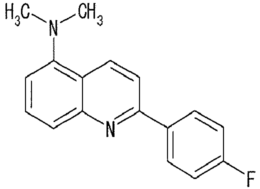 | 2-(4-fluorophenyl)-5 -dimethylaminoquinol ine oxalate |
| THK-5081 | 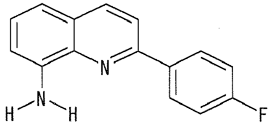 | 8-amino-2-(4-fluorop henyl)quinoline |

**Table 2-7**

| | | |
|---|---|---|
| THK-5082 | 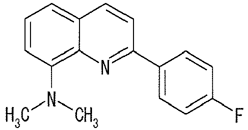 | 2-(4-fluorophenyl)-8 -dimethylaminoquinol ine |
| THK-5086 | 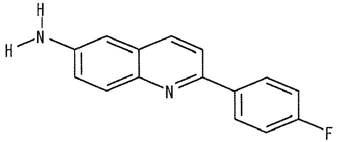 | 6-amino-2-(4-fluorop henyl)quinoline |
| THK-5087 | 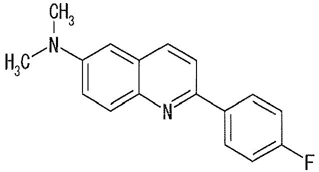 | 2-(4-fluorophenyl)-6 -dimethylaminoquinol ine |
| THK-932 | 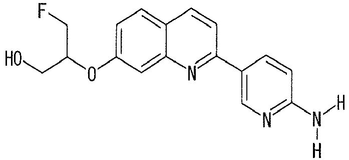 | 2-(2-aminopyrid-5-yl )-7-(1-fluoromethyl-2-hydroxyethoxy)quin oline |

**Table 2-8**

| | | |
|---|---|---|
| THK-5100 | 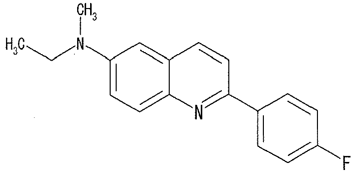 | 6-ethylmethylamino-2 -(4-fluorophenyl)qui noline |
| THK-5088 | 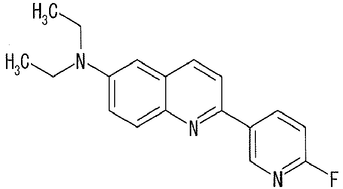 | 6-diethylamino-2-(2-fluoropyrid-5-yl)qui noline |
| THK-5089 | 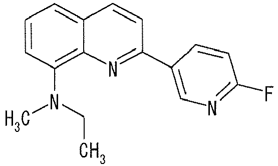 | 8-ethylmethylamino-2 -(2-fluoropyrid-5-yl )quinoline |
| THK-5091 | 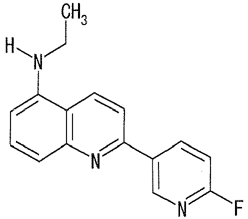 | 5-ethylamino-2-(2-fl uoropyrid-5-yl)quino line |

**Table 2-9**

| | | |
|---|---|---|
| THK-5092 | 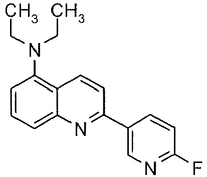 | 5-diethylamino-2-(2-fluoropyrid-5-yl)qui noline |
| THK-5097 | 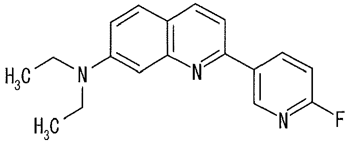 | 7-diethylamino-2-(2-fluoropyrid-5-yl)qui noline |
| THK-5098 | 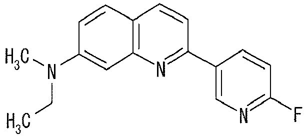 | 7-ethylmethylamino-2 -(2-fluoropyrid-5-yl )quinoline |
| THK-5125 | 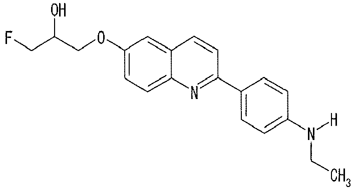 | 2-(4-ethylaminopheny 1)-6-[(3-fluoro-2-hy droxy)propoxy]quinol ine |

**Table 2-10**

| | | |
|---|---|---|
| THK-5127 | 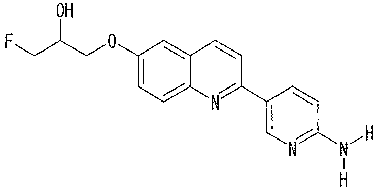 | 2-(2-aminopyrid-5-yl )-6-[(3-fluoro-2-hyd roxy)propoxy]quinoli ne |
| THK-5151 | 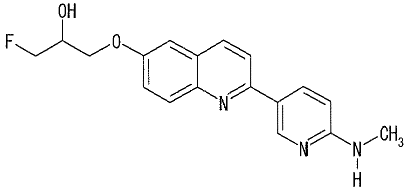 | 2-(2-methylaminopyri d-5-yl)-6-[(3-fluoro -2-hydroxy)propoxy]q uinoline |
| THK-5129 | 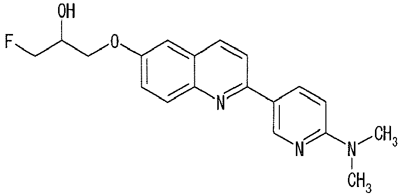 | 6-[(3-fluoro-2-hydro xy)propoxy]-2-(2-dim ethylaminopyrid-5-yl )quinoline |
| THK-5130 | 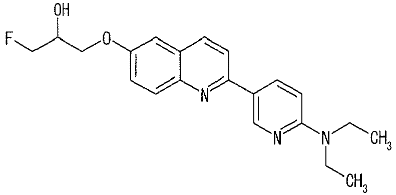 | 2-(2-diethylaminopyr id-5-yl)-6-[(3-fluor o-2-hydroxy)propoxy] quinoline |
| THK-5142 | 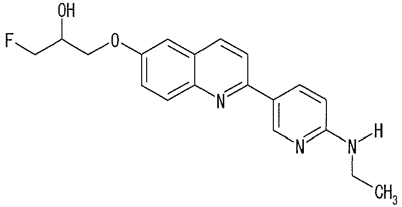 | 2-(2-ethylaminopyrid -5-yl)-6-[(3-fluoro-2-hydroxy)propoxy]qu inoline |

**Table 2-11**

| | | |
|---|---|---|
| THK-5177 | 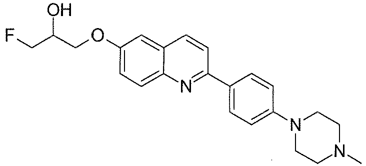 | 1-fluoro-3-{2-[4-(4-methylpiperazin-1-yl )phenyl]quinolin-6-y loxy)propan-2-ol |
| THK-5178 | 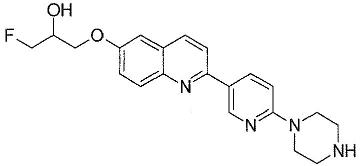 | 1-fluoro-3-{2-[6-(pi perazin-1-yl)pyridin -3-yl]quinolin-6-ylo xy}propan-2-ol |
| THK-5180 | 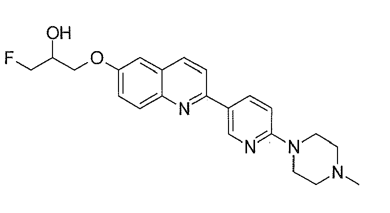 | 1-fluoro-3-{2-[6-(4-methylpiperazin-1-yl )pyridin-3-yl]quinol in-6-yloxy}propan-2-ol |
| THK-5136 | 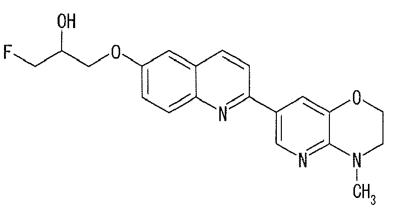 | 6-[(3-fluoro-2-hydro xy)propoxy]-2-(4-met hyl-3,4-dihydro-2H-p yrido[3,2-b][1,4]oxa zin-7-yl)quinoline |
| THK-5153 | 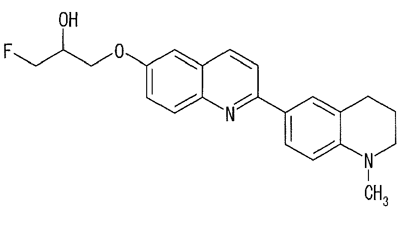 | 6-[(3-fluoro-2-hydro xy)propoxy]-2-(1-met hyl-1,2,3,4-tetrahyd roquinolin-6-yl)quin oline |

**Table 2-12**

| | | |
|---|---|---|
| THK-5157 | 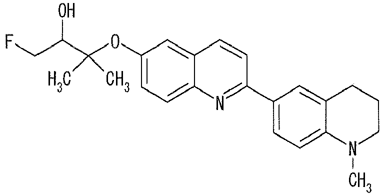 | 6-[(3-fluoro-2-hydro xy-1,1-dimethyl)prop oxy]-2-(1-methyl-1,2 ,3,4-tetrahydroquino lin-6-yl)quinoline |
| THK-5128 | 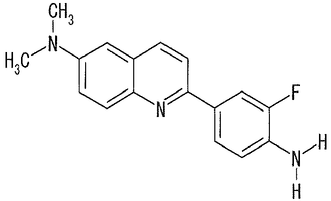 | 2-(4-amino-3-fluorop henyl)-6-dimethylami noquinoline |
| THK-5147 | 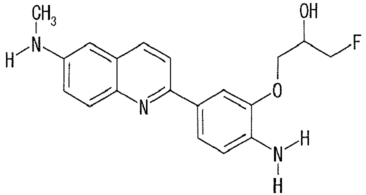 | 2-[4-(amino)-3-[(3-f luoro-2-hydroxy)prop oxy]phenyl]-6-methyl aminoquinoline |
| THK-5148 | 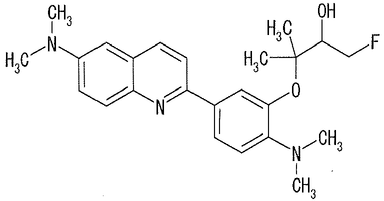 | 2-[3-(3-fluoro-2-hyd roxy-1,1-dimethyl)pr opoxy]-4-(dimethylam ino)-phenyl]-6-dimet hylaminoquinoline |

**Table 2-13**

| | | |
|---|---|---|
| THK-5155 | 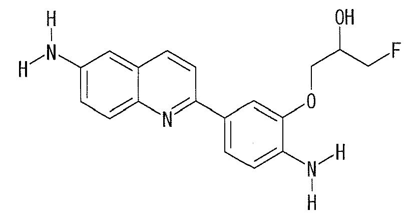 | 6-amino-2-[4-(amino) -3-[(3-fluoro-2-hydr oxy)propoxy]phenyl]q uinoline |
| THK-5156 | 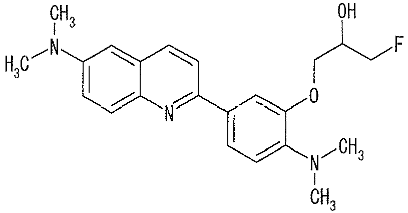 | 2-[3-[(3-fluoro-2-hy droxy)propoxy]-4-(di methylamino)phenyl]-6-dimethylaminoquino line |
| THK-5158 | 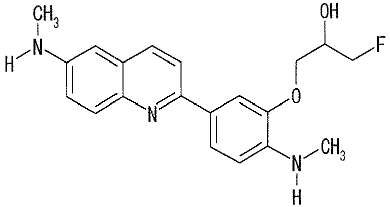 | 2-[3-[(3-fluoro-2-hy droxy)propoxy]-4-(me thylamino)phenyl]-6-methylaminoquinoline |
| THK-5159 | 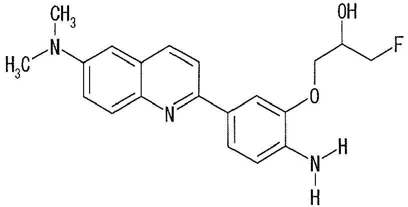 | 2-[4-(amino)-3-[(3-f luoro-2-hydroxy)prop oxy]phenyl]-6-dimethylamino quinoline |

**Table 2-14**

| | | |
|---|---|---|
| THK-5160 | 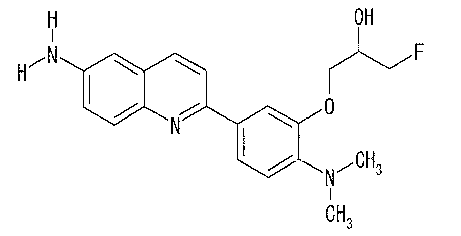 | 6-amino-2-[3-[(3 -fluoro-2-hydrox y)propoxy]-4-(di methylamino)phen yl]-quinoline |
| THK-5161 | 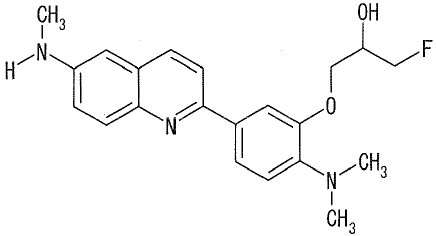 | 2-[3-[(3-fluoro-2-hydroxy)propox y]-4-(dimethylam ino)phenyl]-6-me thylaminoquinoli ne |
| THK-5162 | 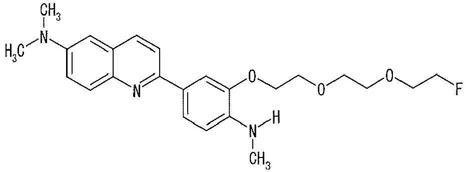 | 2- [3- [2-[2- (2-fl uoroethoxy)ethox y]ethoxy-4-(meth ylamino)phenyl]-6-dimethylaminoq uinoline |
| THK-5164 | 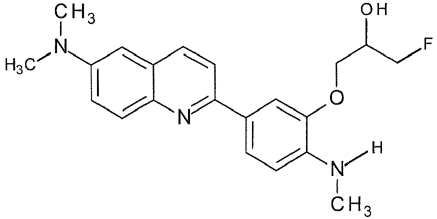 | 2-[3-[(3-fluoro-2-hydroxy)propox y]-4-(methylamin o)phenyl]-6-dime thylaminoquinoli ne |

**Table 2-15**

| | | |
|---|---|---|
| THK-5165 | 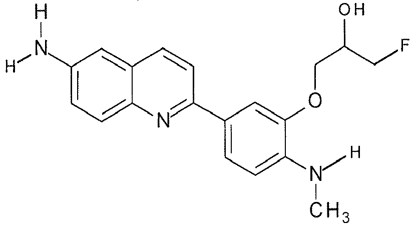 | 6-amino-2-[3-[(3-f luoro-2-hydroxy)pr opoxy]-4-(methylam ino)phenyl]-quinol ine |
| THK-5154 | 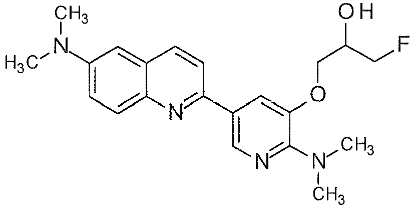 | 2-[3-[(3-fluoro-2-hydroxy)propoxy]-2 -(dimethylamino)py rid-5-yl]-6-dimeth ylaminoquinoline |
| THK-5166 | 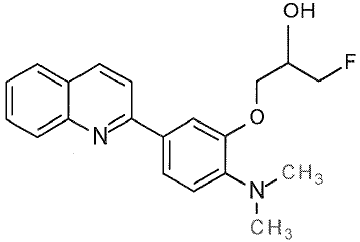 | 2-[3-[(3-fluoro-2-hydroxy)propoxy]-2 -(dimethylamino)py rid-5-yl]quinoline |
| THK-5170 | 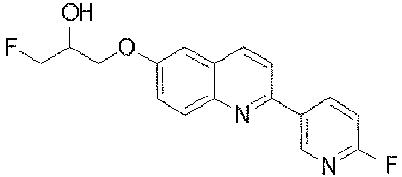 | 6-[(3-fluoro-2-hyd roxy)propoxy]-2-(6 -fluoropyridin-3-y 1)quinoline |

**Table 2-16**

| | | |
|---|---|---|
| THK-5171 | 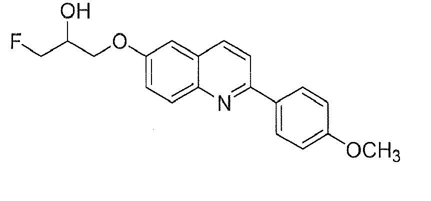 | 6-[(3-fluoro-2-hyd roxy)propoxy]-2-(4 -methoxyphenyl)qui noline |
| THK-5172 | 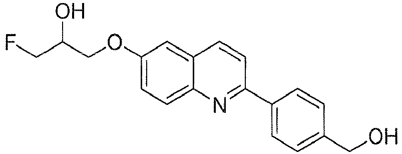 | 6-[(3-fluoro-2-hyd roxy)propoxy]-2-[4 -(hydroxymethyl)ph enyl]quinoline |
| THK-5173 | 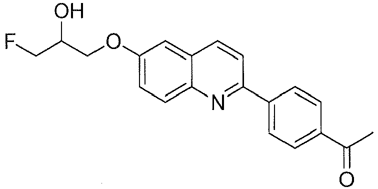 | 6-[(3-fluoro-2-hyd roxy)propoxy]-2-(4 -ethanonephenyl)qu inoline |
| THK-5174 | 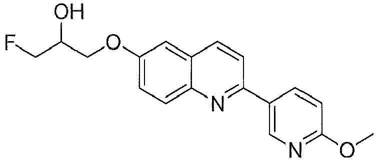 | 6-[(3-fluoro-2-hyd roxy)propoxy]-2-(6 -methoxypyridin-3-yl)quinoline |

**Table 2-17**

| | | |
|---|---|---|
| THK-5175 | 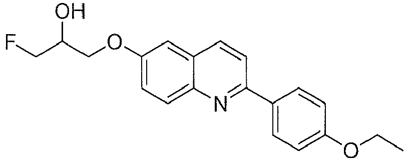 | 6-[(3-fluoro-2-hy droxy)propoxy]-2-(4-ethoxyphenyl)q uinoline |
| THK-5176 | 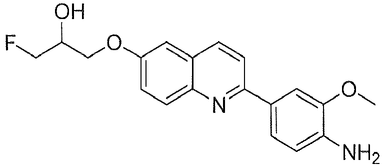 | 6-[(3-fluoro-2-hy droxy)propoxy]-2-(4-amino-3-methox yphenyl)quinoline |
| THK-5179 | 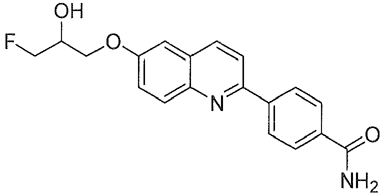 | 6-[(3-fluoro-2-hy droxy)propoxy]-2-(benzamido-4-yl)q uinoline |
| THK-5181 | 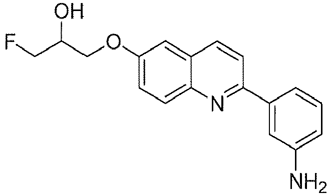 | 6-[(3-fluoro-2-hy droxy)propoxy]-2-(3-aminophenyl)qu inoline |
| THK-5182 | 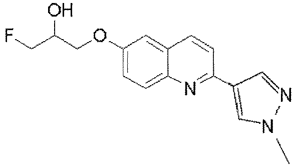 | 6-[(3-fluoro-2-hy droxy)propoxy]-2-(1-methyl-pyrazol -4-yl)quinoline |

One example of a method of optical separation with chiral column is shown below, but is not limited thereto.

### Separation of Racemic form with Chiral column

This experiment shows that a high-performance liquid chromatography using chiral column enables racemic form of THK-5105 (rac-THK-5105) to separate R- form (THK-5105R) and S-form (THK-5015S).

Each ethanol solution of THK-5105R, THK-5105S, and rac-THK-5105 (0.25 mg/ml) was prepared, and analyzed by a high-performance liquid chromatography (column: CHIRALPAK IA-3 (manufactured by Daicel Chemical Industries, Ltd.), particle size 3 µm, column size 4.6 × 150 mm); mobile phase: hexane/ethanol/diethylamine = 50/50/0.1, flow rate: 0.5 mL/min, Absorbance measurement wavelength: 315 nm). As the result, each chromatogram of THK-5105R and THK-5015S was obtained as Figure 8A and Figure 8B, respectively, and had 9.1 min. and 10.5 min., respectively, as each retention time of the R- form and S-form. Also, when the race form of THK-5105 was analyzed by a high-performance liquid chromatography under the same condition, Figure 8 was obtained, wherein two peaks each having the same retention time as R- form and S- form, respectively, were observed, which can thus indicate that a chiral column can be used to separate the racemic form.

Next, a preparation example of the labeled compound of the present invention is shown, but is not limited thereto. Labeling Synthesis of [¹⁸F] THK-5105S
¹⁸F⁻ was synthesized by irradiating [¹⁸O] H₂O having isotope purity of 98% or more with 12 MeV of proton beam accelerated by Cyclotron HM12 (manufactured by Sumitomo Heavy Industries, Ltd.). Subsequently, the solution thereof was passed through an anion-exchange resin (AG1-X8) thereby trapping ¹⁸F⁻ on the resin, followed by elution with a 33mM K₂CO₃ solution. After transferring this aqueous ¹⁸F⁻-containing K₂CO₃ solution (300 µL, 2.78 GBq) in a brown vial, Kryptofix 222 (16 mg) and acetonitrile (2.3 mL) were added and a He gas was sprayed while heating in an oil bath (110°C), and then acetonitrile was completely removed while azeotropically distilling water. Furthermore, an operation of adding acetonitrile (1.5 mL) and removing acetonitrile in the same manner under heating conditions was repeated twice, thereby turning the state inside the vial into a substantially moisture-free state. A DMSO solution (0.70 mL) containing THK-5121S (2.0 mg), as a label precursor, dissolved therein was added, followed by heating and stirring in the oil bath (110°C) for 10 minutes. Thereafter, thereto was added hydrochloric acid (2M, 0.2 mL), and the mixture was reacted at 110 °C for another 3 minutes, and the reaction solution was diluted with potassium acetate solution (4M, 0.1 mL) and distilled water (7.0mL), and loaded into a Sep-Pack tC18 cartridge (manufactured by Waters) and, after washing the cartridge with distilled water, the crude product was eluted with ethanol. A portion of the ethanol solution showing highest radioactivity was diluted with distilled water and subjected to semi-preparative high-performance liquid chromatography (column: Inertsil ODS-4) (10 x 250 mm), mobile phase: MeCN/NaH₂PO₄ (20 mM) = 45/55, flow rate: 6.0 mL/min) and then [¹⁸F] THK-5105S- derived radioactive peak (1.00 GBq, no decay correction) , which is eluted within about 21 to 22 minutes, was dispensed.

Further, test example of the label compound of the present invention is shown.

The preparative HPLC fraction of [¹⁸F] THK-5105S, which was synthesized according to the synthesis procedure described above, was diluted with distilled water, and then subjected to solid-phase extraction using a Sep-Pak tC18 cartridge, followed by elution with ethanol or DMSO and appropriate dilution, and used in binding tests and autoradiography experiments.

For experiments in small animal PET (evaluation of its brain delivery), Polysorbate 80 was added to the ethanol-eluted fraction, from which the ethanol was removed using an evaporator. The [¹⁸F] THK-5105S containing radioactive residue within the flask was dissolved in physiological saline and the solution prepared was used as a solution for injection.

As a comparable compound, [¹⁸F] THK-5105 (R)-enantiomer ([¹⁸F] THK-5105R), [¹⁸F] THK-5117S (S- form of THK-5117), [¹⁸F] THK-5117R (R- form of THK-5117), [¹⁸F] THK-5151S (S- form of THK-5151), and [¹⁸F] THK-5151R (R- form of THK-5151) was similarly synthesized from each corresponding label precursors, and used in a biological evaluation experiment.

### Autoradiography Experiments

A brain specimen in hippocampus of the patient which was definitively-diagnosed as Alzheimer's disease pathologically was used. A paraffin-embedded brain tissue was sliced by 6 µm or 8 µm thick, and stretched on a glass slide, and dried. The paraffin brain sections were deparaffinized by washing sequentially with xylene 10 min. × 2, 100% ethanol 5 min. × 2, 90% ethanol 5 min., and flowing water 10 min. After deparaffinization, the sections were immersed in PBS. Each of about 400 µCi/ml of [¹⁸F]THK-5105S and [¹⁸F]THK-5105R was added dropwise to the sections, and the sections were allowed to a reaction at room temperature for 10 min. Thereafter, the sections were immersed in distilled water for 2 min., and successively, shaked lightly in 50% EtOH for 2 min., and thereafter immersed in distilled water for 2 min. again, and dried on paraffin stretching plate. Then, the sections were contacted with imaging plate, and allowed to stand overnight, and on next day, the imaging was read on BAS5000 (manufactured by FUJIFILM Holdings Corporation).

The autoradiography experiments were also carried out similarly on [¹⁸F] THK-5151S and [¹⁸F] THK-5151R.

Figure 5 shows autoradiography imaging in each hippocampus section. [¹⁸F] THK-5105S and [¹⁸F] THK-5151S were clearly found to give stronger imaging signal in comparison with the corresponding [¹⁸F] THK-5105R and [¹⁸F] THK-5151R in tau lesion region, which means that they binds more strongly to tau pathology.

### Binding experiment (1) with AD brain tissue homogenates Competitive binding test using [³H] THK-5117 as radio ligand

PBS (0.1% BSA) was used as an assay buffer, and the concentration of THK-5105S or THK-5105R was adjusted with 2nM [³H] THK-5117 to 10⁻⁵ to 10⁻¹⁰ M in a reaction system (200 µL) containing 100 µg of the homogenates, and the competitive tests were then carried out. A nonspecific binding was measured with 2 µM of THK-5117. After incubation at room temperature for 3 hours, a glass filter plate was used to separate [³H] THK-5117 bound to the homogenates and unbound [³H] THK-5117, and the filter plate was washed, and then to the separated filter was added liquid scintillation cocktail, and the bound radioactivity was measured on a liquid scintillation counter. The data was analyzed by analysis software GraphPad Prism (Ver. 5). The competitive tests were carried out similarly on THK-5117S, THK-5117R, THK-5151S, and THK-5151R.

The results were shown in Table below. It was found that with respect to all tested compounds, S- form shows relatively smaller Ki value than R- form, which means that S- form has stronger binding affinity.

**Table 3**

| Ki (nM) | | |
|---|---|---|
| Compound | Temporal lobe cortex (607.6 pmol/g tissue) | Entorhinal cortex 601 pmol/g tissue |
| THK-5105S | 2.6 | 4.6 |
| THK-5105R | 16.3 | 27.4 |
| THK-5117S | 9.4 ± 4.8 (n=4) | - |
| THK-5117R | 25.7 ± 10.7 (n=3) | - |
| THK-5151S | 7.2 ± 1.27 (n=3) | - |
| THK-5151R | 23.4 (n=2) | - |

### Binding experiment (2) with AD brain tissue homogenates Organization binding evaluation experiment of [¹⁸F]THK-5105S

According to the above-mentioned assay buffer, nonspecific binding measurement, and filter separation operation, using 10 kinds of AD homogenate samples each having different tau concentration and amyloid β (Aβ) concentration, the reaction system was adjusted such that the tissue concentration was made 100 µg and [¹⁸F]THK-5105S concentration was made 1 nM, and the binding test (1 hour incubation) was carried out in the reaction system.

As comparative example, the binding test was carried out also on [¹⁸F] THK-5105R according to the similar method. The tau concentration or Aβ concentration was plotted on horizontal axis Vertical axis, and the bound amount of [¹⁸F] THK-5105S and [¹⁸F] THK-5105R was plotted on vertical axis, and the correlation thereof was evaluated.

As the result, as shown in Figure 6A, the bound amount of [¹⁸F]THK-5105S tissue showed extremely higher correlation with the tau concentration in comparison with [¹⁸F] THK-5105R, and also showed higher bound amount, and the variation width was increased depending on an increase of the tau concentration. This means that when imaging, [¹⁸F]THK-5105S can be detected the change of the tau concentration more sensitively. While, the bound amounts of the tissues of both label compounds could not be found to have a correlation with the Aβ concentration (see Figure 6B). From these results, [¹⁸F]THK-5105S is tau selective probe, and shows superior tau binding property than [¹⁸F]THK-5105R, and can evaluate the change of tau concentration with higher sensitivity (accuracy).

Further, according to the above-mentioned assay buffer, nonspecific binding measurement, and filter separation operation, each saturation binding experiment of [¹⁸F] THK-5151S and [¹⁸_{F}] THK-5151R was carried out with AD hippocampus tissue homogenate. Zero point one (0.1) to 100 nM of each tested substance was incubated with hippocampus homogenate, and then filtered with a filter, washed, and then measured radioactivity supplemented by the filter, and thereby calculated as total binding and nonspecific binding at each concentration. The data was analyzed with GraphPad Prism (Ver. 5) to calculate binding dissociation constants Kd and Bmax. The binding curve of the results and the analysis results are shown as Figure 6c. [¹⁸F] THK-5151S showed about one tenth small Kd value, and showed about fourth times large Bmax/Kd value as bonding potential, in comparison with those of [¹⁸F] THK-5151R. Thus it was found that a binding affinity with tau lesion of [¹⁸F] THK-5151S is superior to that of [¹⁸F] THK-5151R.

### Dynamic evaluation (1) of Normal Mouse Brain

### Evaluation by Small animal PET

In this experiment, [¹⁸F] THK-5105S or [¹⁸F] THK-5105R was administered intravenously via tail to ICR mouse (male 6 to 7 weeks old) under isoflurane anesthesia (1.5 %), and the time-change of radioactivity distribution in brain was measured on Clairvivopet/CT (manufactured by Shimadzu Corporation, Kyoto). The emission scan was started simultaneously with the administration of the probe, and measured in 3D list mode for 120 min. After imaging, 35 frames (10 × 60 s, 10 × 120 s, 10 × 300 s, 5 × 480 s) were reconstructed, and a region of interest was set in the brain, and quantified as SUV, and a time-activity curve (TAC) was created, and the Dynamism of the both probes were compared.

As the result, it was found as shown in Figure 7A that [¹⁸F]THK-5105S was disappeared more rapidly from normal brain tissue shortly after the administration, and [¹⁸F]THK-5105S had lower retention into the normal brain tissue, in comparison with [¹⁸F) THK-5105R. In order to compare the disappearance change, the peak values of the TAC for both probes were converted into 100 %, and the TAC was then shown as Figure 7B. As evident from the TACs, [¹⁸F]THK-5105S has larger ratio of disappearance per hour than [¹⁸F]THK-5105R, that is, has more rapid rate of disappearance.

Further, when the ratio of 5 min./30 min., 5 min./60 min., 5 min. /120 min. , of each probe was compared using these SUV values, [¹⁸F] THK-5105S showed significantly higher value, which thus shows excellent disappearance and low retention in brain. This means that when PET imaging, [¹⁸F]THK-5105S showed lower non-specific accumulation into normal brain tissue, and can be thus imaged the tau lesion with better contrast, with comparison with [18F]THK-5105R, and can therefore detect tau lesion with more excellent selectivity.

**Table 4**

| SUV Ratio | | |
|---|---|---|
| Time | [¹⁸F] THK-5105S | [¹⁸F] THK-5105R |
| 5 min./30 min. | 2.70 ± 0.1 | 1.92 ± 0.3 |
| 5 min./60 min. | 6.27 ± 0.6 | 3.67 ± 0.9 |
| 5 min./120 min. | 19.2 ± 4.4 | 7.75 ± 1.6 |

### Dynamic evaluation (2) of Normal Mouse Brain

### Evaluation by Biodistribution method

A saline comprising [¹⁸F] THK-5105S or [¹⁸F] THK-5105R was administered intravenously via tail to ICR mouse (male 6 to 7 weeks old), and the time change of radioactivity distribution in brain was measured on Clairvivopet/CT (manufactured by Shimadzu Corporation, Kyoto). The brain dynamics was evaluated by the time-change of the accumulation rate of radioactivity in brain tissue after 2 min., 10 min., 30 min., 60 min., and 120 min.

The accumulation rate of radioactivity was calculated as the ratio of radioactivity per weight of evaluated tissue against total administered radioactivity (% Injected Dose/g of tissue; % ID/g). The measurement of the radioactivity was carried out with Gamma counter (Accuflex _{Υ}7000, measured by Hitachi-Aloka Medical, Tokyo). The experimental procedure was as follows. After administering the label compound intravenously via tail at 2 min. , 10 min., 30 min., 60 min., and 120 min., cervical dislocation of mouse was performed under ether anesthesia, and organ tissues were extracted. The radioactivity and the weight of each sample were measured, and the data was analyzed to calculate %ID/g. Also, as the evaluation index for the disappearance from the brain, the divided value of the accumulation rate at 2 min. after administration by the accumulation rate at 60 min. after administration (2 min. /60 min. ratio) was calculated. This means that higher value represents more superior disappearance from the brain. The above results are shown in Table 5.

[¹⁸F] THK-5151S and [¹⁸F]THK-5151R both were showed almost the same sufficient uptake as about 4% ID/g shortly after administration, but when the accumulation rate value after that was compared, [¹⁸F]THK-5151S showed lower values. When the 2 min./60 min. ratio was compared, [¹⁸F] THK-5151S showed more sufficient values, which indicates that [¹⁸F] THK-5151S is superior for the disappearance from the brain.

**Table 5**

| % ID/g (n=4) | | |
|---|---|---|
| Time (min.) | [¹⁸F] THK-5151S | [¹⁸F] THK-5151R |
| 2 min. | 4.36 ± 0.19 | 3.90 ± 0.16 |
| 10 min. | 1.10 ± 0.19 | 1.62 ± 0.18 |
| 30 min. | 0.21 ± 0.03 | 0.80 ± 0.08 |
| 60 min. | 0.13 ± 0.03 | 0.65 ± 0.07 |
| 120 min. | 0.12 ± 0.02 | 0.59 ± 0.07 |

**Table 6**

| 2min./60 min. ratio | |
|---|---|
| [¹⁸F] THK-5151S | [¹⁸F] THK-5151R |
| 33 | 6.1 |

The compounds of the present invention are very useful, for example, in early detection, treatment and prevention of neurofibrillary tangles including Alzheimer's disease, and can be utilized in the fields of the production of diagnostic agents and diagnostic kits for these diseases, the fields of the production of remedies and preventatives for these diseases, studies of these diseases and the like.

## Claims

1. A compound represented by the formula (I): 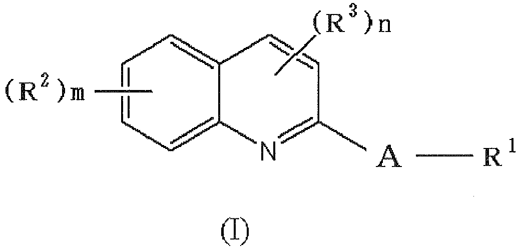 wherein
A is a cyclic group represented by a formula: 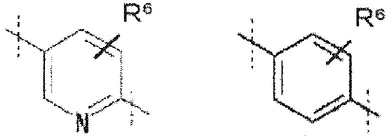 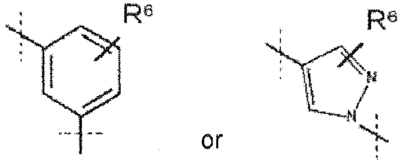 ring A is unsubstituted, or substituted with R⁶ (the R⁶ is one or more substituents selected independently from halogen, OH, COOH, SO₃H, NO₂, SH, NR^{a}R^{b}, lower alkyl (the alkyl group each independently may be optionally substituted with one or more substituents selected from halogen and hydroxy) and -O-lower alkyl (the alkyl group each independently may be optionally substituted with one or more substituents selected from halogen and hydroxy)),
R¹ is a halogen atom, a -C (=O) -lower alkyl group (the alkyl group each independently may be optionally substituted with one or more substituents selected from NR^{a}R^{b}, halogen and hydroxy), a lower alkyl group (the alkyl group each independently may be optionally substituted with one or more substituents selected from halogen atom and hydroxy), a -O-lower alkyl group (the alkyl group each independently may be optionally substituted with one or more substituents selected from halogen and hydroxy), or a group represented by a formula: 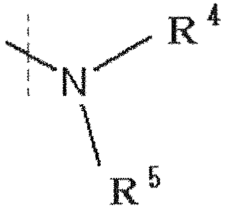 wherein
R⁴ and R⁵ each independently represents a hydrogen atom, a lower alkyl group (the alkyl group each independently may be optionally substituted with one or more substituents selected from halogen and hydroxy) or a cycloalkyl group, or R⁴, R⁵ and the nitrogen atom to which they are attached are taken together to form a 3- to 8-membered nitrogen-containing aliphatic ring (one or more carbon atoms constituting the nitrogen-containing aliphatic ring each independently may be optionally replaced by a nitrogen atom, a sulfur atom or an oxygen atom, and when a carbon atom is replaced by a nitrogen atom, the nitrogen atom may be optionally substituted with lower alkyl), or
R⁹ and the nitrogen atom to which it is attached are taken together with ring A to form a 8- to 16-membered nitrogen-containing fused bicyclic ring (one or more carbon atoms constituting the nitrogen-containing fused bicyclic ring each independently may be optionally replaced by a nitrogen atom, a sulfur atom or an oxygen atom, and when a carbon atom is replaced by a nitrogen atom, the nitrogen atom may be optionally substituted with one or two lower alkyl groups), and R⁵ represents a hydrogen atom, a lower alkyl group (the alkyl group each independently may be optionally substituted with one or more substituents selected from halogen and hydroxy), or a cycloalkyl group,
R² or R³ each independently represents a halogen atom, OH, COOH, SO₃H, NO₂, SH, NR^{a}R^{b}, a lower alkyl group (the alkyl group each independently may be optionally substituted with one or more substituents selected from halogen and hydroxy) or a -O-lower alkyl group (the alkyl group each independently may be optionally substituted with one or more substituents selected from halogen and hydroxy),
R^{a} and R^{b} each independently represents a hydrogen atom or a lower alkyl group (the alkyl group each independently may be optionally substituted with one or more substituents selected from halogen and hydroxy),
m is an integer of 0 to 4, and
n is an integer of 0 to 2,
with the proviso that regardless of the above-mentioned definitions of R¹, R², R³, and R⁶, at least one of the R¹, R², R³, and R⁶ represents a group represented by formula: 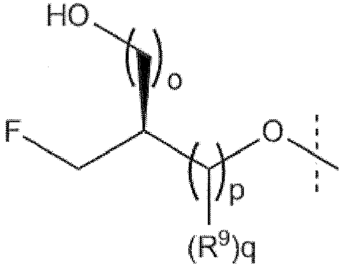 wherein
R⁹ represents each independently a lower alkyl group (the alkyl group each independently may be optionally substituted with one or more substituents selected from halogen and hydroxy),
o is an integer of 0 to 1,
p is an integer of 0 to 1,
q is an integer of 0 to 2, and
each line that the above dotted line intersects with means a bond to the other structural moiety of the above general formula (I),
or a pharmaceutically acceptable salt or solvate thereof.

2. The compound according to claim 1 wherein
A represents a cyclic group represented by formula: 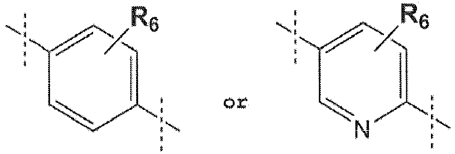 ring A is unsubstituted, or substituted with R⁶ (the R⁶ is one or more substituents selected independently from halogen and -O-lower alkyl (the alkyl group each independently may be optionally substituted with halogen and hydroxy)),
R¹ represents a group represented by formula: 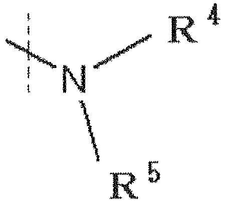 wherein
R⁴ and R⁵ each independently represent a hydrogen atom, a lower alkyl group (the alkyl group each independently may be optionally substituted with one or more substituents selected from halogen and hydroxy) or a cycloalkyl group,
R² represents a group represented by formula: 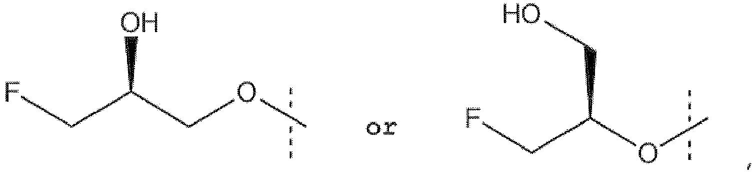
m is an integer of 1,
n is an integer of 0, and
each line that the above dotted line intersects with means a bond to the other structural moiety of the above general formula (I),
or a pharmaceutically acceptable salt or solvate thereof.

3. The compound according to claim 1 or 2 selected from the group consisting of formulae: 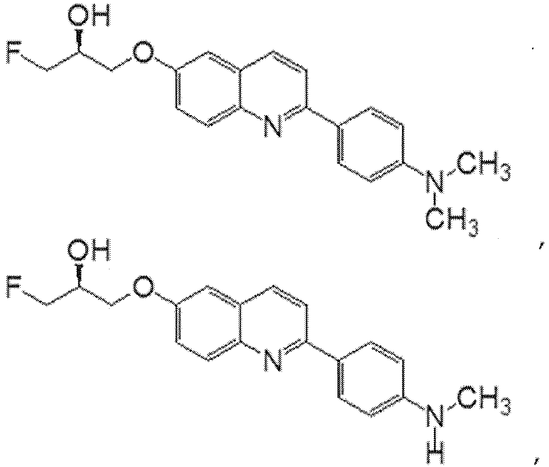 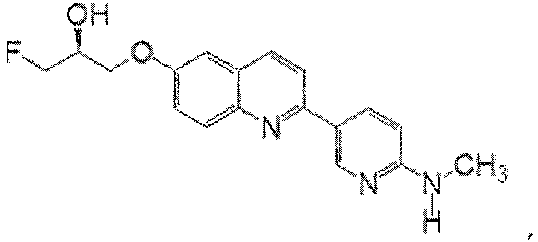 and 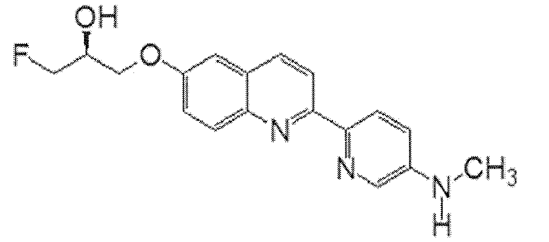 or a pharmaceutically acceptable salt or solvate thereof.

4. A pharmaceutical composition comprising the compound according to any one of claims 1 to 3 or a pharmaceutically acceptable salt or solvate thereof, and a pharmaceutically acceptable carrier.

5. A pharmaceutical composition for the treatment and/or prevention of conformational disease, comprising the compound according to any one of claims 1 to 3 or a pharmaceutically acceptable salt or solvate thereof, and a pharmaceutically acceptable carrier.

6. The pharmaceutical composition according to claim 4 or 5, wherein is an injection.

7. The compound according to any one of claims 1 to 3, wherein the compound is labeled, or a pharmaceutically acceptable salt or solvate thereof.

8. The compound according to claim 7, wherein the label is any one kind of a radioactive nuclide or a positron emitting nuclide.

9. The compound according to claim 8, or a pharmaceutically acceptable salt or solvate thereof, wherein the radioactive nuclide is a _{Υ}-ray emitting nuclide.

10. The compound according to claim 8, or a pharmaceutically acceptable salt or solvate thereof, wherein the positron emitting nuclide is selected from the group consisting of ¹¹C, ¹³N, ¹⁵O, ¹⁸F, ^{34m}Cl, ⁷⁶Br, ⁴⁵Ti, ⁴⁸V, ⁶⁰Cu, ⁶¹Cu, ⁶²Cu, ⁶⁴Cu, ⁶⁶Ga, ⁸⁹Zr, ^{94m}Tc and ¹²⁴I.

11. The compound according to claim 8, or a pharmaceutically acceptable salt or solvate thereof, wherein the positron emitting nuclide is ¹⁸F.

12. The compound according to claim 1, wherein said halogen atom is an F atom(s) and said compound is/are labeled with ¹⁸F.

13. The compound according to claim 12, wherein said halogen atom is an F atom(s) and said compound is/are labeled with ¹⁸F.

14. The compound according to claim 12 or 13 selected from the group consisting of the following formulae: 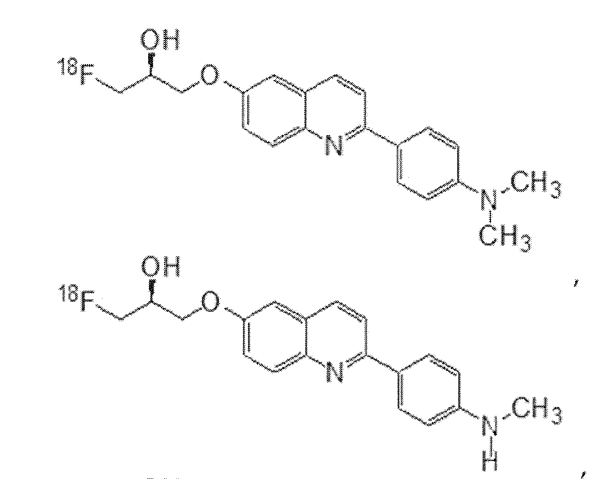 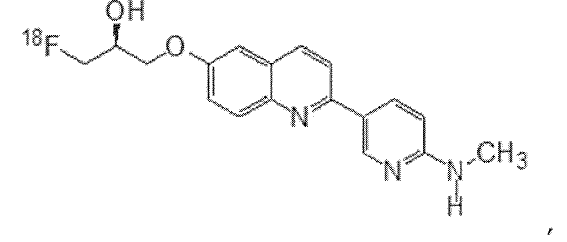 and 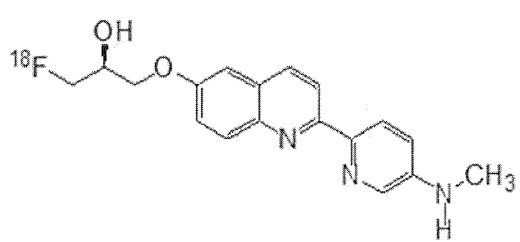 or a pharmaceutically acceptable salt or solvate thereof.

15. A pharmaceutical composition for diagnostic imaging, comprising any one of the compound according to claims 7 to 14, or a pharmaceutically acceptable salt or solvate thereof.

16. The pharmaceutical composition according to claim 15 for the diagnosis of conformational disease.

17. The pharmaceutical composition according to claim 15 for the detection or staining of a β-sheet structure protein.

18. A kit for diagnostic imaging, comprising the compound according to any one of claims 7 to 14 or a pharmaceutically acceptable salt or solvate thereof as an essential ingredient.

19. The kit according to claim 18 for the diagnosis of conformational disease.

20. The kit according to claim 18 for the detection or staining of a β-sheet structure protein.

21. A method of treating and/or preventing conformational disease in a subject, which comprises administering the compound according to any one of claims 1 to 3 or a pharmaceutically acceptable salt or solvate thereof to the subject.

22. A method of diagnostic imaging of conformational disease in a subject, which comprises administering the compound according to any one of claims 7 to 14 or a pharmaceutically acceptable salt or solvate thereof to the subject.

23. A method for diagnostic imaging, which comprises detecting or staining a β-sheet structure protein in a sample by staining the sample with the compound according to any one of claims 7 to 14 or a pharmaceutically acceptable salt or solvate thereof.

24. Use of the compound according to any one of claims 1 to 3 or a pharmaceutically acceptable salt or solvate thereof for the production of a pharmaceutical composition for the treatment and/or prevention of conformational disease in a subject.

25. Use of the compound according to any one of claims 7 to 14 or a pharmaceutically acceptable salt or solvate thereof for the production of a composition or kit for the diagnostic imaging of conformational disease in a subject.

26. Use of the compound according to any one of claims 7 to 14 or a pharmaceutically acceptable salt or solvate thereof for the production of a diagnostic imaging composition or kit for detecting or staining a β-sheet structure protein.

27. The pharmaceutical composition, kit, method or use according to any one of claims 15 to 26, wherein the conformational disease is tauopathy including Alzheimer's disease, and the β-sheet structure protein is tau protein.

28. A method of producing the compound represented by formula (I) according to claim 1, which comprises the following steps of:
(i) reacting a compound of a formula (V): 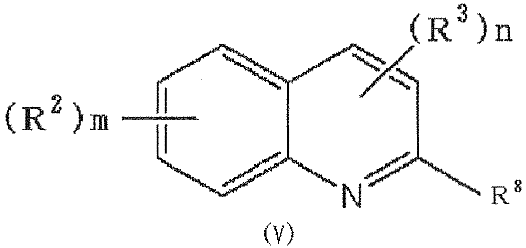 wherein
R⁸ represents a hydroxy group or a halogen atom. R², R³, m and n are as the same as defined as the above claim 1, with the proviso that at least one of R² and R³ represents a hydroxy group, with any compound represented by formula: 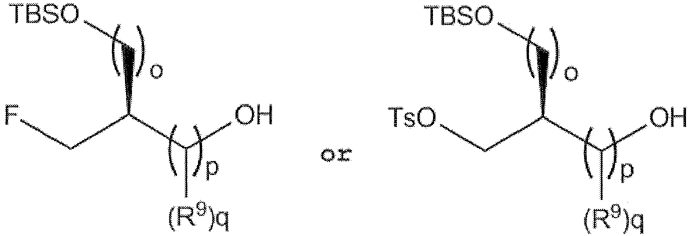 wherein,
R⁹, o, p and q are as the same as defined as the above claim 1,
to obtain a compound represented by a formula (V'): 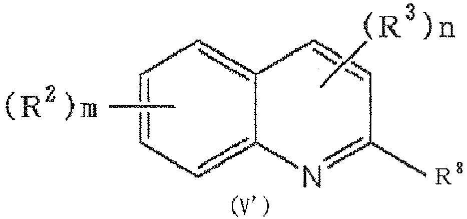 wherein
R⁸ represents a hydroxy group or a halogen atom. R², R³, m and n are as the same as defined as the above formula (V), with the proviso that regardless of the definition of the formula (I) according to claim 1, at least one of R² and R³ represents any group represented by formula: 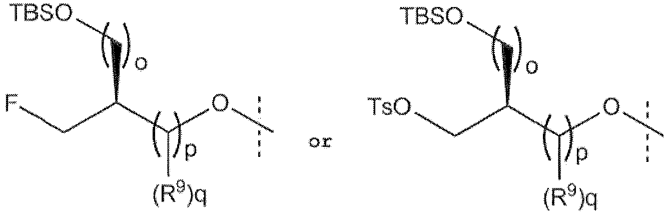 wherein,
R⁹, o, p and q are as the same as defined above,
(ii) reacting a compound represented by the above formula (V') with a compound represented by formula (VI) or (VII): 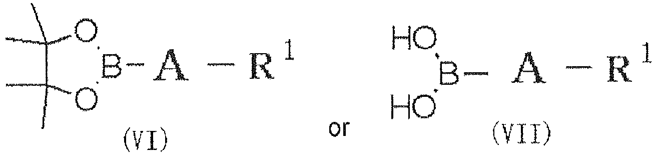 wherein, A and R¹ are as the same as defined as the above claim 1, to obtain the above compound represented by formula (I) wherein at least one of R² and R³ represents a group represented by formula: 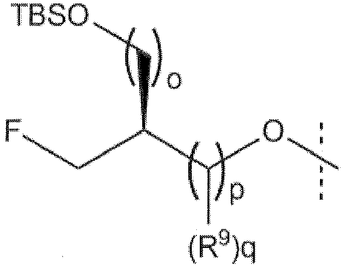 wherein,
R⁹, o, p and q are as the same as defined above,
and then isolating the compound, or
(iii) optionally, converting the obtained compound represented by formula (I) into another compound represented by formula (I), and then isolating the compound.

29. A method of producing the compound according to claim 2, which comprises
(i) reacting the compound represented by formula (V): 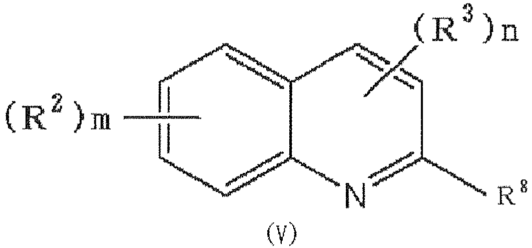 wherein
R⁸ represents a hydroxy group or a halogen atom. R², R³, m and n are as the same as defined as the above-mentioned claim 1, with the proviso that at least one of R² and R³ represents a hydroxy group,
with a compound represented by formula: 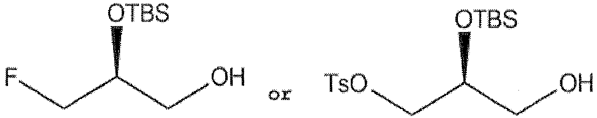 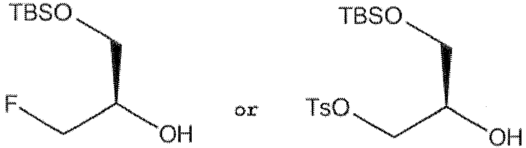 or to obtain a compound represented by formula (V'): 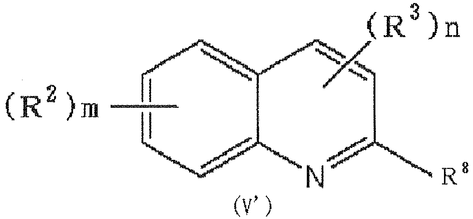 wherein,
R⁸ represents a hydroxy group or a halogen atom. R², R³, m and n are as the same as defined as the above formula (V), with the proviso that regardless of the definition of the formula (I) according to claim 1, at least one of R² and R³ represents any group represented by formula: 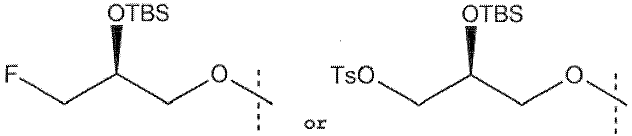 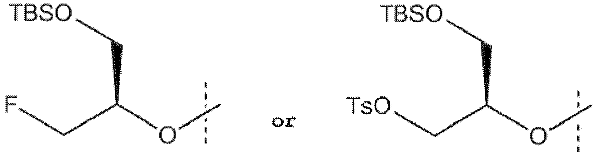 or
(ii) reacting the above compound represented by formula (V') with a compound represented by formula (VI) or (VII): 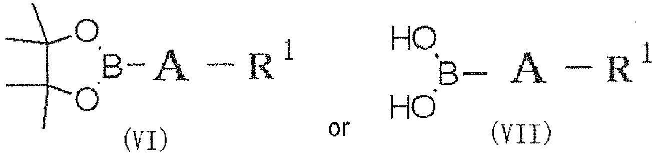 wherein,
A and R¹ are as the same as defined as the above claim 1, to obtain the above compound represented by formula (I) wherein at least one of R² and R³ represents a formula: 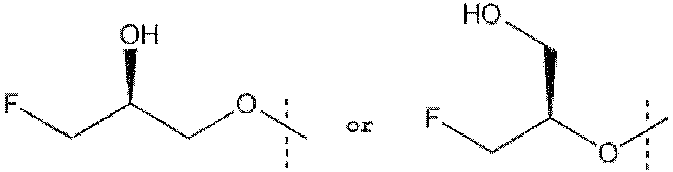 and then isolating the compound, or
(iii) optionally, converting the obtained compound represented by formula (I) into another compound represented by formula (I), and then isolating the compound.

30. A method for producing the compound represented by formula (I) according to claim 1 wherein at least one of R² and R³ represents a group represented by formula: 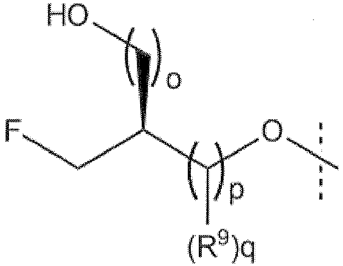 wherein,
R⁹, o, p and q are as the same as defined as the above claim 1,
which comprises
(i) reacting a compound represented by formula (V): 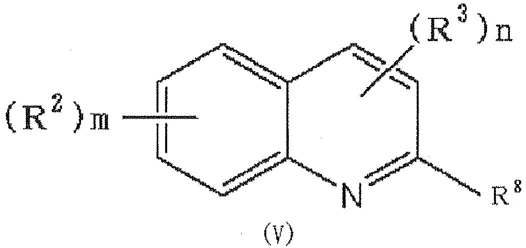 wherein,
R⁸ represents a hydroxyl group or a halogen atom. R², R³, m and n are as the same as defined as the above claim 1, with the proviso that at least one of R² and R³ represents a hydroxy group, with a compound represented by formula (VI) or (VII): 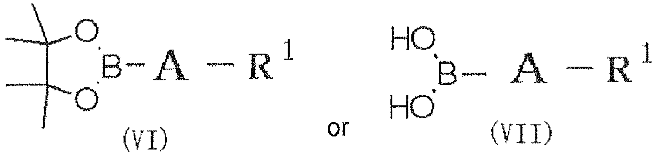 wherein,
A and R¹ are as the same as defined as the above claim 1, to obtain a compound represented by formula (V"): 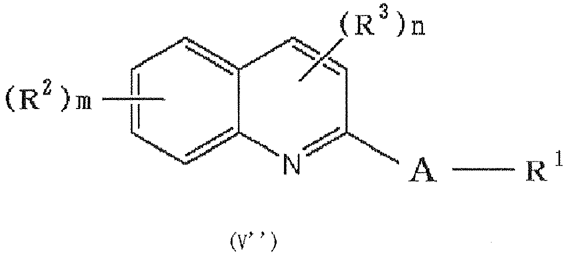 wherein,
R¹, R², R³, A, m and n are as the same as defined as the above formulae (V), (VI), and (VII), with the proviso that at least one of R² and R³ represents a hydroxy group,
(ii) reacting the above compound represented by formula (V'') with a compound represented by formula: 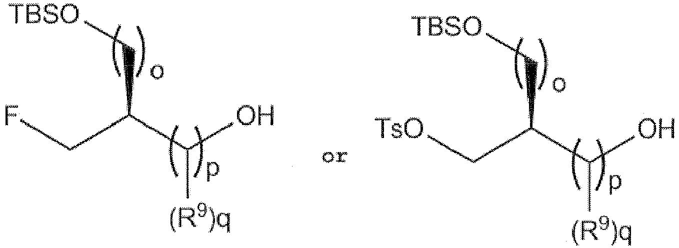 wherein,
R⁹, o, p and q are as the same as defined as the above claim 1,
to obtain the above-mentioned compound represented by formula (I) wherein at least one of R² and R³ represents a formula: 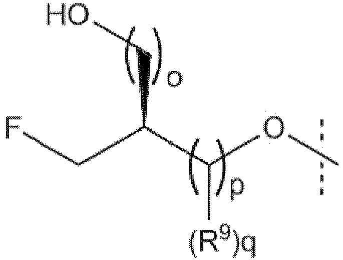 wherein,
R⁹, o, p and q are as the same as defined as above,
and then isolating the compound, or
(iii) optionally, converting the obtained compound represented by formula (I) into another compound represented by formula (I), and then isolating the compound.

31. The method for producing the compound according to claim 2 wherein at least one of R² and R³ represents a formula: 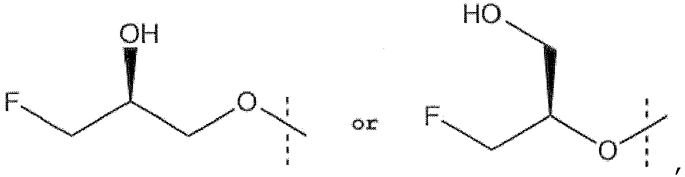 which comprises
(i) reacting a compound represented by formula (V): 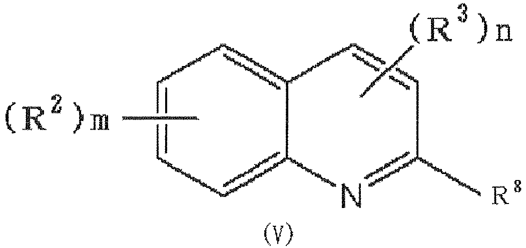 wherein,
R^{a} represents a hydroxyl group or a halogen atom. R², R³, m and n are as the same as defined as the above claim 1, with the proviso that at least one of R² and R³ represents a hydroxy group, with a compound represented by formula (VI) or (VII): 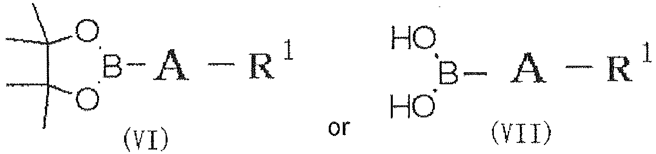 wherein,
A and R¹ are as the same as defined as the above claim 1, to obtain a compound represented by formula (V''): 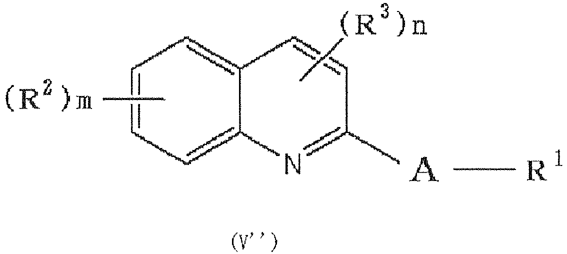 wherein,
R¹, R², R³, A, m and n are as the same as defined as the formula (V), (VI), and (VII), with proviso that at least of R² and R³ represents a hydroxy group,
(ii) reacting the above compound represented by formula (V") with any compound represented by formula: 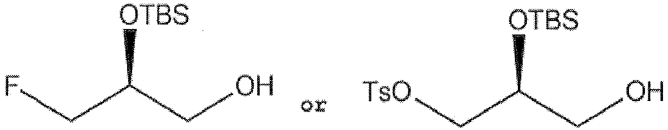 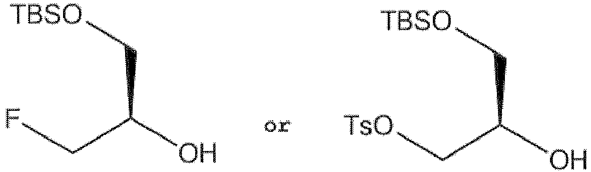 or to obtain the above compound represented by formula wherein at least of R² and R³ represents a formula: 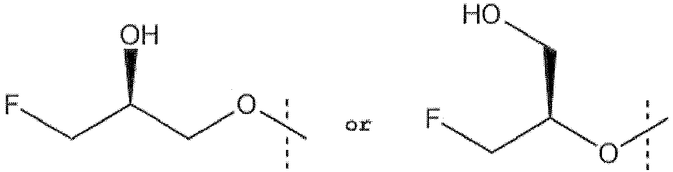 and then isolating the compound, or
(iii) optionally, converting the obtained compound represented by formula (I) into another compound represented by formula (I), and then isolating the compound.

32. The method according to any one of claims 28 to 31, which comprises
starting with a compound represented by formula (1): 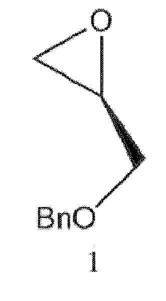 to prepare a compound represented by formula (4): 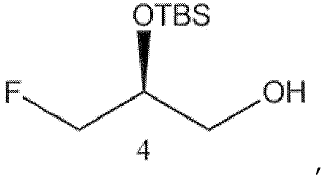 and
using the compound represented by formula (4) in the reaction in each process according to claims 28 t 31.

33. A compound represented by formula (I'): 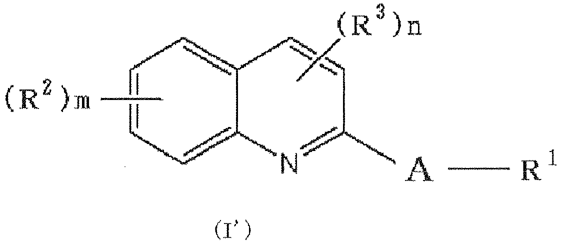 wherein
A is a cyclic group represented by a formula: 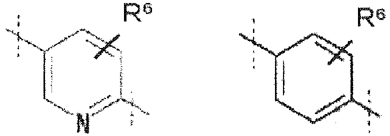 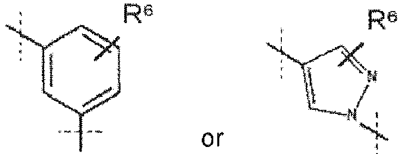 ring A is unsubstituted, or substituted with R⁶ (the R⁶ is one or more substituents selected independently from halogen, OH, COOH, SO₃H, NO₂, SH, NR^{a}R^{b}, lower alkyl (the alkyl group each independently may be optionally substituted with one or more substituents selected from halogen and hydroxy), and -O-lower alkyl (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group protected with a protecting group for hydroxy)),
R¹ is a halogen atom, a -C (=O)-lower alkyl group (the alkyl group each independently may be optionally substituted with one or more substituents selected from NR^{a}R^{b}, halogen and hydroxy), a lower alkyl group (the alkyl group each independently may be optionally substituted with one or more substituents selected from halogen atom and hydroxy), a -O-loweralkyl group (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group that is protected with a protecting group for hydroxy), or a group represented by a formula: 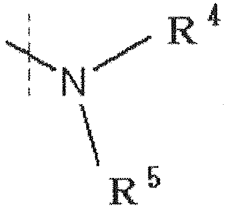 wherein
R⁴ and R⁵ each independently represents a hydrogen atom, a lower alkyl group (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2-tetrahydropyranyloxy group, an acetoxy group, a halogen atom, a hydroxy group, and a hydroxy lower alkyl group that is protected with a protecting group for hydroxy) or a cycloalkyl group, or R⁴, R⁵ and the nitrogen atom to which they are attached are taken together to form a 3- to 8-membered nitrogen-containing aliphatic ring (one or more carbon atoms constituting the nitrogen-containing aliphatic ring each independently may be optionally replaced by a nitrogen atom, a sulfur atom or an oxygen atom, and when a carbon atom is replaced by a nitrogen atom, the nitrogen atom may be optionally substituted with lower alkyl), or
R⁴ and the nitrogen atom to which it is attached are taken together with ring A to form a 8- to 16-membered nitrogen-containing fused bicyclic ring (one or more carbon atoms constituting the nitrogen-containing fused bicyclic ring each independently may be optionally replaced by a nitrogen atom, a sulfur atom or an oxygen atom, and when a carbon atom is replaced by a nitrogen atom, the nitrogen atom may be optionally substituted with one or two lower alkyl groups), and R⁵ represents a hydrogen atom, a lower alkyl group, or a cycloalkyl group,
R² or R³ each independently represents a halogen atom, OH, COOH, SO₃H, NO₂, SH, NR^{a}R^{b}, a -O-lower alkyl group (the alkyl group each independently may be optionally substituted with one or more substituents selected from a p-toluenesulfonyloxy group, a methanesulfonyloxy group, a chloromethanesulfonyloxy group, a trifluoromethanesulfonyloxy group, a 2--tetrahydropyranyloxy group, an acetoxy group, a halogen atom, a hydroxy group, and a hydroxy lower alkyl group that is protected with a protecting group for hydroxy),
R^{a} and R^{b} each independently represents a hydrogen atom or a lower alkyl group (the alkyl group each independently may be optionally substituted with one or more substituents selected from halogen and hydroxy),
m is an integer of 0 to 2, and
n is an integer of 0 to 2,
with the proviso that regardless of the above-mentioned definitions of R¹, R², R³, and R⁶, at least one of the R¹, R², R³, and R⁶ represents a group represented by formula: 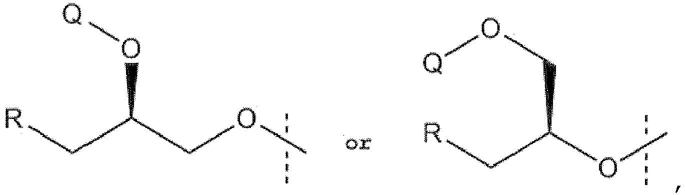
or the R¹ represents a group represented by formula: 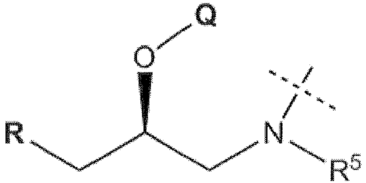 wherein,
R⁵ is the same as defined above, and
the Q substituent represents a protecting group for hydroxy group that has a resistance against a nucleophilic substitution by fluorine anion and may be removed under acidic or alkali condition, and the R substituent represents a functional group that works as a leaving group against a nucleophilic substitution by fluorine anion, and
each line that the above dotted line intersects with means a bond to the other structural moiety of the above general formula I',
or a pharmaceutically acceptable salt or solvate thereof.

34. The compound according to claim 33 or a pharmaceutically acceptable salt or solvate thereof, wherein the protecting group for hydroxy group that has a resistance against a nucleophilic substitution by fluorine anion and may be removed under acidic or alkali condition is selected from a 2-tetrahydropyranyl group, a methoxymethyl group, a 2-methoxyetoxymethyl group, an ethoxyethyl group, an acetyl group, and a pivaloyl group,
the functional group that works as a leaving group against a nucleophilic substitution by fluorine anion is selected from a p-toluenesulfonyloxy group, a methanesulfonyloxy group, a chloromethanesulfonyloxy group, and a trifluoromethanesulfonyloxy group.

35. The compound according to claim 33 wherein
A represents a cyclic group represented by formula: 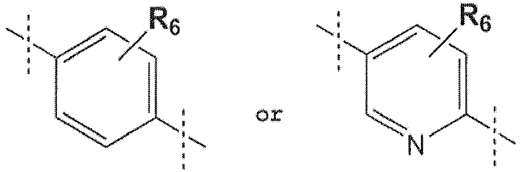 ring A is unsubstituted, or substituted with R⁶ (the R⁶ is one or more substituents selected independently from halogen and -O-lower alkyl (the alkyl group each independently may be optionally substituted with halogen and hydroxy)), R¹ represents a group represented by formula: 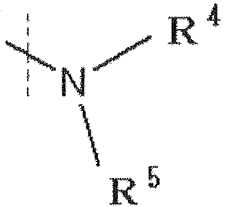 wherein
R⁴ and R⁵ each independently represent a hydrogen atom, a lower alkyl group (the alkyl group each independently may be optionally substituted with one or more substituents selected from halogen and hydroxy) or a cycloalkyl group,
R² represents a group represented by formula: 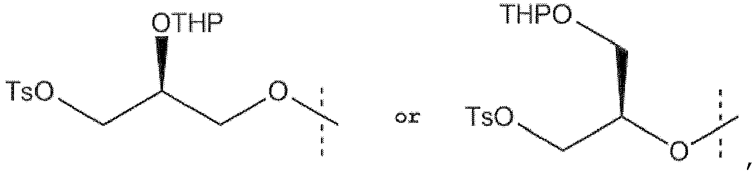
m is an integer of 1,
n is an integer of 0, and
each line that the above dotted line intersects with means a bond to the other structural moiety of the above general formula I',
or a pharmaceutically acceptable salt or solvate thereof.

36. The compound according to any one of claims 33 to 35 selected from the group consisting of formulae: 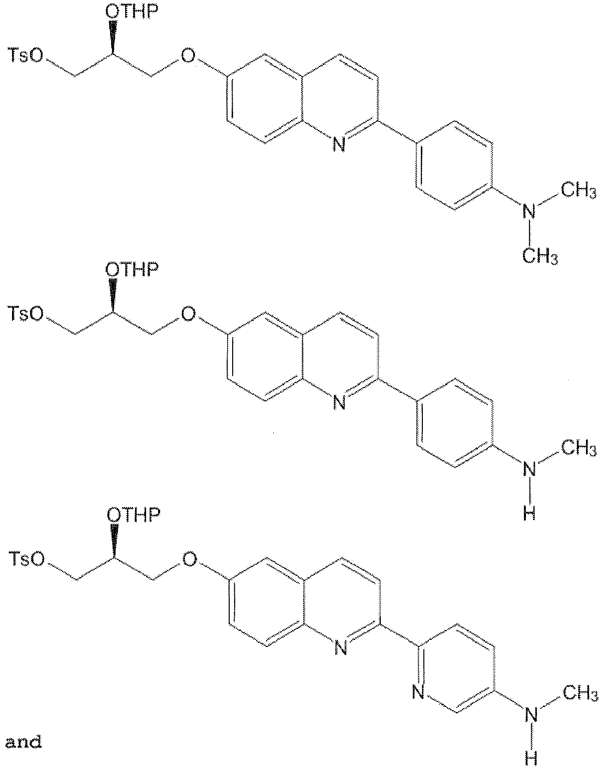 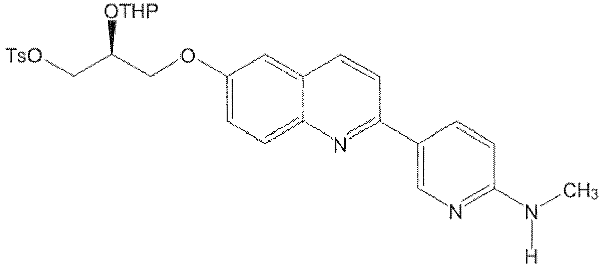 or a pharmaceutically acceptable salt or solvate thereof.

37. A kit for producing the labeled compound according to any one of claims 7 to 14, or a pharmaceutically acceptable salt or solvate thereof, comprising:
the compound according to any one of claims 33 to 36, or a pharmaceutically acceptable salt or solvate thereof,
a labeling agent or a reagent for preparing the agent, or a solvent,
a container or an instrument for use of a labeling synthesis or a formulation such as syringe, three-way stopcock, needle, solid-phase extraction cartridge, and sterilizing filter, and
optionally, instructions for carrying out labeling.

38. The kit according to claim 37, wherein the label is a radioactive nuclide or a positron emitting nuclide, or an agent containing them.

39. The kit according to claim 37, wherein the radioactive nuclide is a γ-ray emitting nuclide.

40. The kit according to claim 37, wherein the positron emitting nuclide as the labeling agent is selected from the group consisting of ¹¹C ¹³N ¹⁵O ¹⁸F, ^{34m}Cl ⁷⁶Br ⁴⁵Ti ⁴⁸V ⁶⁰Cu, ⁶¹Cu, ⁶²Cu, ⁶⁴CU, ⁶⁶Ga, ⁸⁹Zr, ^{94m}Tc and ¹²⁴I.

41. The kit according to according to claim 40, wherein the positron emitting nuclide is ¹⁸F.

42. A separation method of racemic compounds according to [1] represented by formula (I): 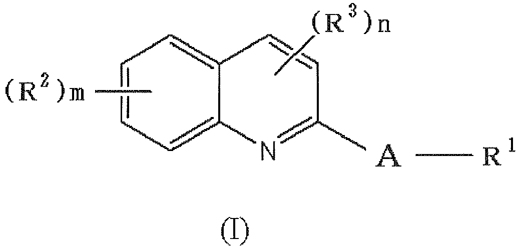 wherein
A represents a cyclic group represented by formula: 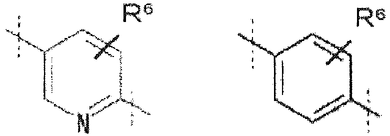 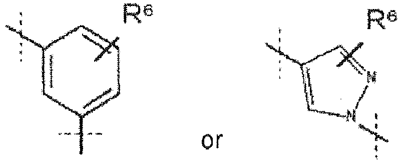
ring A is unsubstituted, or substituted with R⁶ (the R⁶ is one or more substituents selected independently from halogen, OH, COOH, SO₃H, NO₂, SH, NR^{a}R^{b}, lower alkyl (the alkyl group each independently may be optionally substituted with one or more substituents selected from halogen and hydroxy) and -O-lower alkyl (the alkyl group each independently may be optionally substituted with one or more substituents selected from halogen and hydroxy)),
R¹ is a halogen atom, a -C (=O) -lower alkyl group (the alkyl group each independently may be optionally substituted with one or more substituents selected from NR^{a}R^{b}, halogen and hydroxy), a lower alkyl group (the alkyl group each independently may be optionally substituted with one or more substituents selected from halogen and hydroxy), a -O-lower alkyl group (the alkyl group each independently may be optionally substituted with one or more substituents selected from halogen and hydroxy), or a group represented by a formula: 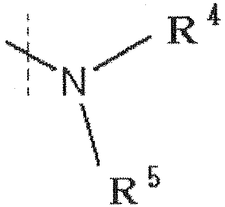 wherein
R⁴ and R⁵ each independently represent a hydrogen atom, a lower alkyl group (the alkyl group each independently may be optionally substituted with one or more substituents selected from halogen and hydroxy) or a cycloalkyl group, or R⁴, R⁵ and the nitrogen atom to which they are attached are taken together to form a 3- to 8-membered nitrogen-containing aliphatic ring (one or more carbon atoms constituting the nitrogen-containing aliphatic ring each independently may be optionally replaced by a nitrogen atom, a sulfur atom or an oxygen atom, and when a carbon atom is replaced by a nitrogen atom, the nitrogen atom may be optionally substituted with lower alkyl), or
R⁴ and the nitrogen atom to which it is attached are taken together with ring A to form a 8- to 16-membered nitrogen-containing fused bicyclic ring (one or more carbon atoms constituting the nitrogen-containing fused bicyclic ring each independently may be optionally replaced by a nitrogen atom, a sulfur atom or an oxygen atom, and when a carbon atom is replaced by a nitrogen atom, the nitrogen atom may be optionally substituted with one or two lower alkyl groups), and R⁵ represents a hydrogen atom, a lower alkyl group (the alkyl group each independently may be optionally substituted with one or more substituents selected from halogen and hydroxy), or a cycloalkyl group,
R² or R³ each independently represents a halogen atom, OH, COOH, SO₃H, NO₂, SH, NR^{a}R^{b}, a lower alkyl group (the alkyl group each independently may be optionally substituted with one or more substituents selected from halogen and hydroxy) or a -O-lower alkyl group (the alkyl group each independently may be optionally substituted with one or more substituents selected from halogen and hydroxy),
R^{a} and R^{b} each independently represents a hydrogen atom or a lower alkyl group (the alkyl group each independently may be optionally substituted with one or more substituents selected from halogen and hydroxy),
m is an integer of 0 to 4, and
n is an integer of 0 to 2,
with the proviso that regardless of the above-mentioned definitions of R¹, R², R³, and R⁶, at least one of the R¹, R², R³, and R⁶ represents a group represented by formula: 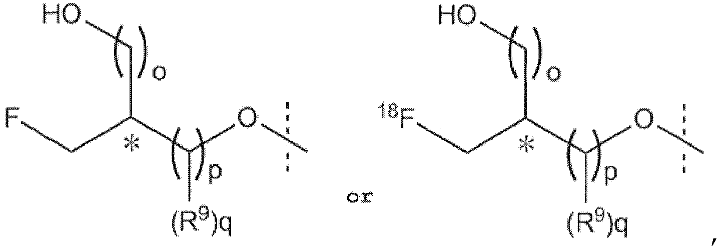 wherein
R⁹ represents each independently a lower alkyl group (the alkyl group each independently may be optionally substituted with one or more substituents selected from halogen and hydroxy),
o is an integer of 0 to 1,
p is an integer of 0 to 1,
q is an integer of 0 to 2, and
each line that the above dotted line intersects with means a bond to the other structural moiety of the above general formula (I),
to the optical active compound according to any one of claims 1
to 3 as one enantiomer by using an optical active column.

43. A method for producing the compound according to any one of claims 7 to 14 by a labeling synthesis via a chemical synthesis method with a labeling agent or an isotope exchange method.

44. The method according to claim 42, which comprises contacting the solution containing the compound according to any one of claims 33 to 36 with an ion-exchange resin supported by ¹⁸F⁻ to convert to the ¹⁸F⁻ labeled compound according to claims 7 to 14.

45. A method for producing the ¹⁸F⁻ labeled compound according to any one of claims 7 to 14 by using the compound according to any one of claims 33 to 36 as a label precursor, and ¹⁸F anion.

46. The method according to claim 45, which comprises reacting the compound according to claims 33 to 36 with ¹⁸F anion in absolute highly polar solvent with heating to introduce ¹⁸F into the compound via nucleophilic substitution, followed by removing a protecting group for hydroxy group under acidic or alkali condition.
